# EUROPEAN PATENT APPLICATION

(11) **EP 4 578 412 A1**
(43) Date of publication of application: **02.07.2025**
(21) Application number: 23856375.3
(22) Date of filing: 25.07.2023
(51) Int. Cl.: A61B 18/12, A61B 18/14, A61B 34/20, A61B 34/10, A61B 18/00, A61M 25/00, A61B 10/02, G16H 50/30

(54) **OBJECT ABLATION SYSTEM, CONTROL METHOD AND APPARATUS, MEDIUM, AND ELECTRONIC DEVICE**

(30) Priority: 26.08.2022 CN 202211037397; 26.08.2022 CN 202222270126 U; 18.11.2022 CN 202223067057 U; 13.12.2022 CN 202211614498; 29.12.2022 CN 202211713102; 29.12.2022 CN 202211716240; 29.12.2022 CN 202211716334
(71) Applicant: Merryspring Medical Technology (Zhejiang) Co, Ltd., Jiaxing, Zhejiang 314000 (CN)
(72) Inventor: XU, Shuhan, Jiaxing, Zhejiang 314000 (CN); TAO, Lin, Jiaxing, Zhejiang 314000 (CN); YANG, Yong, Jiaxing, Zhejiang 314000 (CN); YE, Ping, Jiaxing, Zhejiang 314000 (CN)
(74) Representative: Isern Patentes y Marcas S.L.
(86) International application number: PCT/CN2023/109091
(87) International publication number: WO 2024/041285

(57) **Abstract**

Provided are an object ablation system, control method and apparatus, a medium, and an electronic device. A navigation control device is configured to perform virtual model construction on the basis of region image information of a target intervention region, morphological attribute information of an intervention assembly, movement position information, and intervention position information to obtain a navigation three-dimensional model and an operation object three-dimensional model, the navigation three-dimensional model being configured to characterize three-dimensional spatial features of the target intervention region, three-dimensional spatial features of the intervention assembly, and spatial position information between an object to be ablated, the intervention assembly and the target intervention region. The navigation control device is further configured to determine a target ablation parameter corresponding to the object to be ablated and control an ablation device to work on the basis of the target ablation parameter..

## Description

### TECHNICAL FIELD

The present disclosure relates to the technical field of medical instruments, and in particular, to an object ablation system, a control method, an apparatus, a medium, and an electronic device.

### BACKGROUND ART

Existing ablation methods mainly adopt thermal ablation technologies such as radio frequency ablation, microwave ablation, cryoablation, and focused ultrasound ablation. The fundamental principle of radio frequency ablation is thermal therapy, which is composed of an alternating electric field and a magnetic field through a highfrequency electromagnetic wave, and energy generated by radio frequency waves of 375 kHz to 500 kHz is formed into a closed loop from the emitter to the treatment electrode needle through the negative electrode plate in the human body. The ions around the electrode needle collide with the excitation of the alternating current to form heat, and when the heat exceeds the tolerance of the tumor tissue, the tumor cells will coagulate necrosis, and the small blood vessels around the tumor are blocked due to heat damage, thereby blocking tumor blood supply. During operation, the lesion boundary in real-time ultrasound is completely blurred due to large-area coagulation necrosis at the moment of tumor tissue. Therefore, when determining whether to completely ablate, it is difficult to define the distance between the ablation range and the lesion edge, and may cause complications such as skin blood stasis, skin burn, muscle burn, continuous fat necrosis, and wound infection.

There are mainly two methods for microwave ablation: focused microwave phased array thermotherapy (FMPA) and transcutaneous microwave thermal coagulation (PMC). FMPA microwaves generate dielectric heating through rapid agitation of tissue and intracellular water molecules, resulting in thermally induced coagulation necrosis. PMC is the direct insertion of a probe that can emit microwave energy into the interior of the tumor, resulting in high temperatures that cause protein denaturation or cell coagulation necrosis within a short period of time. The main mechanism of action is because the moisture of the cancer cells is higher than that of the normal cells, so that the cancer cells generate more heat under the action of microwaves to coagulate the cells, but still have the phenomenon of thermal damage to the surrounding tissues of the skin burn.

Laser ablation is mainly to insert tip laser fibers into a tumor, generate a thermal effect between the laser photons and the tissue, improve tissue temperature and generate a thermal ablation region, but may cause permanent damage to surrounding blood vessels and neural tissues, and also cause incomplete ablation or excessive ablation.

High intensity focused ultrasound ablation (HIFU) is a non-invasive non-invasive ablation technique that does not require a minimally invasive placement of a catheter or probe at a tumor site. By focusing the high-energy ultrasound, HIFU reaches a very high sound intensity at the focal point, so that the acoustic energy is rapidly absorbed by the tumor tissue and converted into thermal energy, the local temperature rises to 65°C or more instantaneously, protein denaturation and cell coagulation necrosis of the target region are achieved, and normal tissue passing through the ultrasonic beam and surrounding tissues of the target region do not cause damage or damage. However, in the HIFU treatment process, the target area tissue is required to maintain a static state as much as possible, so that movement is reduced, otherwise, a treatment miss is easily caused, so that complete ablation cannot be achieved; and secondly, although radical treatment can be achieved for ablation of a tumor edge expansion range, the ablation range is increased, and meanwhile, the risk of internal tissue damage is increased.

Cryoablation is to place a cryoprobe in the center of a tumor, so that the temperature of the tumor cell rapidly drops below a freezing point, and in the process of repeated freezing and thawing, tumor cell membrane rupture and organelle damage are caused, and finally tumor cell death is caused. But cells that are not directly frozen and lethal may also relay cell apoptosis, while cryoablation may also induce local microvascular disruption to cause blood flow stasis.

The above ablation principle is mainly to achieve the purpose of tissue cell necrosis through the change of the surrounding temperature of the tissue. For example, US 14023328, US 15099665, CN 202011501204.5, and the like. However, when a heat sink effect occurs, the thermal ablation technology easily causes damage to healthy tissues such as nerves, lymph, blood vessels and the like in the ablation area. For example, some manufacturers ablate lung diseases, such as chronic bronchitis, by means of radio frequency ablation technology, so as to expand the trachea by ablating smooth muscle around the tracheal cartilage and increase the ventilation quantity; however, the method also belongs to a thermal ablation technology, and the cell protein is deteriorated by high temperature, so that tissue necrosis is achieved, and it is difficult to control the ablation range to easily cause health tissues such as blood vessels and nerves to be damaged. In addition, the trachea is mainly supported by cartilage and smooth muscle, and therefore, if smooth muscle is excessively ablated, the method can also easily cause the cartilage to be unable to support, resulting in more serious consequences caused by the collapse of the trachea.

The pulsed electric field ablation technology using irreversible electroporation theory as a non-thermal ablation technology has gained attention to clinical applications. Pulsed electric field ablation technology is to release extremely high energy in a short time by generating a high-voltage pulsed electric field with a pulse width of millisecond, microsecond, or even nanosecond, which enables cell membranes and even organelles in cells such as endoplasmic reticulum, mitochondria, nuclei, and the like to generate a large amount of irreversible micropores. Further, apoptosis of the diseased cells is caused, thereby achieving an expected therapeutic purpose. In the application for treating chronic obstructive pulmonary disease, pulmonary inflammatory cells can be selectively treated by using a pulsed electric field ablation technology without affecting other non-target cell tissues, and at the same time, it also has the characteristics of complete full-layer ablation, precision, rapidness, blood vessel protection, nerve and cartilage. At the same time, the pulse electric field ablation technology can perform superposition ablation multiple times due to the effect of the non-heated pool effect, thereby greatly enhancing the ablation depth.

In current ablation therapy, a surgical operation is configured to cut the surrounding tissue, so that the tumor is completely exposed and puncture ablation and resection are performed on the human body, the incision healing time is long, and the risk of wound infection is also accompanied, so that minimally invasive interventional therapy has become a standard surgical method in the medical community. When biopsy or ablation of a lesion is performed on a target lesion area in an intervention manner, it is crucial to determine the position of the interventional device in the target object body for an accurate implementation procedure. An existing method of determining an accurate position of an interventional device in a target object comprises observing and applying a perspective image through an endoscopic image. In an actual use process, for example, for the treatment of lung lesions, since the lung bronchial anatomical structure presents multi-level branch bifurcation, the accurate position of the interventional device in the bronchus cannot be identified and recorded only through the image of the bronchial endoscope, and is limited to the outer diameter of the bronchial endoscope, and the endoscope cannot reach a finer bronchial branch, so that the help of the image aspect cannot be provided for the interventional device. In the surgery process, because the perspective image has a certain damage to the human body, perspective images cannot be used all the time to observe, and the perspective image is not clear to the image of the lung bronchus, so that the position of the interventional device in the image cannot be determined from the three-dimensional angle. For interventional therapy of a complex region similar to a lung bronchus, the operator can easily "get lost" in the lung bronchus of the "complex bifurcation" when controlling the interventional device, thereby affecting the accuracy and efficiency of the surgery, and increasing the working intensity of the operator.

In the existing treatment of lung diseases, for example, CN201810310511.1, CT imaging technology needs to be adopted to assist in guiding the ablation head to pass through the bronchial branch to arrive at the tumor, so that the patient and the operator can accept a large amount of rays. CN201580060018.3 needs to mark the tissue in the vicinity of the target first, and through cooperative positioning of the fluoroscopic image and the CT image, the positioning method is complex and low in accuracy, and ablation treatment is performed by using a microwave ablation device. CN202010113062.9 needs to use a fusion positioning method matched with a depth camera and a magnetic locator to position the position of the ablation needle, so as to position real-time performance and visibility difference. CN109788979A, which adopts a pulsed electric field ablation technology, but cannot determine the accurate position of the interventional device in the treatment region during interventional and energy delivery, and can only perform control parameter correction based on the feedback of the implemented condition, so that the accuracy of pulse energy ablation, the real-time performance, and the poor pertinence to the lesion region are solved. Patents, such as US 13538947, CN 201711006154.1 and so on, provide a probe-type ablation head to treat a narrow lung region tissue, but the probe-type ablation head cannot be well attached to the lesion tissue, and the ablation area is small.

Most existing interventional devices have the problems of large volume, poor connection reliability, poor bending flexibility, failure to reach farther and narrower bronchial branches, and being unable to be closely attached to lesion tissues, thereby greatly affecting the accuracy, efficiency and treatment effect of surgery, and easily causing damage to patients.

Based on the shortcomings of the prior art, it is necessary to provide a comprehensive system that can navigate and determine the accurate position of the interventional instrument in a complex treatment area by adopting a minimally invasive interventional treatment mode, so that the interventional instrument can accurately reach the treatment area to be uniformly attached to the lesion tissue, and meanwhile, a pulsed electric field ablation technology is adopted to generate more accurate pulse energy to perform targeted treatment on the lesion tissue.

### SUMMARY

In order to solve the problems in the prior art that an object ablation system cannot navigate an interventional device in a complex area to accurately reach a target treatment position and is uniformly attached to a lesion tissue, and the ablation energy sent by the ablation device is uncontrollable in the ablation range of the tissue, the health tissue is easily damaged, and the accuracy, efficiency and treatment effect of the operation are affected. The present application provides an object ablation system, a control method, an apparatus, a medium, and an electronic device.

In one aspect, the present application provides an object ablation system, comprising an interventional device, a navigation control device, a first position acquisition device, and an ablation device; the interventional device comprises a second position acquisition device and an intervention assembly; the first position acquisition device, the second position acquisition device, the intervention assembly and the ablation device are communicatively connected to the navigation control device, respectively.

The first position acquisition device is configured to acquire movement position information of a target object and transmit the movement position information to the navigation control device.

The second position acquisition device is configured to acquire intervention position information of the intervention assembly in a target intervention region, and transmit the intervention position information to the navigation control device, where the target intervention region belongs to the target object.

The navigation control device is configured to construct a virtual model based on region image information of the target intervention region, morphological attribute information of the intervention assembly, the movement position information, and the intervention position information, to obtain a three-dimensional navigation model and an operation object three-dimensional model, where the navigation three-dimensional model is configured to represent three-dimensional space features of the target intervention region, three-dimensional space features of the intervention assembly, and spatial position information between the to be ablated object, the intervention assembly, and the target intervention region, and the operation object three-dimensional model is configured to represent a three-dimensional space feature of the to be ablated object.

The navigation control device is further configured to determine a target ablation parameter corresponding to the to be ablated object according to the navigation three-dimensional model, the operation object three-dimensional model, and object attribute information of the to be ablated object; and control the ablation device to operate based on the target ablation parameter, so that the intervention assembly performs ablation processing on the to be ablated object.

Further, the navigation control device comprises the following modules:
an intervention region sub-model construction module, configured to perform image recognition processing on the region image information of the target intervention region to obtain an image recognition result; and perform image reconstruction based on the image recognition result to obtain an intervention region sub-model and the operation object three-dimensional model, where the intervention region sub-model is configured to represent a three-dimensional space feature of the target intervention region;
an intervention assembly sub-model construction module, configured to perform virtual model construction according to the morphological attribute information of the intervention assembly to obtain an intervention assembly sub-model, where the intervention assembly sub-model is configured to represent a three-dimensional space feature of the intervention assembly;
a fusion module, configured to perform spatial fusion processing on the intervention region sub-model, the operation object three-dimensional model and the intervention assembly sub-model according to the intervention position information and the movement position information to obtain the navigation three-dimensional model.

Further, the navigation control device further comprises a navigation planning module, and the navigation planning module is communicatively connected to the navigation three-dimensional model.

The navigation planning module is configured to perform navigation planning according to the intervention region sub-model and the operation object three-dimensional model to obtain a navigation path of the intervention assembly in the intervention region sub-model, where the navigation path is configured to indicate a path of travel required by the intervention assembly to reach the to be ablated object in the target intervention region.

Further, the navigation planning module is further configured to determine, in a movement process of the intervention assembly in the target intervention region, intervention trajectory information of the intervention assembly based on the intervention position information of the intervention assembly and the movement position information; if the path trajectory information corresponding to the intervention trajectory information and the navigation path meets a preset deviation condition, correcting the intervention position of the intervention assembly in the target intervention region, until the updated intervention trajectory information of the intervention assembly matches the path trajectory information.

Further, the navigation control device further comprises an initial parameter acquisition module and a target parameter determination module.

The initial parameter acquisition module is configured to obtain an initial ablation parameter corresponding to object attribute information of the to be ablated object, impedance data of the to be ablated object, a contact parameter of the intervention assembly, and a dielectric constant of the to be ablated object, where the initial ablation parameter at least comprises at least one of a pulse voltage, a pulse width, a number of pulses, and a number of pulse groups.

The target parameter determination module is configured to obtain ablation effect data corresponding to the initial ablation parameter according to the navigation three-dimensional model, the operation object three-dimensional model, the object attribute information of the object to be ablated, the initial ablation parameter corresponding to the object attribute information of the object to be ablated, the impedance data of the object to be ablated, the abutment parameter of the intervention assembly, and the dielectric constant of the object to be ablated; determining the initial ablation parameter as the target ablation parameter if the ablation effect data meets a target ablation condition.

Further, the interventional device comprises a contact detection device and a dielectric constant detection device, and the contact detection device and the dielectric constant detection device are communicatively connected to the navigation control device, respectively.

The abutting detection device is configured to detect impedance data of the to be ablated object and an abutment parameter of the intervention assembly, where the impedance data is configured to indicate a load of the intervention assembly, and the abutment parameter is configured to indicate a degree of contact between the intervention assembly and the to be ablated object; and send the abutment parameter to the initial parameter acquisition module.

The dielectric constant detection device is configured to detect a dielectric constant of the to be ablated object.

Further, the target parameter determination module comprises a simulation ablation model construction unit.

The simulation ablation model construction unit is configured to perform ablation effect simulation based on the object attribute information, the initial ablation parameter, the impedance data, the abutment parameter, the dielectric constant, the operation object three-dimensional model, and the navigation three-dimensional model as input of an ablation evaluation model, to obtain a simulation ablation model corresponding to the initial ablation parameter, where the simulation ablation model is configured to represent ablation effect data corresponding to the initial ablation parameter.

Further, the navigation control device further comprises an ablation three-dimensional model generation module.

The ablation three-dimensional model generation module is configured to obtain ablation trajectory information and ablation data of the intervention assembly after the intervention assembly performs ablation processing on the to be ablated object; and generate an ablation three-dimensional model according to the ablation trajectory information, the ablation data, and the target ablation parameter, where the ablation data comprises an ablation region that has been ablated and ablation position information of the ablation region, and the ablation three-dimensional model represents a three-dimensional spatial feature of the ablation object to be ablated.

Further, the navigation control device comprises a pulse energy generation module.

The pulse energy generation module is configured to generate an energy generation control signal based on the target ablation parameter to control the ablation device to operate.

Further, the control circuit of the ablation device comprises a switch circuit, the switch circuit comprises at least two branch circuits connected in parallel, a full bridge, a half bridge, or a series connection, and each branch circuit comprises an input terminal switch module and an output terminal switch module; and the switch circuit controls the corresponding input terminal switch module and the output terminal switch module to conduct in response to the energy generation control signal of the pulse energy generation module to generate pulse energy.

Further, the branch circuit comprises a filter module, the filter module comprises a plurality of filtering units, the filtering unit comprises a plurality of filters and a filtering selector, and the filtering selector is configured to select different filters to perform filtering processing.

Further, the interventional device comprises a mandrel and a manipulation assembly, and the mandrel is electrically connected to the intervention assembly and the ablation device, respectively;
the second position acquisition device is disposed on the mandrel and/or the intervention assembly;
the intervention assembly comprises at least one ablation electrode, the at least one ablation electrode is in a mesh shape, the at least one ablation electrode is sequentially arranged along the mandrel, the manipulation assembly is sleeved on an outer side of the mandrel, and the manipulation assembly can move relative to the mandrel to drive the intervention assembly to expand or contract.

Further, the abutting detection device and the dielectric constant detection device are both arranged on the intervention assembly, and the attaching detection device and the dielectric constant detection device are both communicatively connected to the navigation control device.

Further, the interventional device comprises a fixing sleeve, the two ends of the sleeve body of the fixing sleeve are provided with inner tooth structures in the circumferential direction, and both ends of the inner tooth structures are obliquely arranged from the end of the sleeve body to the inner side of the end face.

Further, the second position acquisition device is disposed between the two inner tooth structures of the fixing sleeve.

Further, the manipulation assembly comprises at least one control connected to the ablation electrode.

Further, the at least one ablation electrode can be configured as a unipolar electrode or a bipolar electrode.

Further, a maximum distance that the plurality of ablation electrodes may expand in sequence along the mandrel is sequentially increased in a direction from a distal end to a proximal end of the mandrel.

Further, the ablation electrode comprises a plurality of electrode lines, and the plurality of electrode lines are crossed and woven into a mesh-shaped ablation electrode.

Further, the axial section of the ablation electrode after expansion is elliptical, spindle-shaped, polygonal or umbrella-shaped.

Further, a handle is comprised, and a channel through which the mandrel and the manipulation assembly pass is provided in the operating handle.

Further, the operating handle is provided with a manipulation assembly, the manipulation assembly is slidably or rotatably connected to the operating handle, and the manipulation assembly is configured to control the at least one control tube to move.

In another aspect, the present application provides a medical intervention device, comprising an inner core tube, a control outer tube, an electrode wire and an ablation electrode, wherein the electrode wire is detachably arranged in the inner core tube in a penetrating manner, and the control outer tube is sleeved on an outer side of the inner core tube;
a distal end of the ablation electrode is fixedly connected to a distal end of the inner core tube, a proximal end of the ablation electrode is fixedly connected to an outer wall of the control outer tube, and the control outer tube can move along the inner core tube to drive the ablation electrode to expand or contract.

Further, the inner core tube is a through tube, and a distal end of the electrode wire can pass through a distal end of the inner core tube to be in contact with a lesion tissue.

Further, the electrode wire and the ablation electrode are both electrically connected to the ablation device.

Further, the ablation electrode comprises a plurality of electrode lines, and the plurality of electrode lines are cross-woven into a mesh.

Further, a distal end of the plurality of electrode wires is fixedly connected to an outer wall of the distal end of the inner core tube.

Another aspect of the present application provides an energy delivery apparatus, comprising a guide pipe, an expandable structure, an intervention assembly and a handle, wherein one end of the guide pipe is connected to the handle, and the other end of the guide tube is connected to the expandable structure;
a cooling medium channel is formed in the guide pipe, one end of the cooling medium channel is configured for being communicated with a cooling medium source, and the other end of the cooling medium channel is communicated with the expandable structure.

The intervention assembly comprises a conveying line and an ablation electrode, one end of the conveying line is connected to the ablation electrode, the other end of the conveying line is configured to be connected to an ablation device, the ablation electrode is in a mesh shape, and the ablation electrode covers the expandable structure.

Further, the end of the guide pipe away from the handle is provided with a second position acquisition device.

Further, the guide pipe comprises an outer tube and an inner tube, and a cooling medium channel is formed between the outer tube and the inner tube.

Further, one end of the expandable structure close to the handle is connected to the outer tube, and the other end of the expandable structure is connected to the inner tube.

Further, the ablation electrode is a quadrilateral mesh structure.

Further, the ablation electrode is made by cutting, weaving, or electroforming.

Further, two ends of the ablation electrode are respectively connected to two ends of the expandable structure through connectors.

Further, the ablation electrode can expand with the expansion of the expandable structure and contract with the contraction of the expandable structure.

Further, the handle is provided with a communicating portion, and the communicating portion is respectively in communication with the cooling medium source and the cooling medium channel.

Further, the second position acquisition device is communicatively connected to the navigation control device.

In another aspect, the present application provides an adjustable bending guide tube, comprising a manipulation portion and a first layer tube and a second layer tube, wherein the first layer tube and the second layer tube are connected to the manipulation portion, the second layer tube is arranged in the first layer tube, and the second layer tube is in communication with the interior of the manipulation portion;
a second position acquisition device is arranged at the end, away from the manipulation portion, of the second layer tube, and the second position acquisition device is arranged on the outer wall of the second layer tube;

The manipulation portion is provided with a bending control member and an adjusting wire, one end of the adjusting wire is connected to the bending control member, and the other end of the adjusting wire is configured to be fixedly connected to the bending section of the second layer tube.

Further, a diameter of the first layer tube and a diameter of the second layer tube gradually decrease from a proximal end to a distal end.

Further, a conductive line is comprised, and the second position acquisition device is connected to the navigation control device through the conductive line.

Further, the bending section is disposed on the second layer tube, and a distance from the bending section to the distal end of the second layer tube is 2 -5 cm.

Further, a connecting structure is arranged on the outer side of the bending section, and the bending section is connected with the adjusting wire through the connecting structure.

Further, the bending control member is slidably or rotatably connected to the manipulation portion.

Further, the bending control member pulls the second layer of tube to bend by 0-180° through the adjusting wire.

Further, the adjusting wire is a round wire rope or a flat wire rope composed of a single strand or a plurality of strands of metal wires.

Further, an outer side of the adjusting wire is sleeved with a wire bending channel, and the wire bending channel is axially disposed along the second layer tube and is connected to the manipulation portion.

In another aspect, the present application provides a medical biopsy sampling device, comprising an adjusting portion, a guide tube, a sampling member, and a bending adjustment member; the guide tube is sleeved on the sampling member, the guide tube is connected to the adjusting portion, and the adjusting portion is provided with a first adjusting member and a second adjusting member;

One end of the sampling member is connected to the first adjusting member, and the other end of the sampling member is provided with a sampling head; one end of the bending adjustment member is connected to the second adjusting member, the other end of the bending adjustment member is connected to a bending portion of the guide tube, and the bending adjustment member can pull the guide tube to bend through the bending adjusting portion.

Further, the sampling member is provided with a second position acquisition device, the second position acquisition device is disposed close to the sampling head, and the second position acquisition device is configured to acquire position information of the sampling head and transmit the position information to the navigation control device.

Further, the bending portion is disposed on the guide tube and is close to the distal end of the guide tube.

Further, the bending adjustment member comprises a control wire, one end of the control wire is connected to the second adjusting member, and the other end of the adjusting wire is connected to the bending portion.

Further, the bending adjustment member comprises an adjustment tube, the adjustment tube is sleeved on the adjusting wire, and the adjustment tube is connected to the guide tube.

Further, a connecting member is provided at a bending portion of the guide tube, and the adjusting wire is connected to the bending portion through the connecting member.

Further, the first adjusting member and the second adjusting member are respectively slidably or rotatably connected to the adjusting portion.

Further, the curvature of the bending adjustment member pulling the guide tube is 0° to 120°.

Further, the second position acquisition device is connected to the navigation control device through a signal line.

In another aspect, this application provides an object ablation control method, where the method comprises:
receiving motion location information of a target object collected by a first location collection device and intervention location information of an intervention assembly collected by a second location collection device in a target intervention region, where the target intervention region belongs to the target object; and
performing a virtual model construction based on the regional image information of the target intervention region, the morphological attribute information of the intervention assembly, the movement position information and the intervention position information to obtain a navigation three-dimensional model and an operation object three-dimensional model, wherein the navigation three-dimensional model is configured for representing three-dimensional space features of the target intervention region, three-dimensional space features of the intervention assembly, and spatial position information between the to be ablated object, the intervention assembly and the target intervention region, and the operation object three-dimensional model is configured for representing a three-dimensional space feature of the to be ablated object;
determining a target ablation parameter corresponding to the to be ablated object according to the navigation three-dimensional model, the operation object three-dimensional model, and object attribute information of the to be ablated object; and
controlling the ablation device to operate based on the target ablation parameter, so that the intervention assembly performs ablation processing on the to be ablated object.

In another aspect, this application provides an object ablation control apparatus, where the apparatus comprises:
an information receiving module, configured to receive motion location information of a target object collected by a first location collection device and intervention location information of an intervention assembly collected by a second location collection device in a target intervention region, where the target intervention region belongs to the target object; and
model Construction Module : performing a virtual model construction based on region image information of a target intervention region, morphological attribute information of an intervention assembly, the movement position information, and the intervention position information to obtain a three-dimensional navigation model and an operation object three-dimensional model, wherein the navigation three-dimensional model is configured to represent three-dimensional space features of the target intervention region, three-dimensional space features of the intervention assembly, and spatial position information between the to be ablated object, the intervention assembly and the target intervention region, and the operation object three-dimensional model is configured to represent a three-dimensional space feature of the to be ablated object;
a parameter determining module, configured to determine a target ablation parameter corresponding to the to be ablated object according to the navigation three-dimensional model, the operation object three-dimensional model, and object attribute information of the to be ablated object; and
an object control module, configured to control the ablation device to operate based on the target ablation parameter, so that the intervention assembly performs ablation processing on the to be ablated object.

In another aspect, this application provides a pulse ablation effect evaluation method, including:
performing mapping modeling on the current tissue at the ablation catheter to obtain a three-dimensional model of the current tissue; and
obtaining to-be-evaluated data, where the to-be-evaluated data comprises detection data collected by an ablation catheter, ablation parameters of an ablation device, and basket catheter state data; and
inputting the to-be-evaluated data into a pre-trained ablation state evaluation model to obtain an ablation state evaluation result, wherein the ablation state evaluation model can calculate the ablation state evaluation result corresponding to the to-be-evaluated data according to the to-be-evaluated data and the correspondence between the to-be-evaluated data and the ablation state; and
displaying the ablation state evaluation result in the three-dimensional model of the current tissue.

Further, after inputting the to-be-evaluated data into a pre-trained ablation state evaluation model to obtain an ablation state evaluation result, the method comprises:
storing the to-be-evaluated data and the ablation state evaluation result into sample data to obtain updated sample data; and
training and updating the ablation state evaluation model based on the updated sample data.

Further, before inputting the to-be-evaluated data into a pre-trained ablation state evaluation model, the method comprises:
acquiring sample data;
training a pre-constructed machine learning model by using the sample data, and adjusting parameters of the machine learning model in a training process until the ablation state evaluation result output by the machine learning model meets a requirement; and storing the machine learning model as the ablation state evaluation model.

Further, the displaying the ablation state evaluation result in the three-dimensional model of the current tissue comprises:
determining a display color corresponding to the ablation state evaluation result according to the ablation state evaluation result and the correspondence between the ablation state evaluation result and the display color; and
displaying the ablation state evaluation result and/or the display color corresponding to the ablation state in the three-dimensional model of the current tissue, wherein the ablation state evaluation result comprises an ablation range and an ablation depth.

Further, the detection data comprises an impedance signal, and the obtaining the to-be-evaluated data comprises:
loading a first signal to an electrode of the ablation catheter, where a frequency range of the first signal is a first preset frequency range; and
collecting a feedback signal after the first signal acts on the electrode; and
filtering the feedback signal to convert the feedback signal into the impedance signal.

Further, the detection data comprises a dielectric constant signal, and the obtaining the to-be-evaluated data comprises:
applying a sinusoidal excitation signal to a current tissue through an electrode of the ablation catheter, wherein a frequency range of the sinusoidal excitation signal is a second preset frequency range; and
obtaining a complex impedance electrical signal returned after the sinusoidal excitation signal with different frequencies acts on the current tissue, and using the complex impedance electrical signal as the dielectric constant signal.

Further, the basket catheter state data comprises basket catheter opening state data and a contact area between the basket catheter and the current tissue, and the basket catheter opening state data and the abutment area are configured to determine a thickness of a region to be ablated.

In another aspect, this application provides an apparatus for evaluating a pulse ablation effect, including:
a model establishing module, configured to perform mapping modeling on the current tissue at the ablation catheter to obtain a three-dimensional model of the current tissue; and
a data acquisition module, configured to obtain to-be-evaluated data, where the to-be-evaluated data comprises detection data collected by an ablation catheter, ablation parameters of an ablation device, and basket catheter state data; and
a state evaluation module, configured to input the to-be-evaluated data into a pre-trained ablation state evaluation model to obtain an ablation state evaluation result, wherein the ablation state evaluation model can calculate the ablation state evaluation result corresponding to the to-be-evaluated data according to the to-be-evaluated data and the correspondence between the to-be-evaluated data and the ablation state; and
a result display module, configured to display the ablation state evaluation result in the three-dimensional model of the current tissue.

In another aspect, this application provides a computer-readable storage medium, where the storage medium stores at least one instruction or at least one program, and the at least one instruction or the at least one program is loaded and executed by a processor to implement the foregoing object ablation control method and pulse ablation effect evaluation method.

In another aspect, this application provides an electronic device, where the device comprises a processor and a memory, the memory stores at least one instruction or at least one program, and the at least one instruction or the at least one program is loaded and executed by the processor to implement the foregoing object ablation control method and pulse ablation effect evaluation method.

The implementation of the embodiments of the present disclosure has the following beneficial effects:
In the object ablation system of the present application, for a complex target intervention region, virtual model construction is performed based on the region image information of the target intervention region, to obtain a navigation three-dimensional model and an operation object three-dimensional model. The navigation three-dimensional model is configured to represent three-dimensional space features of the target intervention region, three-dimensional space features of the intervention assembly, and spatial position information between the to be ablated object, the intervention assembly, and the target intervention region. Navigation and positioning can be performed on the intervention assembly by using the navigation three-dimensional model, which provides important visual assistance for determining the position of the intervention assembly in the surgical process, so that the intervention assembly can reach a farther and narrow lesion position, so that the interventional device accurately reaches the treatment area to be uniformly attached to the lesion tissue, and the ablation effect is significantly optimized.

According to the present application, the simulation ablation model is constructed before ablation, so that an operator can intuitively observe the ablation range and ablation effect of the pulse energy generated based on the initial ablation parameter, and can intuitively help the operator to determine whether the initial ablation parameter needs to be adjusted, so as to optimize the initial ablation parameter and the ablation frequency to obtain the target ablation parameter, so as to generate more accurate pulse energy.

The switching circuit of the ablation device of the present application can implement several combinations of amplitude, pulse width, interval, quantity, or direction of the pulse energy by connecting to different branch circuits and controlling corresponding input terminal switch modules and output terminal switch modules on different branch circuits. By matching the control and filter modules of the corresponding input terminal switch module and the output terminal switch module on different branch circuits, the output of pulse energy of different frequencies and forms can be realized. Based on the switching circuit of the present application, the ablation device may generate more targeted pulse energy by having different voltage amplitudes, frequencies and pulse forms in a group of pulse energy, which helps to reduce the problem of stimulation reaction caused in the ablation treatment process while increasing the treatment effect, thereby improving the safety of treatment.

The navigation control device of this application further comprises an ablation three-dimensional model generation module, and the ablation three-dimensional model is configured to record ablation trajectory information and ablation data of the intervention assembly in the target intervention region. The ablation trajectory information and the ablation data of the intervention assembly can be reviewed and observed at any time by means of an ablation three-dimensional model, which provides a very important data support for the operator to determine which parts have been ablated and which parts are still not ablated, which can effectively improve the surgical efficiency and reduce the possibility of repeated ablation of the surgery.

The object ablation system of the present application combines the navigation control device, the ablation device, and the interventional device, thereby improving surgical accuracy, efficiency, therapeutic effect and therapeutic security.

The intervention assembly of the interventional device of the present application comprises at least one mesh-shaped ablation electrode, and the ablation electrode has a compact structure, good support, and uniform energy distribution, can better fit with a lesion tissue, and has a larger fitting area and a more uniform ablation. The two ends of the ablation electrode are fixed through the fixing sleeve with the inner tooth structure, the coaxiality is improved, the coaxial problem caused by bending after the distal end of the ablation electrode and the mandrel is fixed is greatly reduced, the connection is reliable, the problem of falling and cracking of the distal end of the ablation electrode in the operation process can be effectively avoided, the reliability of the interventional device is improved, and the treatment time is shortened.

The object ablation system of the present application combines the navigation control device, the ablation device, and the interventional device, thereby improving surgical accuracy, efficiency, therapeutic effect and therapeutic security.

The inner core tube of the medical interventional device of the present application is detachably provided with an electrode wire in a penetrating manner, and when the lesion tissue is in a narrower trachea, the electrode wire can be conveyed to a finer bronchus through the inner core tube to reach the lesion tissue, so as to perform ablation treatment on a narrower region.

The ablation electrode in the energy delivery device of the present application is in a mesh shape and covers the surface of the expandable structure, and the structure is compact, and the tensile performance and the structural stability are higher. After the expandable structure is controlled to expand, the fit area between the mesh ablation electrode and the target region is larger, more uniform, and the treatment effect is better. The guide pipe is a double-layer catheter, and has a cooling medium channel and a wire channel, so that the structure is more compact, and redundancy of the wire exposed outside is avoided. The energy delivery device can guide the expandable structure and the intervention assembly to accurately reach the target area through the cooperation of the second position acquisition device and the navigation control device.

The second position acquisition device of the adjustable bending guide tube of the present application is disposed at the distal end of the guide tube, and the guide tube may be positioned and navigated by the second position acquisition device. The guide tube further comprises a bending control member and an adjusting wire, and the bending of the guide tube can be controlled by the bending control member and the adjusting wire. The second position acquisition device is matched with the bending manipulation portion and the adjusting wire for controlling the bending of the guide tube to more accurately position the focus, so that the far end of the guide tube can accurately reach a farther and narrower lesion position, the diagnosis accuracy and the treatment accuracy are greatly improved, and diagnosis and treatment wounds are effectively reduced. The adjustable bending guide tube is simple in structure and easy and convenient to operate, and can be matched with other interventional instruments for accurate inspection or treatment such as sampling, microwave, ablation, radiotherapy and the like, which is high in practicability and convenient to popularize and produce. According to the medical biopsy sampling device of the present application, the first adjusting member for controlling the sampling member and the second adjusting member for controlling the bending portion are integrated in the adjusting portion, and are respectively adjusted by means of different adjustment channels, so that the operation is more convenient; and by means of the bending adjustment member, the distal end of the guide tube can be controlled to bend, and the bending radian is increased, so that the flexibility of the guide tube driving the sampling member to bend is increased, fine adjustment can be performed, and a lesion region can be more accurately reached and sampled. The sampling device is also provided with a second position acquisition device, and the positioning and navigation of the guide tube and the sampling head are performed by the second position acquisition device, so that the sampling head can be guided to reach the lesion area more accurately, and the sampling accuracy is improved.

According to the pulse ablation effect evaluation method and device, ablation state evaluation can be achieved in combination with a three-dimensional mapping modeling technology and a pulse electric field ablation technology, and the effectiveness of pulse electric field lung ablation treatment is further improved based on an ablation state evaluation result.

### BRIEF DESCRIPTION OF THE DRAWINGS

In order to more clearly illustrate the technical solutions of the present disclosure, the following briefly describes the accompanying drawings that need to be used in the description of the embodiments or the prior art. Obviously, the accompanying drawings in the following description are merely some embodiments of the present disclosure, and for a person of ordinary skill in the art, other drawings may be obtained according to these accompanying drawings without creative efforts.
FIG. 1 is a schematic structural diagram of an object ablation system according to the present application;
FIG. 2 is a schematic structural diagram of an interventional device according to this application;
FIG. 3 is a schematic structural diagram of an ablation electrode provided with a limiting structure according to an interventional device of the present application;
FIG. 4 is a schematic structural diagram of an interventional device including two ablation electrodes and two control tubes according to an embodiment of this application;
FIG. 5 is a schematic structural diagram of an interventional device including a plurality of ablation electrodes according to the present disclosure;
FIG. 6 is a cross-sectional view of an operating handle of an interventional device of the present disclosure;
FIG. 7 is a schematic structural diagram of a fixing sleeve of an interventional device according to this application;
FIG. 8 is a schematic structural diagram of an embodiment of an ablation electrode of an interventional device according to this application;
FIG. 9 is a schematic structural diagram of another embodiment of an ablation electrode of an interventional device according to this application;
FIG. 10 is a schematic structural diagram of unfolding an ablation electrode of an interventional device of the present application into an umbrella shape;
FIG. 11 is a schematic structural diagram of an interventional device including a plurality of cylindrical ablation electrodes according to the present disclosure;
FIG. 12 is a schematic structural diagram of a medical intervention apparatus according to this application;
FIG. 13 is a cross-sectional view of an inner core tube, a control outer tube and an electrode wire of the medical interventional device of the present application;
FIG. 14 is a schematic diagram of a state of an interventional trachea of a medical interventional device according to this application;
FIG. 15 is a top view of an energy delivery device according to the present disclosure;
FIG. 16 is a schematic structural diagram of an energy delivery apparatus according to this application;
FIG. 17 is a schematic structural diagram of a detail structure of an expandable structure and an intervention assembly of an energy delivery device of the present application;
FIG. 18 is a cross-sectional view of a guide pipe of an energy delivery device of the present application;
FIG. 19 is a schematic structural diagram of an adjustable bending guide tube according to the present application;
FIG. 20 is a schematic structural diagram of a second position acquisition device and a second layer tube distal end of an adjustable bending guide tube of the present disclosure;
FIG. 21 is a schematic structural diagram of a connection between an adjusting wire and a bending section of an adjustable bending guide tube of the present application;
FIG. 22 is a schematic structural diagram of a medical biopsy sampling apparatus according to the present application;
FIG. 23 is a schematic structural diagram of a sampling head and a second position acquisition device of a sampling device of the present disclosure;
FIG. 24 is a schematic structural diagram of a connection between a bending adjustment member and a bending portion of a sampling device of the present application;
FIG. 25 is a schematic structural diagram of a connection between a bending adjustment member and a bending portion in another embodiment of a sampling device according to the present disclosure;
FIG. 26 is a schematic structural diagram of a navigation control device of an object ablation system according to this application;
FIG. 27 is a schematic structural diagram of a target parameter determination module of an object ablation system according to the present disclosure;
FIG. 28 is a schematic diagram of simulation, evaluation and display of pulse energy of the present application on lung ablation effect;
FIG. 29 is a diagram of a relationship between a contact length of an intervention assembly and a bronchus and an inner diameter of a bronchus simulated by the present disclosure;
FIG. 30 is a diagram of a relationship between a contact length and a field intensity as simulated according to this application;
FIG. 31 is a graph showing the relationship between the contact length and the field strength with the increase of the wall thickness of the bronchus according to the present disclosure;
FIG. 32 is an ablation effect diagram of pulse energy in a simulated ablation model generated when a pulse voltage amplitude is 1500 V in the present application to lung tissue;
FIG. 33 is a pathological structural diagram of a bronchial tissue of the present application;
FIG. 34 is an ablation effect prediction diagram of pulse energy generated by different initial ablation parameters of the present application on a bronchial tissue mucosa layer;
FIG. 35 is a schematic module diagram of a control circuit of an ablation device according to this application;
FIG. 36 is a schematic module diagram of a branch circuit of an ablation device according to this application;
FIG. 37 is a flowchart of an object ablation control method according to this application;
FIG. 38 is a flowchart of a pulse ablation effect evaluation method according to the present application;
FIG. 39 is a connection block diagram of a pulse ablation effect evaluation system according to the present disclosure;
FIG. 40 is a schematic block diagram of a pulse ablation effect evaluation method according to this application;
FIG. 41 is an example of an ablation state evaluation model according to this application;
FIG. 42 is a structural block diagram of an apparatus for evaluating a pulse ablation effect according to this application;
FIG. 43 is a schematic structural diagram of a server according to this application.

Reference numbers :
1-interventional device, 11-second position acquisition device, 12-intervention assembly, 120-conveying line, 121-ablation electrode, 122-first ablation electrode, 123-second ablation electrode, 124-limiting structure, 13-mandrel, 14-manipulation assembly, 141-control tube, 142-first control tube, 143-second control tube, 15-fixing sleeve, 151-inner tooth structure, 16-operating handle, 161-channel, 162-fixing bayonet, 163-sealing ring, 17-manipulation assembly, 100-inner core tube, 101-control outer tube, 102-electrode wire, 103-fixing member, 104-operating handle, 105-control member, 20-guide pipe, 201-cooling medium channel, 202-outer tube, 203-inner tube, 204-wire channel, 21-expandable structure, 22-handle, 23-connector, 24-wire, 30-manipulation portion, 31-first layer tube, 32-second layer tube, 321-bending section, 33-conducting wire, 34-bending control member, 35-adjusting wire, 36-rail, 37-connecting structure, 38-wire bending channel, 40-adjusting portion, 401-first adjusting member, 402-second adjusting member, 41-guide tube, 411-bending portion, 42-sampling member, 421-sampling head, 43-bending adjustment member, 431-control wire, 432-adjustment tube, 44-connecting member, 45-guide sleeve, 46-signal line, 2-navigation control device, 021-intervention region sub-model construction module, 022-intervention assembly sub-model construction module, 023-fusion module, 024-navigation planning module, 025-initial parameter acquisition module, 026-target parameter determination module, 0261-simulation ablation model construction unit, 027-ablation three-dimensional model generation module, 028-pulse energy generation module, 3-first position acquisition device, 4-ablation device, 041-pulse power supply, 042-switch circuit, 043-output interface, 0421-branch circuit, 4211-input terminal switch module, 4212-output terminal switch module, 4213-capacitor module, 4214-diode module, 4215-filter module, 1000-electronic device, 1100-central processor, 1200-storage medium, 1210-operating system, 1220-data, 1230-application program, 1300-memory, 1400-input/output interface, 1500-wired or wireless network interface, 1600-power supply, 1700-display.

### DETAILED DESCRIPTION

The following clearly and completely describes the technical solutions in the embodiments of this application with reference to the accompanying drawings in the embodiments of this application. All other embodiments obtained by a person of ordinary skill in the art based on the embodiments of this application without creative efforts shall fall within the protection scope of this application.

It should be noted that the terms "first", "second", and the like in the specification, claims, and accompanying drawings of this application are configured to distinguish similar objects, and are not necessarily configured to describe a specific order or sequence. It should be understood that the data used in this way can be interchanged under appropriate circumstances, so that the embodiments of the present application described herein can be implemented in an order other than those illustrated or described herein. In addition, the terms "comprise" and "have" and any deformation thereof are intended to cover a non-exclusive inclusion, for example, a process, method, system, product, or device that comprises a series of steps or units is not necessarily limited to those steps or units listed clearly, but may comprise other steps or units that are not clearly listed or inherent to these processes, methods, products, or devices.

The present application provides an object ablation system, which can control an interventional device to accurately enter a target intervention region such as a tumor, a lumen and an organ through a navigation positioning function to form a relatively good abutment with a lesion tissue, and can generate a more accurate and more targeted pulse energy by performing an ablation effect evaluation on the ablation effect data before ablation and deliver the pulse energy to the lesion tissue for ablation treatment.

The subject ablation system of the present application may be configured to treat lung trachea and bronchial diseases, such as chronic bronchitis, chronic obstructive pulmonary disease, asthma, and the like. The lung bronchial tissue structure mainly comprises epithelial cells, an intrinsic layer and a mucous membrane bottom layer, wherein the epithelial cells are false multi-layer cells with cilia, cup-shaped cells are clamped between the false multi-layer cells, and the cup-shaped cells secrete a small amount of mucus; and the mucous membrane bottom layer mainly comprises smooth muscle and connective tissue, and the mucus gland located at the connective tissue secretes most of the mucus. Long-term production of excessive mucus and accumulation in the lungs is a major cause of multiple lung diseases. In the subject ablation system of the present application, the mucus glands in the cup-shaped cells and the mucous membrane bottom layer in the epithelial cells can be removed by means of pulse energy damage, thereby reducing the secretion of mucus in the bronchial cavity, and killing the failed long ciliated false multi-layer cells by means of pulse energy, so that the newborn epithelial cells can better help the tracheal mucus out and prevent the accumulation of mucus. The object ablation system of the present application can improve the operation accuracy, efficiency, treatment effect and treatment safety.

It should be noted that when the disease in the lung bronchus is treated, the target intervention region of the present application is the lung bronchial region of the target object, and the operation object is the lesion tissue region to be ablated in the lung bronchus.

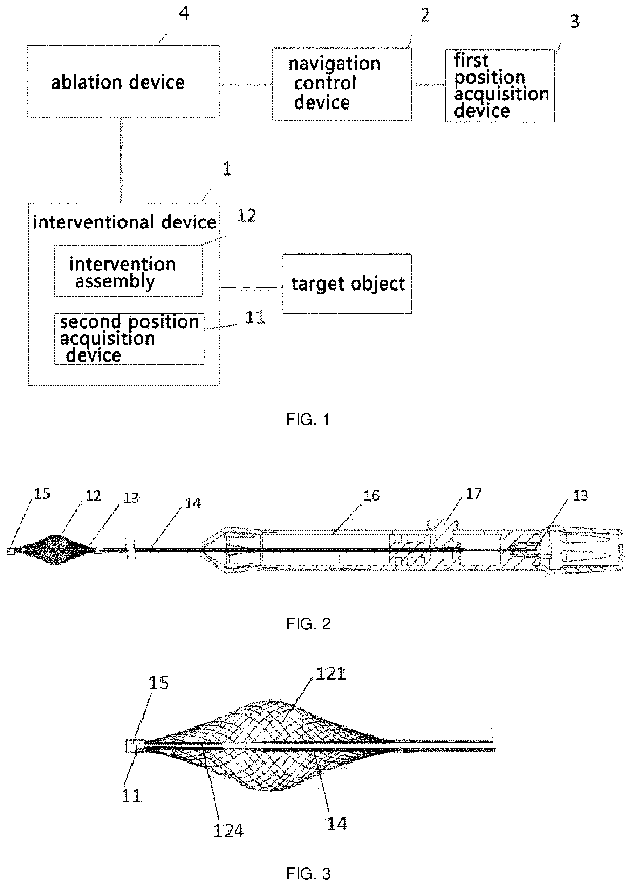

As shown in FIGS. 1-11, the present application provides an object ablation system, comprising an interventional device 1, a navigation control device 2, a first position acquisition device 3 and an ablation device 4, the interventional device 1 comprises a second position acquisition device 11 and an intervention assembly 12, the first position acquisition device 3, the second position acquisition device 11, the intervention assembly 12 and the ablation device 4 are communicatively connected to the navigation control device 2, respectively.

Specifically, the interventional device 1 is configured to deliver pulse energy, and intervene in the target object to perform ablation processing on the to be ablated object. In the present application, the interventional device 1 comprises a mandrel 13, a manipulation assembly 14, a second position acquisition device 11, and an intervention assembly 12, and the mandrel 13 is electrically connected to the intervention assembly 12 and the ablation device 4, respectively.

The second position acquisition device 11 is disposed on the mandrel 13 and/or the intervention assembly 12.

The at least one ablation electrode 121 may be a mesh, the at least one ablation electrode 121 is sequentially disposed along the mandrel 13, the manipulation assembly 14 is sleeved on the mandrel 13, and the manipulation assembly 14 can move relative to the mandrel 13 to drive the intervention assembly 12 to expand or contract.

In some embodiments, the second position acquisition device 11 may be disposed at any position on the mandrel 13 or the intervention assembly 12, and the number of the second position acquisition devices 11 is not limited. The more second position acquisition devices 11, the more comprehensive real-time positioning and presentation of real-time changes of the interventional device 1 in the air pipe, and the positions of the interventional devices in the air pipe are expressed from different dimensions. Preferably, the second position acquisition device 11 is disposed at the distal end of the mandrel 13 or the intervention assembly 12. The second position acquisition device 11 is configured to acquire the intervention position information of the intervention assembly 12 in the target intervention region, and transmit the intervention position information to the navigation control device 2 through the signal line, where the target intervention region belongs to the target object. In some possible implementations, the second position acquisition device 11 may be a magnetic induction sensor, and the magnetic induction sensor may obtain the intervention position information of the intervention assembly 12 in the target intervention region through a magnetic field; in some other possible implementations, the second position acquisition device 11 may be an electric induction sensor, and the electric induction sensor may obtain, by using an electric field, the intervention position information of the intervention assembly 12 in the target intervention region. Preferably, the second position acquisition device 11 comprises at least one five-degree-of-freedom magnetic positioning sensor or a six-degree-of-freedom magnetic positioning sensor.

It should be noted that, in some embodiments, the mandrel 13 may be an electrode line connected to the ablation device 4 and the intervention assembly 12, respectively, and is configured to transfer ablation energy to the intervention assembly 12. In some other embodiments, the mandrel 13 may be a stainless steel spring tube, a hypotube, a spiral tube, or the like, and electrode lines respectively connected to the ablation device 4 and the intervention assembly 12 are disposed in the mandrel 13. The electrode wire may be a single strand or a plurality of strands, the wire diameter may be 0.05 to 1 mm, and the material of the electrode wire is a conductive metal material. In some embodiments, the outer layer of the mandrel 13 is provided with a polytetrafluoroethylene coating or a non-coating layer.

Specifically, the manipulation assembly 14 comprises at least one control tube 141 connected to the ablation electrode 121. In some possible implementations, the intervention assembly 12 comprises an ablation electrode 121, the manipulation assembly 14 comprises a control tube 141, a distal end of the ablation electrode 121 is connected to a distal end of the mandrel 13, a proximal end of the ablation electrode 121 is fixedly connected to an outer wall of the control tube 141, and a distal end of the control tube 141 is spaced apart from a distal end of the mandrel 13. In some other possible implementations, the intervention assembly 12 comprises a first ablation electrode 122 and a second ablation electrode 123, the first ablation electrode 122 and the second ablation electrode 123 are sequentially arranged along the mandrel 13, the manipulation assembly 14 comprises a first control tube 142 and a second control tube 143 that are sequentially sleeved, a distal end of the first ablation electrode 122 is connected to a distal end of the mandrel 13, a proximal end of the first ablation electrode 122 is fixedly connected to an outer wall of the first control tube 142, and a distal end of the first control tube 142 is spaced apart from a distal end of the first ablation electrode 122 by controlling the first control tube 142 to move; A distal end of the second ablation electrode 123 is connected to an outer wall of the first control tube, the outer wall is spaced from a distal end of the first control tube by a certain distance, a proximal end of the second ablation electrode 123 is fixedly connected to an outer wall of the second control tube 143, and the second ablation electrode can be controlled to expand or contract by controlling movement of the second control tube.

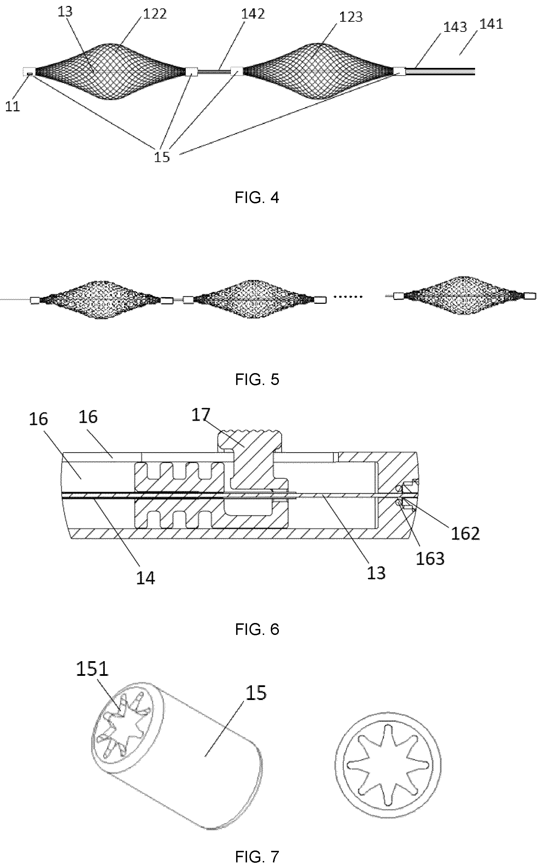

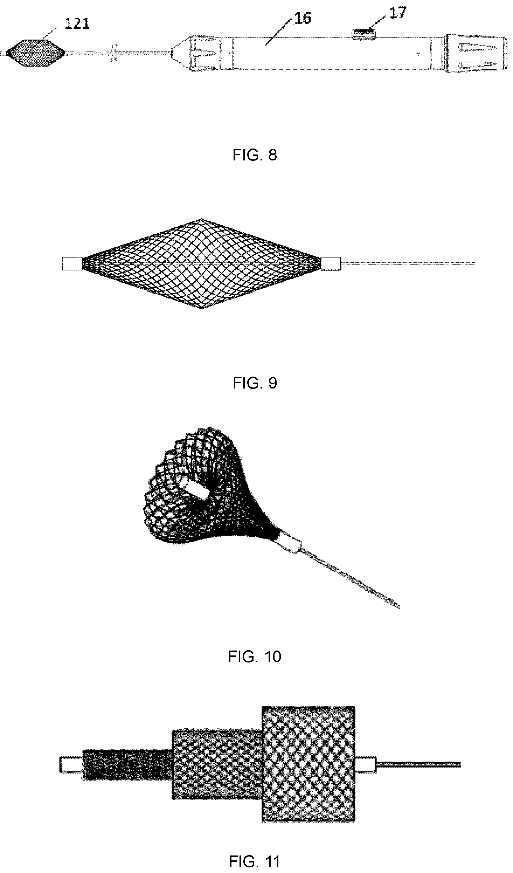

As shown in FIG. 5, in some possible implementations, the intervention assembly 12 comprises a plurality of ablation electrodes 121, and the plurality of ablation electrodes 121 may be opened at the same maximum distance. As shown in FIG. 11, in some other possible implementations, the plurality of ablation electrodes 121 sequentially disposed along the mandrel 13 may be sequentially increased in a direction from a distal end to a proximal end of the mandrel 13. Therefore, the ablation electrodes 121 with different diameters can be controlled to open through the manipulation assembly according to the change of the inner diameter of the trachea to adapt to air pipes with different inner diameter sizes. Specifically, the at least one ablation electrode 121 can be configured as a unipolar electrode or a bipolar electrode. In some possible implementations, the at least one ablation electrode 121 releases a unipolar pulse, a negative plate is attached to the body surface of the target object, and a loop is formed in the body through the combination of the negative plate and the product, so that the pulse energy acts on the treatment region. In some other possible implementations, different ablation electrodes 121 constitute positive and negative electrodes in the target object, positive and negative energy transfer, and form a closed-loop treatment region for the energy ranges in the range of the positive and negative poles and the two poles. The circuit of the ablation electrode 121 at the two ends is controlled to conduct, a larger range of ablation areas can be obtained, the circuit of the two adjacent ablation electrodes 121 is controlled to conduct, the ablation range can be controlled between two adjacent ablation electrodes 121, the treatment range is more accurate, and the stimulation reaction is smaller.

In some possible implementations, the intervention assembly 12 comprises an ablation electrode 121, the ablation electrode 121 is electrically connected to a connection port in the ablation device through a plurality of electrode lines, and the connection port comprises a negative port and seven positive ports. The negative port is configured to connect to a negative plate attached to the body surface of the target object, the other seven positive ports are respectively connected to different electrode lines, and the electrode lines are connected to the ablation electrode 121. For example, the first electrode line is connected to the first port, the second electrode line is connected to the second port, the third electrode line is connected to the third port, the fourth electrode line is connected to the fourth port, the fifth electrode line is connected to the fifth port, the sixth electrode line is connected to the sixth port, and the seventh electrode line is connected to the seventh port. Each electrode connection port may be separately controlled by a user, and the on-off of the line is controlled according to the ablation area and range.

In some embodiments, the ablation electrode 121 comprises a plurality of electrode lines, the plurality of electrode lines are cross-woven into a mesh-shaped ablation electrode 121, the ablation electrode 121 may be formed by weaving a conductive metal wire, and the material of the metal wire may be a material with good conductive performance such as stainless steel, a nickel-titanium alloy, a cobalt-chromium alloy, and the like. The longitudinal section of the metal wire may be oval, circular or polygonal, so that the braided ablation electrode 121 may be more fully attached to the target position. The mesh ablation electrode 121 is easily changed in shape, can expand and contract, has a higher mesh tightness, a higher tensile property and a higher structural stability, can be better attached to a target position, has a larger fitting area, is more uniform, and has a better treatment effect.

In some embodiments, as shown in FIGS. 8-11, the axial cross section of the ablation electrode 121 after expansion may be a spindle shape, a polygon, an ellipse, or an umbrella shape. When the proximal end of the ablation electrode 121 is far away from the distal end of the ablation electrode 121, the ablation electrode 121 contracts into a cylinder coaxial with the mandrel 13 to facilitate movement in the narrow channel; and when the proximal end of the ablation electrode 121 is driven by the control tube to move in a direction close to the distal end of the ablation electrode 121, the ablation electrode 121 may be expanded to an axial section with an elliptical shape, a spindle shape, a polygonal shape, or an umbrella-shaped mesh body, so as to increase a fitting area with different inner diameter bronchial and special shape to be ablated objects. In some possible implementations, the plurality of ablation electrodes 121 are sequentially disposed along the mandrel 3, the maximum diameter of the plurality of ablation electrodes 121 may be sequentially increased in a direction from the distal end to the proximal end of the mandrel 13, and the ablation electrodes 121 with different diameters may be controlled to expand according to the inner diameter of the inlet pipe to adapt to different inner diameter sizes. When the air pipe is particularly small, only the ablation electrode 121 with the smallest distal diameter can be controlled to open, and the cylindrical structure with the rectangular axial section is more suitable for the air pipe with smooth path.

In some embodiments, an insulating layer is disposed on an outer side of the ablation electrode 121, and the insulating layer is configured to increase insulation performance of the ablation electrode 121.

In some embodiments, the control tube 141 may be an extrusion tube such as PI (polyimide), PET (polyethylene terephthalate), PEBAX (block polyether amide resin) or PTFE (polytetrafluoroethylene), or a PI/PTFE composite tube. The outer diameter of the control tube 141 may preferably be 0.5 to 5 mm, the wall thickness of the control tube 141 may preferably be 0.025 to 0.5 mm, and the length may be preferably 40 cm to 80 cm.

In some embodiments, as shown in FIG. 3, the ablation electrode 121 is internally provided with a limiting structure 124, and the limiting structure 124 is spaced apart from a distal end of the control tube 141. In some possible implementations, the limiting structure may be a limiting tube disposed in the ablation electrode 121, the limiting tube is spaced apart from the distal end of the control tube 141, and when the distal end of the control tube 141 abuts against the limiting tube, the ablation electrode 121 is opened to the maximum. In some possible implementations, when the abutting position of the limiting structure 124 and the distal end of the control tube 141 is located at the middle position when the ablation electrode 121 is expanded to the maximum, the overall stability of the ablation electrode 121 is better, and the fitting degree of the ablation electrode 121 is better than that of the air tube. The maximum radial opening distance of the ablation electrode 121 may be adjusted by adjusting the length of the limiting tube. In some possible implementations, when the ablation electrode 121 is opened, the opening direction of the ablation electrode 121 may be limited through the limiting structure 124. In general, when the limiting structure 124 is close to the distal end of the ablation electrode 121, the ablation electrode 121 may be opened to be umbrella-shaped, and the opening direction of the opening may also be at the distal end, so that when the limiting structure 124 is in the short trachea, the ablation electrode 121 may increase the fitting area with the air pipe in this expansion manner, and the umbrella-shaped structure may also conform to a narrow air pipe, so that the inner wall of the air pipe cannot be damaged due to an excessively large opening amplitude of the ablation electrode 121.

Specifically, the interventional device 1 further comprises a fixing sleeve 15, and the fixing sleeve 15 is fixedly connected to an end of the ablation electrode 121 to fix an end of the ablation electrode 121. The two ends of the sleeve body of the fixing sleeve 15 are provided with an inner tooth structure 151 in the circumferential direction, and both inner tooth structures 151 are obliquely arranged from the end of the sleeve body to the inner side of the end face. In some possible implementations, the tooth shape of the inner tooth structure (151) may be wave teeth, triangular teeth, flat teeth, trapezoidal teeth, or the like. Two ends of the ablation electrode 121 are fixedly connected to the mandrel 13 or the manipulation assembly 14 through the fixing sleeve 15. The inner tooth structure (151) of the fixing sleeve 15 can better fix the mesh ablation electrode 121, and the arrangement of the inner tooth structure (151) enables the openings at the two ends of the fixing sleeve 15 to become smaller, thereby improving the coaxiality, and greatly reducing the coaxial problem caused by bending the distal end of the ablation electrode 121 and the mandrel 13. In some possible implementations, the second position acquisition device 11 may be placed between the two inner tooth structures (151) of the fixing sleeve 15 to reduce the space occupied by the installation of the second position acquisition device 11. The two ends of the ablation electrode 121 are fixed through the fixing sleeve 15, the coaxiality and connection reliability of the two ends of the ablation electrode 121 are improved, the ablation electrode 121 is prevented from falling off in the operation process, the reliability of the interventional device is improved, the treatment time is shortened, and the placement space can be reserved for the second position acquisition device 11.

Specifically, the interventional device 1 further comprises an operating handle 16, the operating handle 16 is internally provided with a operating handle capable of accommodating the mandrel 13 and the operating handle 16 passing through the manipulation assembly 14 as an interventional device, and the mandrel 13 and the signal line of the interventional device may be integrated into one body, penetrate out of the channel 161 of the operating handle 16, have a compact structure, and avoid redundancy of the signal line exposed outside. In some possible implementations, the proximal end of the channel 161 is provided with a fixing bayonet 162, the fixing bayonet 162 is connected to the proximal end of the mandrel 13, the fixing bayonet 162 is configured to fix the inner side of the fixing bayonet 162 of the mandrel 13, a sealing ring 163 may be provided, and the sealing ring 163 is configured to improve the sealing performance and the conductive safety of the operating handle 16. In some embodiments, the operating handle 16 may be formed by 3D printing or injection molding of a material such as plastic, nylon, or silica gel. The operating handle 16 is provided with a manipulation assembly 17, the manipulation assembly 17 is slidably or rotatably connected to the operating handle 16, and the other end of the manipulation assembly 17 is fixedly connected to the proximal end of the manipulation assembly 14. In some possible implementations, one end of the manipulation assembly 17 is configured to be a sliding block slidably connected to an outer wall of the operating handle 16, the other end of the manipulation assembly 17 is configured to be a plunger portion slidably connected to an inner wall of the operating handle 16, and the plunger portion is fixedly connected to a proximal end of the manipulation assembly 14. In some other possible embodiments, the manipulation assembly 17 may be configured as a knob, the knob inner ring is in threaded connection with the manipulation assembly 14, and rotation of the knob may drive the manipulation assembly to move axially. In this application, the manipulation assembly 17 may control the manipulation assembly 14 to move relative to the mandrel 13 to control the expansion degree of the intervention assembly 12.

Specifically, the interventional device 1 further comprises a contact detection device and a dielectric constant detection device, and the contact detection device and the dielectric constant detection device are communicatively connected to the navigation control device 2, respectively. The abutting detection device is configured to detect impedance data of the to be ablated object and an abutment parameter of the intervention assembly 12. The impedance data is configured to indicate a load of the intervention assembly 12, and the abutment parameter is configured to indicate a degree of contact between the intervention assembly 12 and the to be ablated object; and the impedance data is sent to the initial parameter acquisition module 025; and the dielectric constant detection device is configured to detect a dielectric constant of the to be ablated object. In some embodiments, the detection device and the dielectric constant detection device may be disposed on an outer wall of the intervention assembly 12.

In the present application, the above interventional device is configured to treat a disease in a pulmonary bronchus region: The mandrel 13 and the manipulation assembly are controlled by the operating handle 16 to feed the intervention assembly 12 in the contracted state into the trachea; When the intervention assembly reaches the target lesion tissue area, the opening degree of the ablation electrode 121 is controlled through the manipulation assembly 17 according to the inner diameter of the air pipe, so that the ablation electrode 121 is evenly attached to the lesion tissue; the ablation energy is sent through the ablation equipment connected with the mandrel 13, and the ablation electrode 121 releases ablation energy to perform ablation treatment on the lesion tissue; and the time and frequency of ablation treatment are controlled and operated for lesion types and different treatment schemes of different target areas and the size and type of the delivered ablation energy.

It should be noted that the object ablation system of the present application further comprises a first position acquisition device 3, the first position acquisition device 3 is configured to acquire movement position information of a target object and transmit the movement position information to the navigation control device 2, the movement position information of the target object in the present application may be breathing state information of the target object, and the first position acquisition device 3 may be a magnetic positioning electrode placed on the surface of the body of the target object or surrounding the chest activity information when the target object is breathing. In some possible implementations, the first position acquisition device 3 comprises three or six electrodes including a unique form of a built-in magnetic positioning sensor, and is placed on the front chest and/or the back of the patient. When the target object inhales, the first position acquisition device 3 moves upward along with the chest, and moves downward along with the chest when the target object exhales. The chest movement of the target object in the magnetic positioning signal field may be captured by the first position acquisition device 3, and by means of the movement position information of the target object provided by the first position acquisition device 3, the intervention position information of the intervention assembly 12 provided by the second position acquisition device 11 in the target intervention region may be compensated and calculated, and the compensation calculation may eliminate respiratory interference experienced by the second position acquisition device 11 during the movement process, so as to acquire more accurate intervention position information.

It should be noted that the object ablation system of this application further comprises a positioning generation device, and the positioning generation device is communicatively connected to the navigation control device. The positioning generation device is configured to form a multi-dimensional positioning signal field in the working area, and when the first position acquisition device and the second position acquisition device move within the positioning signal field, position information of the corresponding three-dimensional space may be acquired.

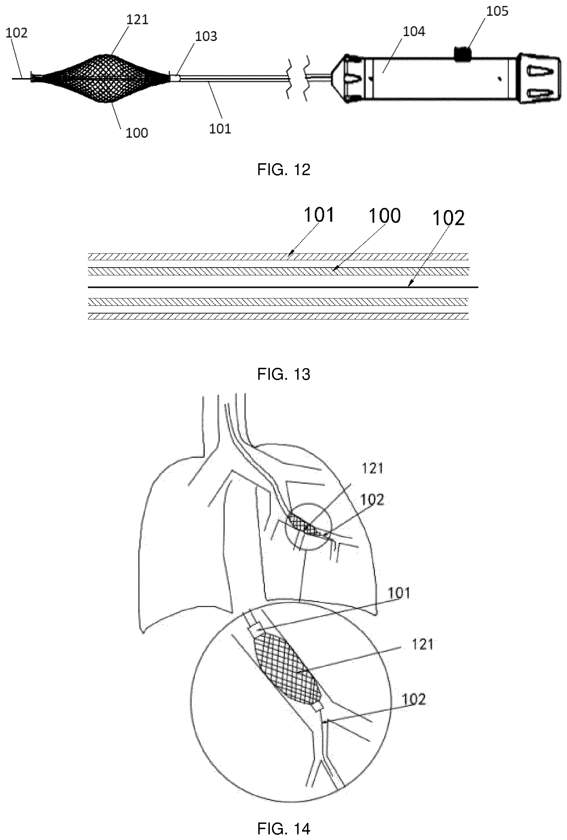

In some embodiments, as shown in FIGS. 12-14, the interventional device of the present application may also be a medical intervention device, and the medical intervention device is also configured for accurately entering a target intervention region such as a tumor, a lumen, and an organ, forming a good abutment with the lesion tissue, connecting to the navigation control device and the ablation device, delivering ablation energy generated by the ablation device and applying the ablation energy to the lesion tissue, so as to implement ablation treatment.

The medical intervention device comprises an inner core tube 100, a control outer tube 101, an electrode wire 102 and the ablation electrode 121, the electrode wire 102 is detachably arranged in the inner core tube 100 in a penetrating manner, and the control outer tube 101 is sleeved on the outer side of the inner core tube 100; A distal end of the ablation electrode 121 is fixedly connected to a distal end of the inner core tube 100, a proximal end of the ablation electrode 121 is fixedly connected to an outer wall of the control outer tube 101, and the control outer tube 101 can move along the inner core tube 100 to drive the ablation electrode 121 to expand or contract.

Specifically, both the electrode wire 102 and the ablation electrode 121 are electrically connected to the ablation device 4. In some embodiments, the inner core tube 100 may be a tubular structure such as a spring tube, a hypotube or a hypotube spiral tube, an electrode wire passes through the inner core tube 100, the ablation electrode 121 is electrically connected to the ablation device through an electrode wire, the ablation energy is transmitted to the ablation electrode 121, the electrode wire may be a single strand or a plurality of strands, the wire diameter of the electrode wire may be 0.05 to 1 mm, and the material of the electrode wire is a conductive metal material. In some possible implementations, when the ablation electrode 121 enters the trachea for ablation treatment, after the target lesion tissue is found, the ablation electrode 121 may be controlled to be opened by pushing the control outer tube 101 according to the size of the inner diameter of the trachea, so as to be uniformly attached to the lesion tissue.

In some embodiments, the inner core tube 100 is a through tube, and the distal end of the electrode wire 102 can pass through the distal end of the inner core tube 100 to be in contact with the lesion tissue. Preferably, the electrode wire may be a conductive wire, and the material of the metal wire may be preferably made of stainless steel, nickel-titanium alloy, cobalt-chromium alloy, or the like with good conductivity. When ablation treatment is performed on a narrower trachea, the electrode wire 102 can be inserted from the operating handle end, and the electrode wire 102 penetrates out of the distal end of the inner core tube 100 to reach the target lesion tissue, so as to perform ablation treatment on the lesion tissue.

Specifically, the ablation electrode 121 comprises a plurality of electrode lines, and the plurality of electrode lines are cross-woven into a mesh. Preferably, the electrode line may be a conductive metal wire, and the material of the metal wire may be a material with good conductivity, such as stainless steel, nickel-titanium alloy, cobalt-chromium alloy, and the like. The longitudinal section of the metal wire may be oval, circular or polygonal, so that the braided ablation electrode 121 may be more fully attached to the lesion tissue. The mesh ablation electrode 121 is easily changed in shape, can be expanded and contracted, has a higher mesh tightness, a higher tensile property and a higher structural stability, can be better attached to a lesion tissue, has a larger bonding area, is more uniform, and has a better treatment effect. The axial section of the ablation electrode 121 after expansion of the present application may be elliptical, spindle-shaped, polygonal, or umbrella-shaped. When the proximal end of the ablation electrode 121 is far away from the distal end of the ablation electrode 121, the ablation electrode 121 contracts into a cylinder coaxial with the inner core tube 100, so as to facilitate movement in the narrow channel; and when the proximal end of the ablation electrode 121 is driven by the control outer tube 101 to move in a direction close to the distal end, the ablation electrode 121 may be expanded to an axial section with an elliptical shape, a spindle shape, a polygonal shape, or an umbrella-shaped mesh body, so as to increase a fitting area with different inner diameter bronchus and special shape lesion tissue.

In some embodiments, an electrode line of the ablation electrode 121 is electrically connected to a connection port in the ablation device. For example, in some possible implementations, the connection port comprises a negative port and a plurality of positive ports. The negative port is configured to be connected to the negative electrode plate attached to the body surface of the target object, one end of the electrode wire 102 is connected to the negative electrode port, and the other positive electrode ports are respectively connected to different electrode lines. Each electrode connection port may be separately controlled by a user, and the on-off of the line is controlled according to the ablation area and range.

In some embodiments, the ablation electrode 121 comprises a plurality of electrode lines, the plurality of electrode lines may be expanded into a lantern-shaped structure, and the lantern-shaped structure may be attached to different inner diameter bronchus and special shape lesion tissues.

Specifically, the distal ends of the plurality of electrode lines are fixedly connected to the outer wall of the distal end of the inner core tube 100. In some possible implementations, the distal ends of the plurality of electrode wires may be fixedly connected to the outer wall of the distal end of the inner core tube 100 by welding, hot melting or bonding.

Specifically, the interventional device further comprises a fixing member 103, and the fixing member 103 is configured to fix the proximal end of the ablation electrode 121. The two ends of the sleeve body of the fixing member 103 are provided with an inner tooth structure in the circumferential direction, and both inner tooth structures are obliquely arranged from the end of the sleeve body to the inner side of the end face. The tooth shape of the internal tooth structure may be a wave tooth, a triangular tooth, a flat tooth, or a trapezoidal tooth. The inner tooth structure can better fix the mesh ablation electrode 121, and the arrangement of the inner tooth structure enables the openings at the two ends of the tube body of the fixing member 103 to become smaller, thereby improving the coaxiality. In some possible implementations, the second position acquisition device 11 is disposed between the inner tooth structures at the two ends of the fixing member 103, and the second position acquisition device 11 is configured to acquire the intervention position information of the ablation electrode 121 in the target intervention region, and transmit the intervention position information to the external navigation control device 2 through the signal line, and the second position acquisition device 11 is disposed inside the fixing member 103, so that the space occupied by the installation of the second position acquisition device 11 can be greatly reduced. In this application, the ablation electrode 121 is fixed through the fixing member 103, so that the connection reliability of the ablation electrode 121 is improved, the ablation electrode 121 is prevented from falling off in the operation process, the reliability of the intervention device is improved, the treatment time is shortened, and a placement space can be reserved for the second position collection device. In some embodiments, the second position acquisition device may be disposed at any position of the inner core pipe 100 or the ablation electrode 121, and the number of the second position acquisition devices is not limited. The more the second position acquisition devices, the more comprehensive real-time positioning and presentation of the real-time change of the interventional device in the air pipe, and express the positions of the products in the air pipe from different dimensions.

In some embodiments, an insulating layer is disposed on an outer side of the ablation electrode 121, and the insulating layer is configured to increase insulation performance of the ablation electrode 121.

In some embodiments, the control outer tube 101 may be a PI, PET, PEBAX or PTFE extrusion tube, or a PI/PTFE composite tube. The outer diameter of the control outer tube 101 is preferably 0.5 to 5 mm, the wall thickness of the control outer tube 101 is preferably 0.025 to 0.5 mm, and the length is preferably 40 to 80 cm.

Specifically, the interventional device of this application further comprises an operating handle 104, and the operating handle 104 is internally provided with a channel through which the inner core tube 100 and the control outer tube 101 pass. The inner core tube 100, the electrode wire 102, and the signal line of the interventional device may be integrated as a whole, penetrate out of the operating handle 104, have a compact structure, and avoid redundancy of the signal line exposed outside. In some embodiments, the proximal end of the channel is provided with a fixing bayonet, the fixing bayonet is connected with the proximal end of the inner core tube 100, the fixing bayonet is configured for fixing the inner side of the fixing bayonet of the inner core tube 100, a sealing ring can be arranged, and the sealing ring is configured for improving the sealing performance and the conductive safety of the operating handle 104. In some embodiments, the operating handle 104 may be formed by 3D printing or injection molding of a material such as plastic, nylon, or silica gel.

Specifically, the operating handle 104 is provided with a control member 105, the control member 105 is slidably or rotatably connected to the operating handle 104, and the control member 105 is configured to control the control outer tube 101 to move. In some possible implementations, one end of the control member 105 is slidably or rotatably connected to the operating handle 104, and the other end of the control member 105 is fixedly connected to a proximal end of the control outer tube 101. In some possible implementations, one end of the control member 105 is configured to be a sliding block slidably connected to an outer wall of the operating handle 104, the other end of the control member 105 is configured to be a plunger portion slidably connected to an inner wall of the operating handle 104, and the plunger portion is fixedly connected to a proximal end of the control outer tube 101. In some other possible embodiments, the control member 105 may be configured as a knob, the knob inner ring is in threaded connection with the operating handle 104, and the control outer tube 101 may be driven to move axially by rotating the knob. By means of the control member 105, the control outer tube 101 can be controlled to move relative to the inner core tube 100 to control the opening degree of the expandable electrode 3.

The above interventional device is applied in the present application: the inner core tube 100 and the control outer tube 101 are controlled by the operating handle 104 to feed the ablation electrode 121 in the contracted state into the air tube; When the ablation electrode 121 reaches the target lesion tissue region, the opening degree of the ablation electrode 121 is controlled by the control member 105 according to the inner diameter of the air tube, so that the ablation electrode 121 is uniformly attached to the lesion tissue; When the lesion tissue is in a narrower trachea, the ablation electrode 121 is firstly contracted to reach the smallest bronchus that the lesion tissue can reach, then the electrode wire 102 penetrates into the inner core tube 100 at the end of the operating handle 104, and extends from the distal end of the inner core tube 100 to be conveyed into a finer bronchus to reach the lesion tissue; The ablation energy is sent by an ablation device electrically connected to the electrode wire 102 and the ablation electrode 121, and the electrode wire 102 and/or the ablation electrode 121 release ablation energy to perform ablation treatment on the lesion tissue; the time and frequency of ablation treatment are controlled and operated for lesion types and different treatment schemes of different target areas and the size and type of the delivered ablation energy.

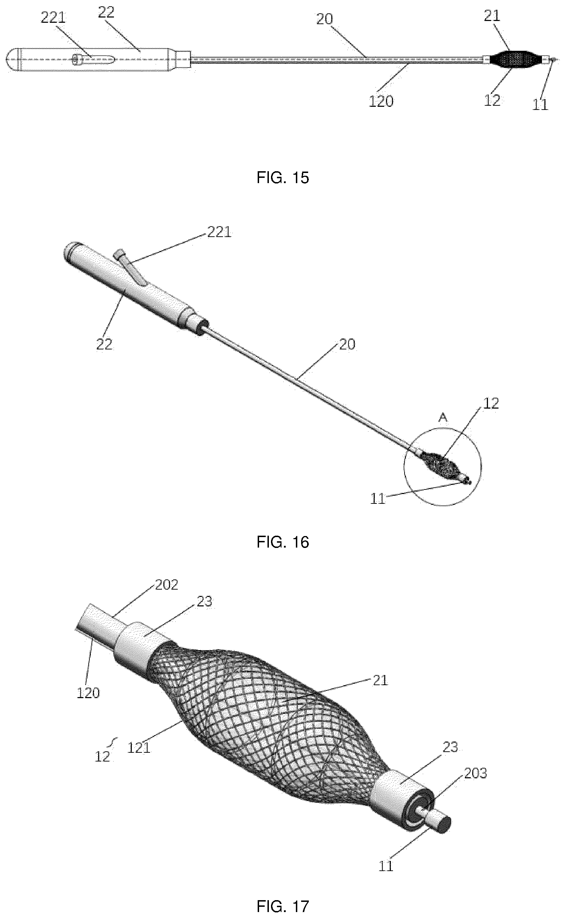

In some embodiments, as shown in FIGS. 15-18, the interventional device of the present application may also be an energy delivery device, which is configured to accurately enter a tissue region such as a tumor, a lumen, and an organ, can form a better abutment with the target region, is connected to the navigation control device and the ablation device, performs navigation and accurate positioning by cooperating with the navigation control device, applies pulse energy to the target region by means of the ablation device and the intervention assembly, implements pulse ablation, and does not damage surrounding normal tissues.

The energy delivery device of the present application comprises a guide pipe 20, an expandable structure 21, an intervention assembly 12 and a handle 22, wherein one end of the guide pipe 20 is connected to the handle 22, and the other end of the guide pipe 20 is connected to the expandable structure 21. The guide pipe 20 is configured for connecting the handle 22 and the expandable structure 21 to form a cooling medium channel 201, one end of the cooling medium channel 201 is configured for communicating with the cooling medium source, and the other end of the cooling medium channel 201 is in communication with the expandable structure 21.

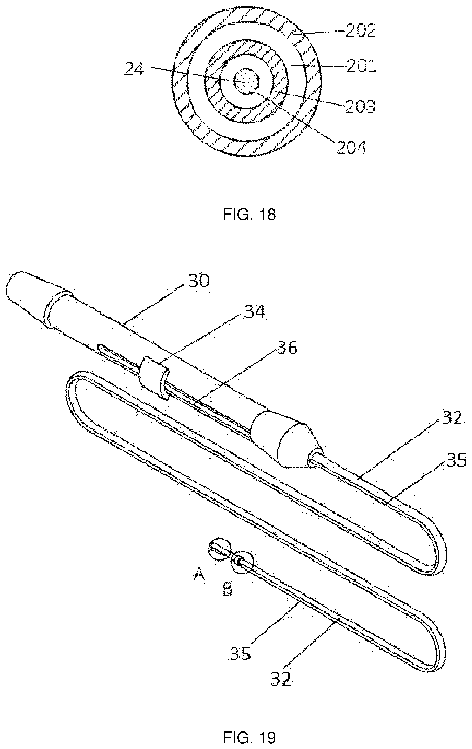

In some embodiments, a cooling medium channel 201 is formed inside the guide pipe 20, and the cooling medium enters the expandable structure 21 through the cooling medium channel 201 inside the guide pipe 20. As shown in FIG. 18, the guide pipe 20 comprises an outer tube 202 and an inner tube 203, a cooling medium channel 201 is formed between the outer tube 202 and the inner tube 203 to form a wire channel 204, the guide pipe 20 is configured as a double-layer catheter, the inner tube 203 is hollow, and a cooling medium channel 201 and a wire channel 204 are formed at the same time, so that the structure is more compact.

Specifically, one end of the expandable structure 21 close to the handle 22 is connected to the outer tube 202, and the other end of the expandable structure 21 is connected to the inner tube 203. A length of the inner tube 203 is greater than that of the outer tube 202, one end of the expandable structure 21 close to the handle 22 is connected to the outer tube 202, the other end of the expandable structure 21 is connected to the inner tube 203, the cooling medium is input through one end of the expandable structure connected to the outer tube, one end of the expandable structure 21 connected to the inner tube 203 is a closed end, and the cooling medium does not flow out. After the cooling medium enters the expandable structure 21 through the cooling medium channel 201 located between the outer tube 202 and the inner tube 203, the cooling medium may return to the original path through the cooling medium channel 201. The input and output channels of the cooling medium of the present application are the same channel, which facilitates precise control of the volume of the cooling medium in the input expandable structure 21 while simplifying the structure, thereby controlling the degree of expansion of the expandable structure 21. The expandable structure 21 expands with the input of the cooling medium and contracts with the output of the cooling medium, so that the energy delivery assembly is better attached to and separated from the target area.

In some embodiments, the guide pipe 20 may be a sheath. Specifically, it may be a PEBAX tube or a nylon tube. The outer diameter of the outer tube 202 of the guide pipe 20 is 1 -5 mm, the wall thickness of the outer tube 202 is 0.025 -0.5 mm, and the length is at least 40 cm; the inner diameter of the inner tube 203 is 0.5 -3 mm, the wall thickness is 0.025 -0.5 mm, and the length is at least 40 cm. For example, the outer diameter of the outer tube 202 is 4 mm, the wall thickness is 0.2 mm, and the length is 60 cm; the inner diameter of the inner tube 203 is 1.6 mm, the wall thickness is 0.2 mm, and the length is 68 cm. In some other embodiments, the guide pipe 20 is connected to the expandable structure 21 and the intervention assembly for long enough to extend from the outside of the body to the target region for pulse ablation therapy. Preferably, the length of the guide pipe 20 may be a range of at least 50 cm, 60 cm, 70 cm, 80 cm, 90 cm, 100 cm, 110 cm, 120 cm, 130 cm, 140 cm, or period. The guide pipe 20 has excellent bending resistance, can better adapt to complex and bendability requirements of a human body, and has a smooth inner wall and a small frictional resistance.

It should be noted that the cooling medium source is configured to input the cooling medium into the cooling medium channel 201, and the cooling medium source may be a cooling medium injector or a cooling medium supplier. In some embodiments, the cooling medium source may control the volume and pressure of the cooling medium in the input expandable structure 21. In this application, expansion of the expandable structure 21 is controlled by inputting a cooling medium, and when the cooling medium is controlled to output, the expandable structure 21 contracts. The cooling medium comprises, but is not limited to, one or more of water, a sodium chloride solution, a glucose aqueous solution, sodium lactate, a compound sodium chloride solution, sodium bicarbonate, an isotonic saline solution, and the like. In some embodiments, the cooling medium cools the surface of the expandable structure by absorbing heat.

In particular, the expandable structure 21 is configured as a bladder that can expand with the input of the cooling medium. In some embodiments, the expandable structure 21 may be expanded into one of a spherical capsule, an elliptical capsule, a conical capsule, a dumbbell-shaped capsule, or a cylindrical capsule. In some embodiments, the expandable structure 21 may be made of a material such as PU, PEBAX, or nylon. The expandable structure 21 has a length of at least 5 mm. Preferably, the expandable structure 21 may have a length of 6 mm, 7 mm, 8 mm, 10 mm, 12 mm, 15 mm, 16 mm, 18 mm, 20 mm, or a range thereof. Preferably, an outer diameter of the expandable structure 21 ranges from 4 to 10 mm.

Specifically, the handle 22 is an operating handle that controls the guide pipe 20 to deliver the expandable structure 21 and the intervention assembly 12 to the target area for treatment. One end of the guide pipe 20 passes through the handle 22, and may be adhered to the handle 22 by glue such as a quick-drying adhesive, a UV adhesive, and an epoxy adhesive. In some embodiments, the handle 22 may be formed by 3D printing or injection molding of a material such as plastic, nylon, or silica gel. In this application, the handle 22 is provided with a communicating portion 221, the communicating portion 221 communicates with the cooling medium source and the cooling medium channel 201, in some embodiments, the communicating portion 221 may be a communicating hole disposed on the handle 22, and the cooling medium source communicates with the cooling medium channel 201 through the communicating hole; in some embodiments, the communicating portion 221 may be a connecting tube disposed on the handle 22, and the cooling medium source is in communication with the cooling medium channel 201 through the connecting tube. In some embodiments, the connecting tube and the handle 22 are integrally formed.

Specifically, as shown in FIG. 17, the intervention assembly 12 comprises a conveying line 120 and an ablation electrode 121, one end of the conveying line 210 is connected to the ablation electrode 121, and the other end of the conveying line 120 is configured to be connected to an energy supply device, that is, an ablation device. The ablation electrode 121 is in a mesh shape, and the ablation electrode 121 covers the expandable structure 21.

Specifically, two ends of the ablation electrode 121 are respectively connected to two ends of the expandable structure 21 through the connector 23. In some embodiments, the connector 23 may be a connecting sleeve, and preferably, may be a cold shrink tube or a heat shrink tube. Two ends of the ablation electrode 121 of the present application may be fixedly connected to two ends of the expandable structure 21 through heat shrink tubes, respectively. The ablation electrode 121 can expand with the expansion of the expandable structure 21 and contract with the contraction of the expandable structure 21. When the expandable structure 21 contracts, the ablation electrode 121 can enter the human body along with the guide pipe 20 together with the expandable structure 21; after the expandable structure 21 is expanded, the ablation electrode 121 is tightly attached to the surface of the expandable structure 21, the surface of the ablation electrode 121 is uniformly attached to the target region, and the control conveying line 120 transmits the pulse energy to the ablation electrode 121 to implement pulse discharge.

Specifically, the conveying line 120 may be a stainless steel wire, a copper wire, or an enameled wire. The wire diameter of the conveying line 120 is 0.05 -0.5 mm. In some embodiments, the outer layer of the conveying line 120 is sleeved with a PTFE heat shrink tube, a PET heat shrink tube, or a PI sheath. In some other embodiments, the outer layer of the conveying line 120 is further provided with a PTFE coating. The conveying line 120 is configured to transmit the pulse energy to the ablation electrode 121.

Specifically, the ablation electrode 121 may be a quadrilateral mesh structure. In some embodiments, the ablation electrode 121 may be a parallelogram structure. Preferably, the ablation electrode 121 may be a diamond-shaped mesh structure. The quadrilateral mesh has instability and is easily changed in shape, so that when the ablation electrode 121 is of a quadrilateral mesh structure, the mesh is tighter, the tensile property and the structural stability are higher, the mesh can be better attached to the surface of the expandable structure 21, and the mesh ablation electrode 121 expands and contracts along with the expandable structure 21. In some embodiments, the ablation electrode 121 may be cut, woven, or electroformed. The ablation electrode 121 may be made of stainless steel, nickel-titanium alloy, cobalt-chromium alloy, or the like with good conductivity. The mesh ablation electrode 121 has a wire diameter of 0.05 to 0.30 mm. A length of the ablation electrode 121 is greater than a length of the expandable structure 21, and a length of the ablation electrode 121 is at least 5 mm. Preferably, the length of the ablation electrode 121 may be in a range of 6 mm, 8 mm, 10 mm, 15 mm, 18 mm, 20 mm, or period.

Specifically, the end of the guide pipe 20 away from the handle 22 is further provided with a second position acquisition device 11, and the second position acquisition device 11 may be connected to the inner tube 203 of the guide pipe 20. In some embodiments, the second position acquisition device 11 may be bonded to the guide pipe 20 through glue such as quick-drying glue, UV glue, epoxy glue and the like. In some other embodiments, the second position acquisition device 11 may be further connected to the guide pipe 20 through a heat shrink tube such as PU, PET, PTFE or PEEK.

Specifically, the second position acquisition device 11 comprises at least one multidegree-of-freedom magnetic positioning sensor. In an embodiment, the second position acquisition device 11 comprises at least one five-degree-of-freedom magnetic positioning sensor or a six-degree-of-freedom magnetic positioning sensor. The second position acquisition device 11 is communicatively connected to the in-vitro detection device. In some embodiments, the second position acquisition device 11 is connected to the in-vitro detection apparatus by using the wire 24, and the wire 24 is configured to transmit information such as the collected position and direction to the in-vitro detection apparatus. As shown in FIG. 18, the wire 24 passes through the wire channel 204 and penetrates out of the handle 22, so that the structure is more compact, and redundancy of the wire exposed outside is avoided. In some other embodiments, the second position acquisition device 11 may also be wirelessly connected to the in-vitro detection device.

Specifically, the second position acquisition device 11 of the present application is connected to the in-vitro detection device, that is, the navigation control device, acquires, in real time, the movement position and the direction coordinate of the end of the guide pipe 20 away from the handle 22 in the electromagnetic positioning system in the magnetic field space through the second position acquisition device 11, performs positioning, and transmits the position information of the guide pipe 20 to the navigation control device 2

In this application, the foregoing energy delivery apparatus is applied: The guide pipe 20 is controlled by the handle 22 to send the expandable structure 21 and the intervention assembly 12 in the contracted state into the in-vivo target region, and the second position acquisition device 11 is configured for navigation and accurate positioning; the cooling medium is input through the cooling medium channel 201 into the expandable structure 21 through a cooling medium source; after the expandable structure 21 is expanded, the ablation electrode 121 expands with the expandable structure 21 and is uniformly attached to the lesion tissue; pulse energy release is controlled by the ablation device, and pulse therapy is performed on the target area; in the multiple pulse therapy processes, the expandable structure can be controlled by controlling the input and output of the cooling medium, and meanwhile, heat generated by therapy is absorbed and taken away; the time and frequency of pulse therapy are controlled and operated for the lesion types and different treatment schemes of different target areas and the size of the delivered pulse energy.

In the present application, the ablation electrode 121 is in a mesh shape and covers the surface of the expandable structure, so that the structure is more compact, and the tensile performance and the structural stability are higher. The mesh ablation electrode 121 has a larger fit area with the lesion tissue, is more uniform, and has a better treatment effect. The guide pipe is a double-layer catheter, has a cooling medium channel and a wire channel, has a compact structure, and avoids redundancy of the wire exposed outside. The energy delivery device of the present application can guide the expandable structure and the intervention assembly to accurately reach the target area through the positioning and navigation of the second position acquisition device, and is simple in structure, simple and convenient to operate, low in cost, convenient to popularize and produce, and beneficial to reducing the economic burden of the patient.

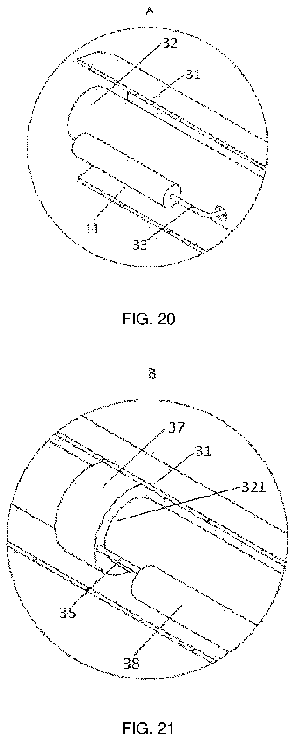

In some embodiments, as shown in FIGS. 19-21, the interventional device of the present application may also be an adjustable bending guide tube, and the guide tube may be used in cooperation with an endoscope, or may be used separately to reach a further lesion position. The distal end of the guide tube is provided with a second position acquisition device 11, the second position acquisition device 11 is externally connected to the navigation control device 2, and the navigation control device 2 tracks the position of the distal end of the guide tube by means of the second position acquisition device 11, thereby achieving the purpose of navigation. The guide tube may cooperate with interventional instruments such as biopsy forceps and ablation catheters to perform precise inspection or treatment such as sampling, microwave, ablation, and radiotherapy.

FIG. 19 is a schematic structural diagram of an adjustable bend guide tube. The adjustable bending guide tube of the present application comprises a manipulation portion 30 and a first layer tube 31 and a second layer tube 32, wherein the first layer tube 31 and the second layer tube 32 are connected to the manipulation portion 30, the second layer tube 32 is disposed in the first layer tube 31, and the second layer tube 32 is in communication with the inside of the manipulation portion 30.

Specifically, the manipulation portion 30 may be an internal hollow adjustment handle or an adjustment rod. The manipulation portion 30 may be formed by 3D printing or injection molding of a material such as plastic, nylon or silica gel. The proximal ends of the first layer tube 31 and the second layer tube 32 may be bonded to the manipulation portion 30 by glue such as quick-drying glue, UV glue, epoxy glue and the like. The first layer tube 31 is sleeved outside the second layer tube 32, and the second layer tube 32 may be configured as a single-cavity sheath tube or a multicavity sheath tube according to actual requirements. In some embodiments, the guide tube may be extruded tubes such as PI PET PEBAX PTFE, PI/PTFE composite tubes, PEBAX and stainless steel wire braided tubes or multilayer tubes. The second layer tube 32 may have a diameter of 0.5 to 5 mm and a tube wall thickness of 0.025 to 0.5 mm. In some embodiments, a length of the second layer tube 32 is greater than or equal to a length of the first layer tube 31, and a distal end of the second layer tube 32 is flush with or protrudes from a distal end of the first layer tube 31. In some embodiments, the length of the second layer tube 32 is at least 60 cm. Preferably, the length of the second layer tube 32 may be at least a range of 60 cm, 70 cm, 80 cm, 90 cm, 100 cm, 110 cm, 120 cm, or period. In some embodiments, the diameters of the first layer tube 31 and the second layer tube 32 remain unchanged from the proximal end to the distal end. In some other embodiments, the diameters of the first layer tube 31 and the second layer tube 32 are gradually reduced from the proximal end to the distal end. Preferably, the diameter of the proximal end of the second layer tube 32 is 2 to 5 mm, and the diameter of the distal end is 0.5 to 3 mm. The diameter of the first layer tube 31 and the diameter of the second layer tube 32 gradually decrease, which is beneficial to increase the flexibility of the distal end of the guide tube, which facilitates the guide tube to bend along with the trend, thereby avoiding damage to the human body to the greatest extent.

Specifically, the hollow pipeline of the second layer of tube 32 may be used as an external treatment such as biopsy forceps or ablation catheter to enter the working channel of the human body. Interventional instruments such as biopsy forceps and ablation catheters can avoid friction damage to a human body by conveying hollow pipelines inside the guide tube.

Specifically, a second position acquisition device 11 is provided at a distal end of the second layer tube 32 away from the manipulation portion 30, and the second position acquisition device 11 is connected to an outer wall of the second layer tube 32.

In some embodiments, the distance between the second position acquisition device 11 and the distal end of the second layer tube 32 is 0-2 cm. Preferably, the distance from the second position acquisition device 11 to the distal end of the second layer tube 32 may be 0, 0.5 cm, 1 cm, 2 cm, or the distance during the period. The second position acquisition device 11 is connected to the outer wall of the second layer tube 32, does not occupy the space of the hollow pipe of the second layer tube 32, and is beneficial to operation with an interventional instrument such as sampling or ablation. The distance from the second position acquisition device 11 to the distal end of the second layer tube 32 is smaller, and the position and direction information of the distal end of the second layer tube 32 acquired by the second position acquisition device 11 are more accurate. In some embodiments, the second position acquisition device 11 may be bonded to an outer wall of the second layer tube 32. In some other embodiments, the second position acquisition device 11 may be thermally connected to the second layer tube 32 through a heat shrink tube such as PU, PET, PTFE, or PEEK.

In some embodiments, the second position acquisition device 11 is connected to the conductive line 33, the conductive line 33 is configured to be externally connected to the navigation control device 2, one end of the conductive line 33 is connected to the second position acquisition device 11, and the other end is communicatively connected to the navigation control device 2. Specifically, the conductive line 33 may be connected to the second position acquisition device 11 by means of laser welding or tin soldering. The conductive line 33 may pass through the hollow pipe of the second layer tube 32, or may pass through the gap between the second layer tube 32 and the first layer tube 31. The conductive wire 33 is a single-strand or multi-strand enameled wire, and the wire diameter of the conductive wire 33 is between 0.05 mm and 0.3 mm. The conductive line 33 is configured to transmit information such as a position and a direction of the guide tube collected by the second position acquisition device 11 to the in-vitro navigation control device 2

Specifically, the second position acquisition device 11 comprises at least one multi-degree-of-freedom magnetic positioning sensor, and the multi-degree-of-freedom magnetic positioning sensor is connected to an outer wall of the second layer tube 32. In some embodiments, the second position acquisition device 11 comprises at least one five-degree-of-freedom magnetic positioning sensor or a six-degree-of-freedom magnetic positioning sensor. In the present application, the second position acquisition device 11 may acquire the position and direction coordinates of the distal movement of the guide tube, and transmit the position information of the guide tube to the navigation control device 2, thereby navigating and positioning the guide tube.

Specifically, the manipulation portion 30 is provided with a bending control member 34 and an adjusting wire 35, one end of the adjusting wire 35 is connected to the bending control member 34, and the other end of the adjusting wire 35 is configured to be fixedly connected to the bending section 321 of the second layer tube 32. The bending section 321 of the present application is disposed on the second layer tube 32 and is close to the distal end of the second layer tube 32. In some embodiments, the distance from the bending section 321 to the distal end of the second layer tube 32 is 2 -5 cm. Preferably, the distance from the bending section 321 to the distal end of the second layer tube 32 is 2 cm, 3 cm, 4 cm, 5 cm, or the distance during the period. When the bending control member 34 adjusts the bending wire 12 to pull the second layer tube 32 to bend, the distance from the bending section 321 to the distal end of the second layer tube 32 is too large or too small to affect the flexibility and accuracy of the adjusting wire 35 driving the bending of the second layer tube 32.

Specifically, the bending control member 34 is slidably or rotatably connected to the manipulation portion 30. In some embodiments, the bending control member 34 may be configured as a slider structure slidably connected to the manipulation portion 30. The slider structure may be formed by 3D printing or injection molding of a material such as plastic, nylon or silica gel. The manipulation portion 30 is provided with a rail 36, and the bending control member 34 slides along the rail 36 to pull the bending section 321 of the second layer tube 32 through the adjusting wire 35 to bend the second layer tube 32 to control the distal direction of the second layer tube 32, so as to more accurately reach the lesion region. Specifically, the rail 36 may be a guide rail chute. In some other embodiments, the bending control member 34 is configured as a rotating wheel structure connected to the manipulation portion 30, and the bending control member 34 rotates to pull the adjusting wire 35 to move, thereby driving the second layer tube 32 to bend. The bending structure of the present application is simple and easy to implement.

Specifically, the bending control member 34 pulls the second layer of tube 32 to bend by 0-180° by adjusting the wire 35. Preferably, the maximum bending radian of the second layer tube 32 may be in a range of 100°, 110°, 120°, 150°, 160°, 180°, or duration. In the present application, the bending control member 34 pulls the bending radian of the second layer tube 32 by the adjusting wire 35 to be relatively large, and the flexibility of the distal end of the second layer tube 32 is increased, so that the bending control member 34 can enter a focus position with a closer angle. The first layer tube 31 and the second layer tube 32 both have certain flexibility, the first layer tube 31 is sleeved on the outer side of the second layer tube 32, and the first layer tube 31 is bent along with the second layer tube 32 while the second layer tube 32 is bent.

Specifically, the outer side of the bending section 321 is provided with a connecting structure 37, and the bending section 321 is connected to the adjusting wire 35 through the connecting structure 37. The connecting structure 37 is configured to connect the adjusting wire 35 and the bending section 321 in some embodiments, the connecting structure 37 may be configured to be a connecting pipe or a connecting clip fixed on the bending section 321, and the adjusting wire 35 is welded to the connecting structure 37. In some other embodiments, the connecting structure 37 may be a heatshrinkable connecting pipe or a cold-shrink connecting pipe, the connecting structure 37 is sleeved on the outer side of the bending section 321 and the adjusting wire 35, and after the connecting structure 37 contracts, the adjusting wire 35 is tightly connected to the bending section 321.

Specifically, the adjusting wire 35 is a round wire rope or a flat wire rope composed of a single strand or a plurality of strands of metal wires. The outer diameter of the adjusting wire 35 may be 0.05 to 0.3 mm. In practical applications, the guide tube can be controlled to bend by pulling the control wire 431 connected to the bending section 321.

Specifically, an outer side of the adjusting wire 35 is sleeved with a wire bending channel 38, and the wire bending channel 38 is axially disposed along the second layer tube 32 and is connected to the manipulation portion 30. Specifically, the wire bending channel 38 is sleeved on the adjusting wire 35, and the wire bending channel 38 is connected to the second layer tube 32. One end of the wire bending channel 38 is connected to the manipulation portion 30, and the other end of the wire bending channel 38 may be welded to the outer wall of the second layer tube 32. A distance between one end of the wire bending channel 38 close to the bending section 321 and a distal end of the second layer tube 32 is 2.5 to 5.5 cm. The wire bending channel 38 may be a PTFE or nylon tube with a diameter of 0.15 to 0.35 mm. The wire bending channel 38 is a moving channel for the adjusting wire 35 to move, and it can be avoided that the adjusting wire 35 is out of control or excessive bending under the condition that the force is pulled, which affects the accuracy of adjusting the distal end of the guide tube.

In the method for using the adjustable bending guide tube of the present application, the guide tube is placed in the working channel of the endoscope, reaches the lung along with the endoscope, and passes out of the channel outlet; The navigation guidance of the second position acquisition device is accurately positioned and reaches the lesion tissue, and the distal bending aiming position of the guide tube can be controlled by the bending control member and the adjusting wire in the process; and interventional instruments such as biopsy forceps and ablation intervention assemblies are accessed through the guide tube for accurate inspection or treatment such as sampling, microwave, ablation or radiotherapy.

A second position acquisition device is arranged at the end of the adjustable bending guide tube, and the guide tube can be positioned and navigated through the second position acquisition device. The guide tube further comprises a bending control member and an adjusting wire, and the bending of the guide tube can be controlled by the bending control member and the adjusting wire. The second position acquisition device is matched with the bending manipulation portion and the adjusting wire for controlling the bending of the guide tube to more accurately position the lesion tissue, so that the far end of the guide tube can accurately reach a farther and narrower lesion position, the diagnosis accuracy and the treatment accuracy are greatly improved, and diagnosis and treatment wounds are effectively reduced. The adjustable bending guide tube is simple in structure, easy and convenient to operate, low in cost, high in practicability and convenient to popularize and produce.

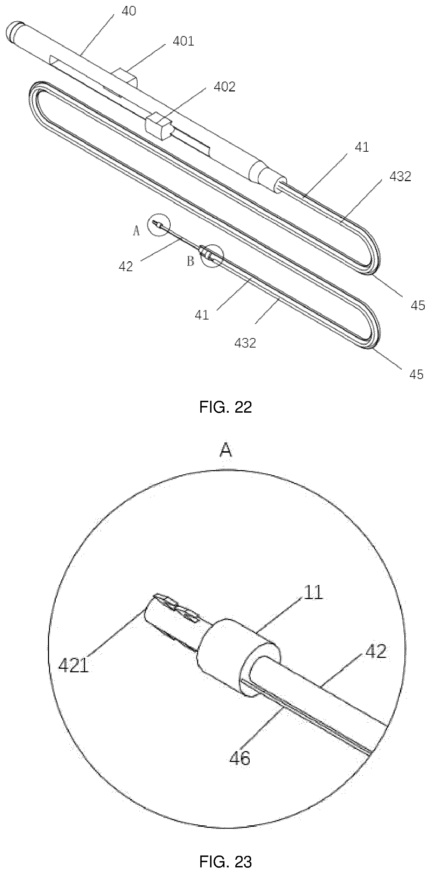

In some embodiments, as shown in FIGS. 22-25, the interventional device of the present application may also be a medical biopsy sampling device, which is mainly configured for sampling lung lesion tissue during clinical use. The sampling device can cooperate with the bronchoscope to enter the trachea through the nose or mouth of the patient, and reach the lungs through the bronchus, thereby sampling the lesion or dispersion pulmonary disease at the peripheral part of the lung. The sampling device of the present application may be used in conjunction with a bronchoscope, and may also be used in cooperation with an external navigation control device by means of a self-contained second position acquisition device, and the guide tube and the sampling member are adjusted by means of the bending adjustment member, so as to accurately reach a lesion position to obtain a lesion tissue.

As shown in FIG. 22, which is a schematic structural diagram of a biopsy sampling device of the present application, the biopsy sampling device comprises an adjusting portion 40, a guide tube 41, a sampling member 42 and a bending adjustment member 43, the guide tube 41 is sleeved on the sampling member 42, and the guide tube 41 is connected to the adjusting portion 40. The guide tube 41 comprises a proximal end and a distal end, and a proximal end of the guide tube 41 is connected to the adjusting portion 40. In an actual application, the proximal end of the guide tube 41 may be bonded to the adjusting portion 40 through glue such as a quick-drying adhesive, a UV adhesive, and an epoxy adhesive. The inner portion of the guide tube 41 is of a hollow structure, and is configured for the working channel for pushing and contracting the sampling member 42. The guide tube 41 may be a sheath, and may be a single-cavity sheath or a multi-cavity sheath according to actual requirements. In some embodiments, specifically, it may be an extrusion tube such as PI/PET PEBAX PTFE, or a PI/PTFE composite tube or PEBAX and a stainless steel wire braided tube. The outer diameter of the guide tube 41 may be 0.5 -5 mm, and the tube wall thickness may be 0.025 -0.5 mm. The length of the guide tube 41 is at least 40 cm. Preferably, the length of the guide tube 41 may be at least 40 cm, 50 cm, 60 cm, 70 cm, 80 cm, 90 cm, 100 cm, 110 cm, or the range of the period. In some embodiments, the outer diameter of the guide tube 41 gradually decreases from the proximal end to the distal end. Preferably, the outer diameter of the proximal end of the guide tube 41 is 2 -5 mm; and the outer diameter of the distal end is 0.5 -2.5 mm. The gradual decrease of the outer diameter of the guide tube 41 is beneficial to increasing the flexibility of the guide pipe, facilitating bending along with the trend of the bronchus, and avoiding damage to the bronchus to the greatest extent.

Specifically, the adjusting portion 40 is provided with a first adjusting member 401 and a second adjusting member 402, one end of the sampling member 42 is connected to the first adjusting member 401, and the other end of the sampling member 42 is provided with a sampling head 421 for driving the sampling member 42 and the sampling head 421 to be pushed out or retracted into the guide tube 41 from the distal end of the guide tube 41.

In some embodiments, the sampling member 42 may be a metal wire or a metal tube passing through the guide tube 41. The material of the sampling member 42 may be stainless steel, a nickel-titanium alloy, a cobalt-chromium alloy, or the like, and has good bending performance and corrosion resistance. Preferably, the stainless steel may be 304 or 316 stainless steel. The diameter of the sampling member 42 may be 0.2 -2 mm. The outer layer of the sampling member 42 may be provided with a PTFE coating having a thickness of 0.005 to 0.1 mm.

In some embodiments, the sampling head 421 may be a diamond cone head, and the focus is punctured and sampled by the diamond cone head. In some other embodiments, at least one barb structure is distributed or circumferentially distributed on the sampling head 421, and the lesion tissue is punctured and sampled through the barb structure. Preferably, the sampling head 421 is provided with two barb structures. In some other embodiments, the tip of the sampling head 421 is provided with a sampling groove for sampling through the sampling groove. The sampling member 42 may be integrally formed with the sampling head 421, and the sampling head 421 may be formed by machining abrasive dust or wire cutting. Compared with a conventional forceps-type sampling head, the sampling head of the present application is smaller in size, convenient to operate and reach a precise position, and the sampling wound is smaller.

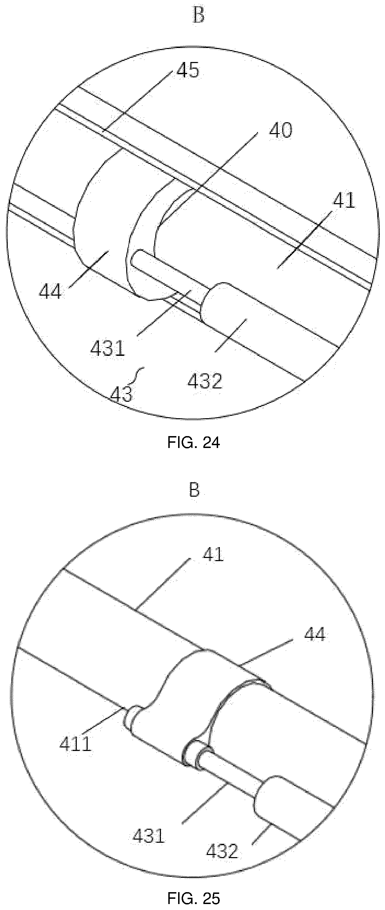

As shown in FIG. 24, the biopsy sampling device of the present application comprises a bending adjustment member 43, one end of the bending adjustment member 43 is connected to the second adjusting member 402, the other end of the bending adjustment member 43 is connected to the bending portion 411 of the guide tube 41, and the bending adjustment member 43 can pull the guide tube 41 through the bending portion 411 to bend. It should be noted that the bending portion 411 is disposed on the guide tube 41 and is close to the distal end of the guide tube 41. In some embodiments, the distance between the bending portion 411 and the distal end of the guide tube 41 is 0.5 -6 cm. Preferably, the distance between the bending portion 411 and the distal end of the guide tube 41 is 0.5 cm, 1 cm, 2 cm, 3 cm, 4 cm, 5 cm, 6 cm, or the distance during the period. Specifically, the distance from the bending portion 411 to the distal end of the guide tube 41 may affect the flexibility of the bending adjustment member 43 driving the guide pipe to bend, and the distance from the bending portion 411 to the distal end of the guide tube 41 may affect the accuracy of the bending adjustment member 43 controlling the distal end of the guide pipe.

In some embodiments, the bending adjustment member 43 comprises a regulating wire 431, one end of the regulating wire 431 is connected to the second adjusting member 402, and the other end of the regulating wire 431 is connected to the bending portion 411. The second adjusting member 402 can drive the adjusting wire 431 to move, so that the adjusting wire 431 pulls the guide tube 41 to bend through the bending portion 411. The adjusting wire 431 may be a single-strand or multi-strand stainless steel wire, and the outer diameter of the adjusting wire may be 0.1 to 0.3 mm. In practical applications, the length of the guide tube 41 is unchanged, and the guide tube 41 can be pulled to bend towards one side by tensioning the control wire 431 fixedly connected to the bending portion 411.

In some embodiments, the bending adjustment member 43 comprises an adjustment tube 432, the adjustment tube 432 is sleeved on the adjusting wire 431, and the adjustment tube 432 is connected to the guide tube 41. Specifically, one end of the adjustment tube 432 is connected to the adjusting portion 40, and the other end of the adjustment tube 432 may be fixedly connected to the guide tube 41 by means of thermal welding or the like. The adjustment tube 432 is a sheath tube through which the control wire 431 passes, and may be a PTFE or nylon tube with an outer diameter of 0.15 -0.35 mm. The adjustment pipe 432 is a guide channel for the regulation and control wire 431 to move, so that the regulation and control wire 431 can be prevented from being out of control or excessive bending under the condition that the stress is pulled, and the adjustment accuracy of the far end of the guide tube is affected.

In some embodiments, as shown in FIG. 24, a connecting member 44 is provided at the bending portion 411 of the guide tube 41, and the adjusting wire 431 is connected to the bending portion 411 through the connecting member 44. The connecting member 44 may be a connecting tube or a connecting clip fixed on the bending portion 411, and the adjusting wire 431 is connected to the connecting member 44 in a welded manner. In some other embodiments, as shown in FIG. 25, the connecting member 44 may be a heat shrinkable connecting tube or a cold shrink connecting tube, the connecting member 44 is sleeved on the outer side of the bending portion 411 and the adjusting wire 431, and after the connecting member 44 contracts, the adjusting wire 431 is tightly connected to the bending portion 411.

In the present application, the curvature of the bending adjustment member 43 pulling the guide tube 41 is 0° to 120°. Preferably, the maximum bending radian may be in the range of 90°, 100°, 110°, 120°, or duration. The bending adjustment member 43 of the present application drives the guide tube 41 to bend in a relatively large radian, so that the flexibility of the distal end of the guide tube 41 driving the sampling head 421 to move is increased, fine adjustment can be performed, the position can be accurately reached, the lesion tissue can be effectively obtained, and the bending adjustment member 43 has a simple structure and is easy to implement.

In some embodiments, the guide sleeve 45 is sleeved on the outer side of the guide tube 41 and the bending adjustment member 43, one end of the guide sleeve 45 is connected to the distal end of the guide tube 41, and the other end of the guide sleeve 45 is connected to the adjusting portion 40. The bending action of the bending adjustment member 43 driving the guide tube 41 to bend is performed in the guide sleeve 45.

Specifically, the first adjusting member 401 and the second adjusting member 402 are respectively slidably or rotatably connected to the adjusting portion 40. The adjusting portion 40 may be an adjustment handle or an adjustment rod. The adjusting portion 40 may be formed by 3D printing or injection molding of a material such as plastic, nylon, or silica gel. In this application, the first adjusting member 401 for controlling the sampling member 42 and the second adjusting member 402 for controlling the bending adjustment member 43 are integrated in the adjusting portion 40, and are respectively adjusted through different adjusting channels, so that the operation is more accurate and convenient. In some embodiments, the first adjusting member 401 and the second adjusting member 402 may be configured as a slider structure. The slider structure may be formed by 3D printing or injection molding of a material such as plastic, nylon, or silica gel. The adjusting portion 40 is provided with a first sliding groove and a second sliding groove, the second adjusting member 401 is slidably connected to the first sliding groove, and the second adjusting member 402 is slidably connected to the second sliding groove. In this embodiment, the first sliding groove and the second sliding groove are integrated into a strip-shaped groove penetrating through the adjusting part 40, and the second adjusting member 401 and the second adjusting member 402 are respectively connected to two sides of the strip-shaped groove. The second adjusting member 402 is controlled to slide along the second sliding groove, the adjusting wire 431 can be pulled to move, and the adjusting wire 431 pulls the guide tube 41 through the bending portion 411 to bend, thereby controlling the direction of the distal end of the guide tube to more accurately reach the lesion. The first adjusting member 401 is controlled to slide along the first sliding groove to pull the sampling member 42 to move, so that the sampling head 421 can be controlled to be pushed out or contracted from the distal end of the guide tube 41 to more accurately obtain the lesion tissue. In some other embodiments, the first adjusting member 401 and the second adjusting member 402 may be configured as a rotating wheel structure, and the first adjusting member 401 may be rotated to drive the sampling member 42 to move, so as to control the sampling head 421 to be pushed out or contracted from the distal end of the guide tube 41. The rotation of the second adjusting member 402 can drive the adjusting wire 431 to move, so that the adjusting wire 431 pulls the guide tube 41 to bend through the bending portion 411.

In some embodiments, the maximum push-out distance that the first adjusting member 401 controls the sampling head 421 to be pushed out from the distal end of the guide tube 41 is 10 cm. Preferably, the maximum push-out distance of the sampling head 421 may be a distance of 5 cm, 6 cm, 7 cm, 8 cm, 9 cm, 10 cm, or period.

Specifically, as shown in FIG. 23, the sampling member 42 is provided with a second position acquisition device 11, and the second position acquisition device 11 is disposed close to the sampling head 421. In some embodiments, the distance between the second position acquisition device 11 and the sampling head 421 may be 0-3 cm. Preferably, the distance from the second position acquisition device to the sampling head 421 may be 0.5 cm, 1 cm, 2 cm, 3 cm, or a distance during the period. The distance from the second position acquisition device to the sampling head 421 of the present application is smaller, and the position and direction information of the acquired sampling head 421 are more accurate. In some embodiments, the second position acquisition device 11 may be bonded to the sampling member 42 by glue such as a quick-drying adhesive, a UV adhesive, and an epoxy adhesive. In some embodiments, the second position acquisition device 11 may be further connected to the sampling member 42 through a heat shrink tube such as PU, PET, PTFE, or PEEK. In some embodiments, the second position acquisition device and the sampling head 421 shrink together into the guide pipe.

In some embodiments, the second position acquisition device 11 is connected to the in-vitro navigation control device 2 through the signal line 46. The signal line 46 passes through the guide tube 41 and is connected to the in-vitro navigation control device through a through hole formed in the adjusting portion 40. The signal line 46 may be a single strand or a plurality of enameled wires, and the wire diameter of the signal line 46 is between 0.05 mm and 0.3 mm. The signal line 46 and the second position acquisition device 11 may be welded and connected by laser welding or tin soldering. The signal line 46 is configured to transmit information such as a position and a direction acquired by the second position acquisition device 11 to the navigation control device. Specifically, the second position acquisition device 11 comprises at least one multi-degree-of-freedom magnetic positioning sensor. In some embodiments, the second position acquisition device 11 comprises at least one five-degree-of-freedom magnetic positioning sensor or a six-degree-of-freedom magnetic positioning sensor. The second position acquisition device 11 may acquire the moving position and direction coordinates of the movement of the sampling head 421 in real time, and transmit the position information of the sampling head 421 to the navigation control device 2, and the navigation control device 2 navigates and positions the sampling head 421 according to the acquired position information.

In the present application, the sampling step is implemented by applying the biopsy sampling device: connecting the signal line 46 of the second position acquisition device 11 to the in-vitro navigation control device 2, and shrinking the sampling head into the guide tube; The guide tube is placed in the bronchoscope working channel, reaches the lungs with the bronchoscope, and passes out of the channel exit; In the process, the bending adjustment member 43 can be pulled by the second adjusting member 402 to control the bending and aiming position of the guide tube 41; the first adjusting member 401 controls the sampling head 421 to push out of the distal end of the guide tube for puncturing and sampling; the first adjusting member 401 controls the sampled sampling head 421 to retract back to the guide tube, and then the sampling device is taken out from the bronchoscope working channel to remove the tissue sample.

In the present application, the first adjusting member for controlling the sampling member and the second adjusting member for controlling the bending portion are integrated in the adjusting portion, and are respectively adjusted by different adjustment channels, which is more convenient to operate. By means of the bending member, the distal end of the guide tube can be controlled to bend, and the bending radian is increased, so that the flexibility of bending of the sampling member driven by the guide tube is increased, fine adjustment can be performed, the lesion area can be more accurately reached and sampled, and the sampling can also be flexibly exited after sampling. The sampling device of the present application is provided with a second position acquisition device, and by navigation and positioning of the second position acquisition device and the in-vitro navigation control device, the sampling part can be guided to directly reach the lesion area, so that the sampling accuracy is improved, and the problems of sampling failure and repeated sampling can be effectively avoided. The biopsy sampling device is simple in structure, easy and convenient to operate, low in cost, convenient to popularize and produce, and beneficial to reducing the economic burden of a patient.

The navigation control device 2 of the present application is configured to construct a virtual model based on the regional image information of the target intervention region, the morphological attribute information, the movement position information, and the intervention position information of the intervention assembly 12, to obtain a three-dimensional navigation model and an operation object three-dimensional model, where the navigation three-dimensional model is configured to represent three-dimensional space features of the target intervention region, three-dimensional space features of the intervention assembly 12, and spatial position information between the to be ablated object, the intervention assembly 12, and the target intervention region, and the operation object three-dimensional model is configured to represent a three-dimensional space feature of the to be ablated object.

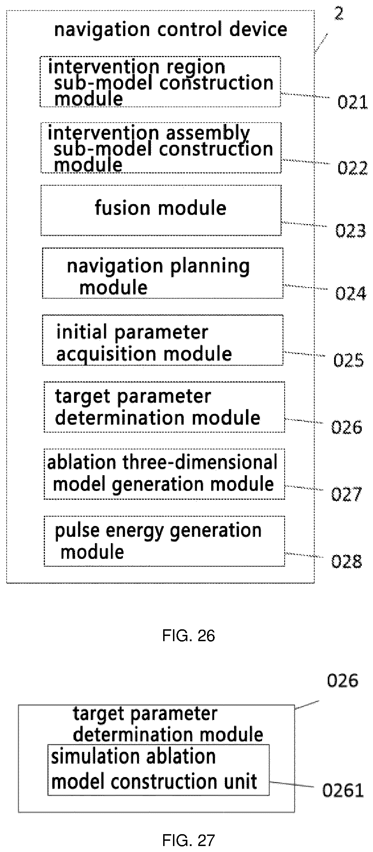

Specifically, as shown in FIG. 26, the navigation control device 2 of this application comprises:
The intervention region sub-model construction module 021 is configured to perform image recognition processing on the region image information of the target intervention region to obtain an image recognition result; and perform image reconstruction based on the image recognition result to obtain an intervention region sub-model and an operation object three-dimensional model, where the intervention region sub-model is configured to represent a three-dimensional space feature of the target intervention region.

It should be noted that the region image information of the target intervention region of the present disclosure may be a CT image or a nuclear magnetic image of the target intervention region. The three-dimensional data of the target intervention region and the three-dimensional data of the operation object may be extracted by performing image recognition processing on the CT image or the nuclear magnetic image of the target intervention region to provide data support for constructing the three-dimensional model. When the CT image of the target intervention region is the lung CT image of the target object, the three-dimensional data of the lung bronchus and the three-dimensional data of the lesion tissue area in the lung bronchus can be extracted by performing image recognition processing on the lung CT image, and image reconstruction is performed according to the three-dimensional data of the lung bronchus and the three-dimensional data of the lesion tissue area in the lung bronchus to obtain the lung bronchial three-dimensional model and the lesion tissue three-dimensional model.

The intervention assembly sub-model construction module 022 is configured to perform virtual model construction according to the morphological attribute information of the intervention assembly 12 to obtain an intervention assembly sub-model, where the intervention assembly sub-model is configured to represent three-dimensional space features of the intervention assembly 12. Specifically, the morphological attribute information of the ingress component comprises information such as the model and specification of the intervention assembly 12, and model construction is performed according to the model and specification information of the intervention assembly 12 to obtain the intervention assembly sub-model. The intervention assembly sub-model of the present application is a three-dimensional model of the intervention assembly 12.

The fusion module 023 is configured to perform spatial fusion processing on the intervention region sub-model, the operation object three-dimensional model and the intervention assembly sub-model according to the intervention position information and the movement position information to obtain a navigation three-dimensional model.

Specifically, the fusion module 023 first dynamically matches the intervention position information and the movement position information of the intervention assembly 12 obtained in real time with the coordinates of the intervention assembly sub-model to obtain a dynamic intervention assembly sub-model, and then displays the dynamic intervention assembly sub-model in the static intervention region sub-model in real time, that is, spatial fusion processing is performed on the intervention region sub-model, the operation object three-dimensional model and the intervention assembly sub-model to obtain a navigation three-dimensional model. The navigation three-dimensional model of the present application can visually display the dynamic trajectory and the real-time position of the intervention assembly 12 in the target intervention region, and provide a crucial help for determining the position of the intervention assembly in the operation process.

The navigation control device 2 further comprises a navigation planning module 024, and the navigation planning module 024 is communicatively connected to the navigation three-dimensional model.

The navigation planning module 024 is configured to perform navigation planning according to the intervention region sub-model and the operation object three-dimensional model to obtain a navigation path of the intervention assembly 12 in the intervention region sub-model, where the navigation path is configured to instruct the intervention assembly 12 to arrive at a travel path of a required path in the process of reaching the to be ablated object in the target intervention region.

It should be noted that the navigation planning module 024 of the present application may perform navigation planning on the movement path of the intervention assembly 12 reaching the to be ablated object in the target intervention region according to the intervention region sub-model and the operation object three-dimensional model to obtain a navigation path of the intervention assembly 12 in the intervention region sub-model, generate corresponding two-dimensional, three-dimensional and navigation data, and display the planned navigation path from the entrance of the intervention assembly to the to be ablated object in the navigation three-dimensional model. In some possible embodiments, a path key point of the planned navigation path may be highlighted in the navigation three-dimensional model, the operator may control, according to the displayed navigation path, the intervention assembly 12 to move in the navigation three-dimensional model, and control the intervention assembly 12 to arrive at the to be ablated object.

In some embodiments, the navigation planning module 024 is further configured to determine, in the movement process of the intervention assembly 12 in the target intervention region, the intervention trajectory information of the intervention assembly 12 based on the intervention position information and the movement position information of the intervention assembly 12; and if the path trajectory information corresponding to the intervention trajectory information and the navigation path meets a preset deviation condition, correct the intervention position of the intervention assembly 12 in the target intervention region, until the updated intervention trajectory information of the intervention assembly 12 matches the path trajectory information.

It should be noted that, specifically, during the movement of the intervention assembly 12 in the target intervention region, the real-time intervention trajectory information of the intervention assembly 12 may be determined according to the intervention position information and the movement position information of the intervention assembly 12, the trajectory key points of the intervention trajectory are generated according to the real-time intervention trajectory information of the intervention assembly 12, and the trajectory key points are dynamically matched with the planned path key points of the navigation path; when the matching degree between the trajectory key point and the planned path key point of the navigation path meets a preset deviation condition, the real-time intervention position of the intervention assembly 12 in the target intervention region is corrected to match the real-time intervention trajectory information of the intervention assembly 12 with the path trajectory information. The preset deviation condition is whether the deviation between the trajectory key point of the intervention trajectory and the path key point of the planned navigation path exceeds a preset deviation threshold, and when the deviation between the trajectory key point of the intervention trajectory and the path key point is greater than the preset deviation threshold, the real-time intervention position of the intervention assembly 12 in the target intervention region is corrected; When the deviation between the trajectory key point of the intervention trajectory and the path key point of the planned navigation path is less than or equal to the preset deviation threshold, the intervention assembly 12 is controlled to continue to move along the navigation path. In the present application, by dynamically matching the real-time intervention trajectory of the intervention assembly 12 and the navigation path, the movement direction of the intervention assembly 12 can be adjusted at any time in the movement process of the intervention assembly 12 in the target intervention region, thereby preventing the intervention assembly 12 from offsetting the navigation path or causing damage to surrounding healthy tissues, and improving the navigation accuracy.

In some possible implementations, when the navigation path is blurred or occluded in the navigation three-dimensional model, the operator may still determine, based on the displayed navigation three-dimensional model, whether the front of the path is a path, and determine whether to continue to move along the planned navigation path. For example, when the front end of the intervention assembly 12 encounters mucus according to the displayed navigation three-dimensional model, it can continue to explore forward, and when the front end of the intervention assembly 12 encounters mucus according to the displayed navigation three-dimensional model, the mucus can be flushed out according to the guidance of the navigation path, so that the mucus does not stick to the front end of the intervention assembly 12 along the wall of the trachea, thereby solving the problem that the visual field is blurred and interfered, and saving the operation time.

As shown in FIGS. 26 and 27, the navigation control device 2 further comprises an initial parameter acquisition module 025 and a target parameter determination module 026.

The initial parameter acquisition module 025 is configured to obtain an initial ablation parameter corresponding to the object attribute information of the to be ablated object, impedance data of the to be ablated object, an abutment parameter of the intervention assembly 12, and a dielectric constant of the to be ablated object, where the initial ablation parameter at least comprises at least one of a pulse voltage, a pulse width, a pulse time, and a pulse frequency.

It should be noted that the object attribute information of the object to be ablated in the present application comprises and is not limited to the lesion type and lesion degree information of the lesion tissue; the impedance data of the object to be ablated may indicate the current load condition of the intervention assembly 12; and the abutment parameters of the intervention assembly 12 comprise and are not limited to information such as the contact area and the contact length of the intervention assembly 12 and the bronchus.

In the present application, the organization characteristics of the current object to be ablated may be determined according to the dielectric constant of the object to be ablated. Specifically, different cells have different electroporation thresholds, so the dielectric constants of different tissues are different, and different tissue proportions can be guided through the dielectric constant ratio of different tissues to determine the content of different cells in the tissue. In some possible real-time manners, the detection of the dielectric constant may be performed by generating a sinusoidal excitation signal with a plurality of frequencies in the 5 KHz to 300 MHz, exciting the change of the complex impedance electrical signal corresponding to the reaction cell tissue under different frequency signals, and then converting the signals into a frequency domain signal by means of a method such as Fourier transform, so as to obtain the dielectric constant of the tissue. According to the comparison of the dielectric constants of the to be ablated objects before and after ablation, the change of the content of different types of cells in the bronchial tissue corresponding to the to be ablated object can be known, so that the effective degree of the ablation parameters can be evaluated.

The target parameter determination module 026 is configured to perform an ablation effect evaluation according to the navigation three-dimensional model, the operation object three-dimensional model, the object attribute information of the object to be ablated, the initial ablation parameter corresponding to the object attribute information of the object to be ablated, the impedance data of the object to be ablated, the abutment parameter of the intervention assembly 12, and the dielectric constant of the object to be ablated, to obtain ablation effect data corresponding to the initial ablation parameter; and if the ablation effect data satisfies the target ablation condition, determine the initial ablation parameter as the target ablation parameter.

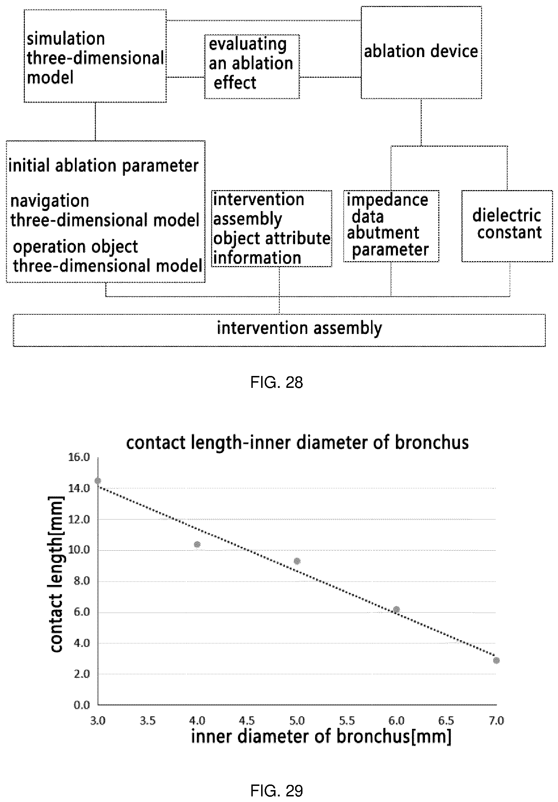

FIG. 28 is a schematic diagram of simulation, evaluation and display of the lung ablation effect of the pulse energy of the present application.

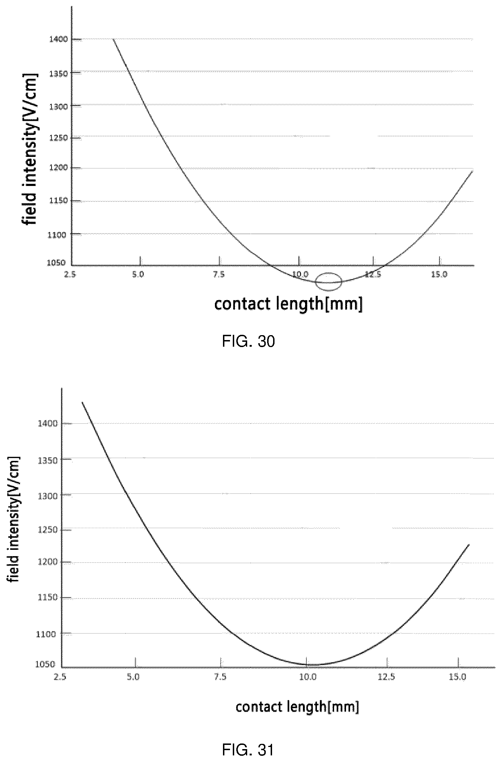

It should be noted that the impedance data of the present application, together with parameters such as a pulse voltage, a pulse width, a pulse time, and a pulse frequency, may calculate the pulse energy currently loaded to the object to be ablated. The abutment parameter of the intervention assembly 12 can be obtained through the change rate of the impedance, so that the attaching quality of the intervention assembly 12 and the to be ablated object is judged. In some possible implementations, the diameter of the inner wall of the bronchus may be calculated by the abutment parameter of the intervention assembly 12, so as to determine the thickness of the bronchial mucosa layer. As shown in FIG. 29, as the relationship between the inner diameter of the bronchus and the contact length, when the model and specification of the intervention assembly 12 are unchanged, the contact length between the intervention assembly 12 and the bronchus decreases as the inner diameter of the bronchus increases. Further, according to the impedance data, a relationship between a bronchial inner diameter, an ablation range, a contact parameter, a pulse parameter, and a bronchial thickness and an ablation effect may be further obtained through simulation. For example, in some embodiments, as shown in FIG. 30, when the contact length between the intervention assembly 12 and the bronchus is 5.0 mm, 7.5 mm, 10.0 mm, 12.5 mm, and 15.0 mm, the corresponding field strength is approximately 1220 V/cm, 1120 V/cm, 900 V/cm, 1000 V/cm and 1125 V/cm, respectively, and when the contact length is approximately 11.0 mm, the corresponding field strength is approximately 1220 V/cm, 1120 V/cm, 900 V/cm, 1000 V/cm and 1125 V/cm, and when the contact length is approximately 11.0 mm, the inner diameter decreases, the contact length between the intervention assembly 12 and the bronchus increases, the field intensity at the mark point has a decreasing trend, but the minimum field strength is not less than 900 V cm. As shown in FIG. 31, when the contact length between the intervention assembly 12 and the bronchus is 5.0 mm, 7.5 mm, 10.0 mm, 12.5 mm, and 15.0 mm, the corresponding field strength is about 1600 V/cm, 1310 V/cm, 1120 V/cm, 1060 V/cm, 1080 V/cm, and 1200 V/cm, respectively, when the contact length is about 10.0 mm, the contact length of the intervention assembly 12 and the bronchus increases, the field strength at the marking point decreases, but the minimum field strength is not less than 1000 V/cm, which can meet the threshold field strength when the preset ablation effect is reached. Obviously, in this application, when the inner diameter of the bronchus is reduced or the wall thickness of the bronchus increases, the contact length between the intervention assembly 12 and the bronchus is increased, and no greater influence is generated on the ablation effect.

Specifically, the target parameter determination module 026 comprises a simulation ablation model construction unit 0261.

The simulation ablation model construction unit 0261 is configured to perform ablation effect simulation based on the object attribute information, the initial ablation parameter, the impedance data, the abutment parameter, the dielectric constant, the operation object three-dimensional model, and the navigation three-dimensional model as input of the ablation evaluation model, to obtain a simulation ablation model corresponding to the initial ablation parameter, where the simulation ablation model is configured to represent ablation effect data corresponding to the initial ablation parameter.

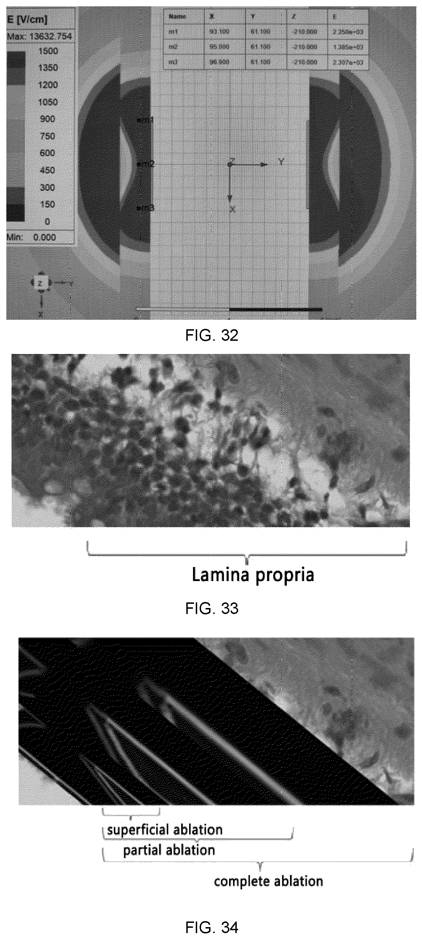

It should be noted that the simulation ablation model construction unit 0261 inputs the object attribute information, the initial ablation parameter, the impedance data, the contact parameter, the dielectric constant, the operation object three-dimensional model and the navigation three-dimensional model together into the ablation evaluation model for ablation effect simulation to obtain a simulation ablation model corresponding to the initial ablation parameter. The ablation evaluation model of the present application may be an ablation evaluation database established by pre-simulated data and clinically collected data. The pre-simulated data is relationship data between a bronchial inner diameter, an ablation range, a contact parameter, a pulse parameter, and a bronchial thickness and an ablation effect obtained by simulation according to impedance data. According to the simulation ablation model, the ablation range and the ablation effect of the pulse energy generated based on the initial ablation parameter on the lung tissue can be visually observed, and the operator can be intuitively helped to judge whether the initial ablation parameter needs to be adjusted or not. As shown in FIG. 32, the ablation effect of the pulse energy in the simulated ablation model generated when the pulse voltage amplitude is 1500 V on the lung tissue can be seen, and it can be seen that most of the regions in the bronchus are ablated.

In some embodiments, in the simulation ablation model, the ablation range and the ablation effect of the lung tissue based on different initial ablation parameters may be distinguished based on the color of the ablation model, and the operator may adjust the initial ablation parameter according to the simulation ablation model, or may avoid possible risks. As shown in FIG. 33, in the pathological structure diagram of the bronchial tissue, as shown in the simulation model shown in FIG. 34, the ablation effects of pulse energy generated by predicting different initial ablation parameters on superficial ablation, partial ablation and complete ablation of the bronchial tissue mucosa layer can be seen.

It should be noted that, after the ablation effect data corresponding to the obtained initial ablation parameter meets the target ablation condition, the initial ablation parameter is determined as the target ablation parameter. In some possible embodiments, the target ablation condition comprises a lung tissue ablation range and an ablation effect observed by using the simulation ablation model to achieve an effect of ablation, and the dielectric constant of the to be ablated object after ablation meets a preset condition, that is, the content of different types of cells in the bronchial tissue after ablation reaches a normal organization standard.

It should be noted that, in the present application, the object attribute information, the initial ablation parameter, the impedance data, the abutment parameter, the dielectric constant, the operation object three-dimensional model and the navigation three-dimensional model can be input into the ablation evaluation model together before ablation to perform ablation effect simulation to obtain a simulation ablation model, so as to indicate the ablation effect before ablation in advance according to the simulation ablation model, thereby optimizing the initial ablation parameter and the ablation frequency, thereby improving the effectiveness of treatment. In some other embodiments, the updated object attribute information, the ablation parameter, the impedance data, the contact parameter, the dielectric constant, the operation object three-dimensional model, and the navigation three-dimensional model may be input into the ablation evaluation model together after ablation to perform effect simulation after ablation, thereby helping to determine whether ablation is valid. The ablation effect evaluation of the ablation effect after ablation may also be configured to optimize an ablation evaluation database, to help optimize and learn, so as to improve the quality of evaluation.

The navigation control device 2 comprises a pulse energy generation module 027

The pulse energy generation module 027 is configured to generate an energy generation control signal based on the target ablation parameter to control the ablation device 4 to operate.

Specifically, the navigation control device 2 further comprises an ablation three-dimensional model generation module 028, configured to obtain ablation trajectory information and ablation data of the intervention assembly 12 after the intervention assembly 12 performs ablation processing on the to be ablated object; and generate an ablation three-dimensional model according to the ablation trajectory information, the ablation data, and the target ablation parameter, where the ablation data comprises ablation location information of the ablation region and the ablation region that have been ablated, and the ablation three-dimensional model represents a three-dimensional spatial feature of the ablated object to be ablated.

It should be noted that the ablation three-dimensional model in this application is configured to record ablation trajectory information and ablation data of the intervention assembly 12 in the target intervention region. In some possible implementations, the ablation data comprises ablation location information of the ablation region and the ablation region that have been ablated. In some other possible implementations, the ablation data further comprises information such as an ablation parameter, an ablation time, an ablation state, and the like of the ablation region and the ablation region that have been ablated. Through the ablation three-dimensional model of the present application, an operator can review and observe ablation trajectory information and ablation data of the intervention assembly 12 at any time, and in a "complex" lung bronchus "maze", the information is that an operator determines which parts have been ablated and which parts are still not ablated, which provides a very important data support, which can effectively improve the surgical efficiency and reduce the possibility of repeated ablation of the surgery.

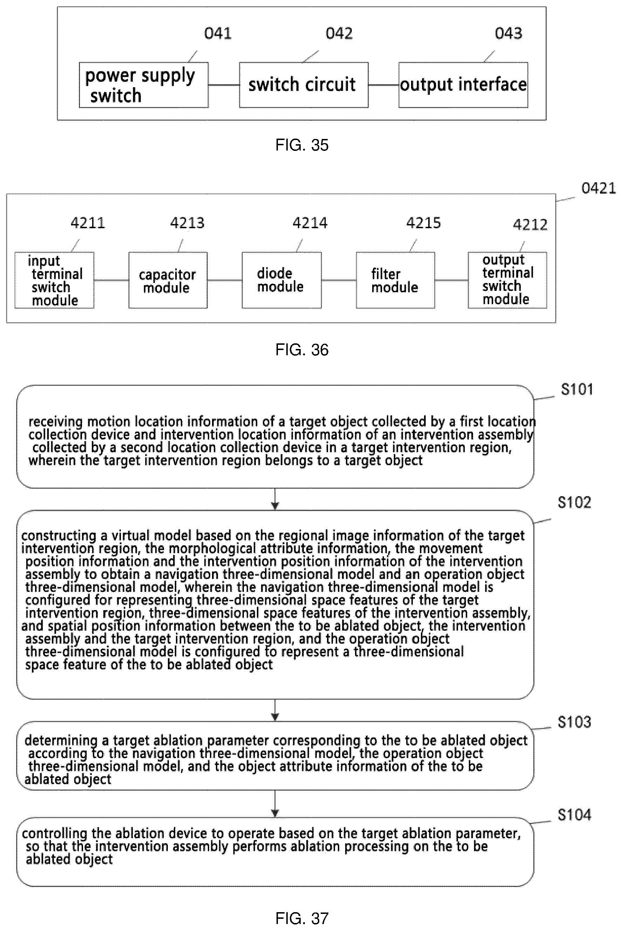

As shown in FIGS. 35 and 36, the control circuit of the ablation device 4 of the present application comprises a pulse power supply 041, a switch circuit 042 and an output interface 043, wherein the pulse power supply 041 is electrically connected to the switch circuit 042, the output interface 043 is configured to be electrically connected to the interventional device 1, each branch circuit 0421 comprises an input terminal switch module 4211 and an output terminal switch module 4212, and the switch circuit 042 controls the corresponding input terminal switch module 4211 and the output terminal switch module 4212 to conduct in response to the energy generation control signal of the pulse energy generation module 027 to generate pulse energy.

Further, the branch circuit 0421 further comprises a capacitor module 4213, and the capacitor module 4213 comprises a plurality of capacitor groups connected in series or in parallel. Each capacitor group may comprise different models and different numbers of capacitors according to different outputs. By controlling the startup and disconnection of the input terminal switch module 4211 and the output terminal switch module 4212 on the branch circuit 0421, the capacitor in the capacitor module 4213 can be charged at the input terminal, and the pulse energy output of different voltage amplitudes is realized at the output terminal. In some possible implementations, pulse energy output of different voltage amplitudes may be implemented by controlling the charging and discharging time of the capacitor module 4213. In some other possible implementations, the control of the multi-path controllable branch circuit 0421 and the control of the corresponding input terminal switch module 4211 and the output terminal switch module 4212 on the different branch circuits 0421 may also control the output of the pulse energy and the charging time of the capacitor module 4213, thereby improving the circuit security, and being more convenient and efficient.

Further, the branch circuit 0421 further comprises a diode module 4214, the diode module 4214 comprises a plurality of diodes, and a forward and reverse arrangement combination is presented between the plurality of diodes. Due to the arrangement of the diode module 4214, the state of each switch on the different branch circuits 0421 is controlled in the charging and discharging process of the switch circuit 042, so that a specific capacitor can be charged or discharged, meanwhile, the capacitance of the capacitor group cannot be reversely charged during discharging, and the unnecessary loss of energy in the capacitor bank capacitor is reduced.

Further, the branch circuit 0421 further comprises a filter module 4215, the filter module 4215 comprises a plurality of filtering units, the filtering unit comprises a plurality of filters and a filtering selector, and the filtering selector is configured to select different filters to perform filtering processing. In some possible implementations, the filtering unit comprises one or more of a low-pass filter, a high-pass filter, a band-pass filter, a band-stop filter, and an all-pass filter. The energy generation unit of this application may implement output of pulse energy of different frequencies and forms by performing filtering processing by using different filters or different filter combinations. In some possible implementations, different filters or different filter combinations are selected for filtering, and pulse energy output of different waveforms or combined waveforms such as sine waves, square waves and triangular waves may be implemented.

In some possible implementations, the branch circuit 0421 may comprise, but is not limited to, an input terminal switch module 4211, a capacitor module 4213, a diode module 4214, a filter module 4215, and an output terminal switch module 4212.

The switching circuit of the ablation device of the present application may implement several combinations of amplitude, pulse width, interval, quantity, or direction of the pulse energy by controlling the connection of the plurality of branch circuits 0421 and the control of the corresponding input terminal switch module 4211 and the output terminal switch module 4212 on the different branch circuits 0421. By cooperating with the control and filter module 4215 of the corresponding input terminal switch module 4211 and the output terminal switch module 4212 on the branch circuit 0421, output of pulse energy of different frequencies and forms can be realized. Based on the switching circuit of the present application, the ablation device may have different voltage amplitudes, frequencies, and pulse morphology changes in a group of pulse energy to generate more targeted pulse energy, which is beneficial to reducing the stimulation reaction problem caused in the ablation treatment process while increasing the treatment effect, and improving the treatment safety.

Specifically, the step of applying the subject ablation system of the present application to treat a lung bronchial disease comprises:
feeding the intervention assembly 12 in the contracted state into the pulmonary bronchus through the operating handle 16;
collecting movement position information of the target object, and transmitting the movement position information to the navigation control device 2;
acquiring, by the acquisition device, the intervention position information of the intervention assembly 12 in the lung bronchus, and transmitting the intervention position information to the navigation control device 2;
performing virtual model construction based on the lung CT image to obtain a lung bronchial three-dimensional model and a lesion tissue three-dimensional model; performing virtual model construction according to the morphological attribute information of the intervention assembly 12 to obtain an intervention assembly sub-model; performing spatial fusion processing on the lung bronchial three-dimensional model, the lesion tissue three-dimensional model and the intervention assembly sub-model according to the intervention position information and the movement position information to obtain a navigation three-dimensional model;
performing navigation planning according to the lung bronchial three-dimensional model and the lesion tissue three-dimensional model to obtain a navigation path of the intervention assembly 12 in the lung bronchial three-dimensional model, and generating corresponding two-dimensional and three-dimensional navigation data;
during the movement of the intervention assembly 12 in the lung bronchus, correcting the real-time intervention position of the intervention assembly 12 in the lung bronchus at any time according to the navigation path until the intervention assembly 12 reaches the lesion tissue area in the lung bronchus;
controlling the expansion degree of the intervention assembly 12 by the manipulation assembly 17 on the operating handle 16, so that the intervention assembly 12 is uniformly attached to the lesion tissue;
obtaining an initial ablation parameter corresponding to the object attribute information of the lesion tissue, impedance data of the lesion tissue, an abutment parameter of the intervention assembly 12, and a dielectric constant of the lesion tissue;
performing ablation effect simulation based on the object attribute information, the initial ablation parameter, the impedance data, the abutment parameter, the dielectric constant, the lesion tissue three-dimensional model and the navigation three-dimensional model as input of the ablation evaluation model to obtain a simulation ablation model corresponding to the initial ablation parameter;
determining a target ablation parameter according to the simulation ablation model if the ablation effect data corresponding to the initial ablation parameter meets a target ablation condition; and
generating an energy generation control signal based on the target ablation parameter to control the ablation device 4 to operate; and
controlling the corresponding switch circuit 042 by the ablation device 4 to conduct in response to the energy generation control signal to generate pulse energy;
transmitting the pulse energy to the interventional device 1, so that the intervention assembly 12 performs ablation processing on the lesion tissue; and
acquiring ablation trajectory information and ablation data of the intervention assembly 12, and generating an ablation three-dimensional model according to the ablation trajectory information, the ablation data, and the target ablation parameter.

The present application further provides an object ablation control method, as shown in FIG. 37, which is a flowchart of an object ablation control method, and the method comprises:
S 101: receiving motion location information of a target object collected by a first location collection device and intervention location information of an intervention assembly collected by a second location collection device in a target intervention region, wherein the target intervention region belongs to a target object;
S 102: constructing a virtual model based on the regional image information of the target intervention region, the morphological attribute information, the movement position information and the intervention position information of the intervention assembly to obtain a navigation three-dimensional model and an operation object three-dimensional model, wherein the navigation three-dimensional model is configured for representing three-dimensional space features of the target intervention region, three-dimensional space features of the intervention assembly, and spatial position information between the to be ablated object, the intervention assembly and the target intervention region, and the operation object three-dimensional model is configured to represent a three-dimensional space feature of the to be ablated object;
S 103: determining a target ablation parameter corresponding to the to be ablated object according to the navigation three-dimensional model, the operation object three-dimensional model, and the object attribute information of the to be ablated object. S 104: controlling the ablation device to operate based on the target ablation parameter, so that the intervention assembly performs ablation processing on the to be ablated object.

Further, step S 102 further comprises:
S 1021: Perform image recognition processing on the region image information of the target intervention region to obtain an image recognition result; and perform image reconstruction based on the image recognition result to obtain an intervention region sub-model and an operation object three-dimensional model, where the intervention region sub-model is configured to represent a three-dimensional space feature of the target intervention region.
S 1022: Perform virtual model construction according to the morphological attribute information of the intervention assembly to obtain an intervention assembly sub-model, where the intervention assembly sub-model is configured to represent a three-dimensional space feature of the intervention assembly.
S 1023: performing spatial fusion processing on the intervention region sub-model, the operation object three-dimensional model and the intervention assembly sub-model according to the intervention position information and the movement position information to obtain a navigation three-dimensional model.

Further, step S 102 further comprises:
S 1024: performing navigation planning according to the intervention region sub-model and the operation object three-dimensional model to obtain a navigation path of the intervention assembly in the intervention region sub-model, where the navigation path is configured to indicate a travel path of a required path in the process of reaching the to be ablated object in the target intervention region by the intervention assembly.

Further, step S 102 further comprises:
S 1025: determining, in a movement process of the intervention assembly in the target intervention region, intervention trajectory information of the intervention assembly based on the intervention position information and the movement position information of the intervention assembly; and if the path trajectory information corresponding to the intervention trajectory information and the navigation path meets a preset deviation condition, correcting the intervention position of the intervention assembly in the target intervention region until the updated intervention trajectory information of the intervention assembly matches the path trajectory information.

Further, step S 103 further comprises:
S 1031: Obtain an initial ablation parameter corresponding to the object attribute information of the to be ablated object, impedance data of the to be ablated object, a contact parameter of the intervention assembly, and a dielectric constant of the to be ablated object, where the initial ablation parameter at least comprises at least one of a pulse voltage, a pulse width, a number of pulses, and a number of pulse groups.
S 1032: performing an ablation effect evaluation according to the navigation three-dimensional model, the operation object three-dimensional model, the object attribute information of the to be ablated object, the initial ablation parameter corresponding to the object attribute information of the to be ablated object, the impedance data of the to be ablated object, the abutment parameter of the intervention assembly and the dielectric constant of the to be ablated object to obtain ablation effect data corresponding to the initial ablation parameter; and if the ablation effect data meets the target ablation condition, determining the initial ablation parameter as the target ablation parameter.

Further, step S 103 further comprises:
S 1033: Perform ablation effect simulation based on the object attribute information, the initial ablation parameter, the impedance data, the abutment parameter, the dielectric constant, the operation object three-dimensional model, and the navigation three-dimensional model as input of the ablation evaluation model, to obtain a simulation ablation model corresponding to the initial ablation parameter, where the simulation ablation model is configured to represent ablation effect data corresponding to the initial ablation parameter.

Further, step 104 further comprises:
S 1041: generating an energy generation control signal based on the target ablation parameter to control the ablation device to operate.

Further, the object ablation control method further comprises:
S 105: after the intervention assembly performs ablation processing on the to be ablated object, obtaining ablation trajectory information and ablation data of the intervention assembly; and generating an ablation three-dimensional model according to the ablation trajectory information, the ablation data, and the target ablation parameter, the ablation data including ablation location information of the ablation region and the ablation region that have been ablated, and the ablation three-dimensional model representing a three-dimensional spatial feature of the ablation object after ablation.

This application further provides an object ablation control apparatus, and the apparatus may comprise the following modules:
an information receiving module, configured to receive motion location information of a target object collected by a first location collection device and intervention location information of an intervention assembly collected by a second location collection device in a target intervention region, wherein the target intervention region belongs to a target object; and
a model construction module, configured to construct a virtual model based on the regional image information of the target intervention region, the morphological attribute information, the movement position information and the intervention position information of the intervention assembly to obtain a navigation three-dimensional model and an operation object three-dimensional model, wherein the navigation three-dimensional model is configured for representing three-dimensional space features of the target intervention region, three-dimensional space features of the intervention assembly, and spatial position information between the to be ablated object, the intervention assembly and the target intervention region, and the operation object three-dimensional model is configured for representing a three-dimensional space feature of the to be ablated object;
a parameter determining module, configured to determine a target ablation parameter corresponding to the to be ablated object according to the navigation three-dimensional model, the operation object three-dimensional model, and the object attribute information of the to be ablated object; and
wherein the object control module is configured to control the ablation device to operate based on the target ablation parameter, so that the intervention assembly performs ablation processing on the to be ablated object.

In some embodiments, the model construction module comprises:
an intervention region sub-model construction module, configured to perform image recognition processing on the region image information of the target intervention region to obtain an image recognition result; and perform image reconstruction based on the image recognition result to obtain an intervention region sub-model and an operation object three-dimensional model, where the intervention region sub-model is configured to represent a three-dimensional space feature of the target intervention region;
the intervention assembly sub-model construction module, configured to perform virtual model construction according to the morphological attribute information of the intervention assembly to obtain an intervention assembly sub-model, where the intervention assembly sub-model is configured to represent a three-dimensional space feature of the intervention assembly;
a fusion module, configured to perform spatial fusion processing on the intervention region sub-model, the operation object three-dimensional model and the intervention assembly sub-model according to the intervention position information and the movement position information to obtain a navigation three-dimensional model.

Further, the model construction module further comprises a navigation planning module, and the navigation planning module is communicatively connected to the navigation three-dimensional model.

The navigation planning module is configured to perform navigation planning according to the intervention region sub-model and the operation object three-dimensional model to obtain a navigation path of the intervention assembly in the intervention region sub-model, where the navigation path is configured to indicate a travel path of a required path in the process of reaching the to be ablated object in the target intervention region by the intervention assembly.

Further, the navigation planning module is further configured to determine, in a movement process of the intervention assembly in the target intervention region, intervention trajectory information of the intervention assembly based on the intervention position information and the movement position information of the intervention assembly; and if the path trajectory information corresponding to the intervention trajectory information and the navigation path meets a preset deviation condition, correct the intervention position of the intervention assembly in the target intervention region, until the updated intervention trajectory information of the intervention assembly matches the path trajectory information.

In some embodiments, the parameter determination module comprises:
the initial parameter acquisition module, configured to obtain an initial ablation parameter corresponding to the object attribute information of the to be ablated object, impedance data of the to be ablated object, an abutment parameter of the intervention assembly, and a dielectric constant of the to be ablated object, where the initial ablation parameter at least comprises at least one of a pulse voltage, a pulse width, a number of pulses, and a number of pulse groups;
the target parameter determination module, configured to perform an ablation effect evaluation according to the navigation three-dimensional model, the operation object three-dimensional model, the object attribute information of the to be ablated object, the initial ablation parameter corresponding to the object attribute information of the to be ablated object, the impedance data of the to be ablated object, the abutment parameter of the intervention assembly, and the dielectric constant of the to be ablated object to obtain ablation effect data corresponding to the initial ablation parameter; and if the ablation effect data meets the target ablation condition, determine the initial ablation parameter as the target ablation parameter.

Further, the target parameter determination module further comprises:
a simulation ablation model construction unit, configured to perform ablation effect simulation based on the object attribute information, the initial ablation parameter, the impedance data, the abutment parameter, the dielectric constant, the operation object three-dimensional model, and the navigation three-dimensional model as input of the ablation evaluation model, to obtain a simulation ablation model corresponding to the initial ablation parameter, where the simulation ablation model is configured to represent ablation effect data corresponding to the initial ablation parameter.

Further, the object control module comprises:
the pulse energy generation module, configured to generate an energy generation control signal based on the target ablation parameter to control the ablation device to operate.

In some embodiments, the object ablation control apparatus further comprises:
an ablation three-dimensional model generation module, configured to obtain ablation trajectory information and ablation data of the intervention assembly after the intervention assembly performs ablation processing on the to be ablated object; and generate an ablation three-dimensional model according to the ablation trajectory information, the ablation data, and the target ablation parameter, where the ablation data comprises ablation location information of the ablation region and the ablation region that have been ablated, and the ablation three-dimensional model represents a three-dimensional spatial feature of the ablation object after ablation.

The subject ablation system, control method, apparatus, medium, and electronic device of the present application further provide a pulse ablation effect evaluation method and apparatus, which can be applied to ablation therapy of lung tissue. However, the present application is not limited thereto, and the technical solutions provided in the embodiments of this application may further ablate other body parts, such as a stomach. The following describes the embodiments of this application by taking lung ablation as an example.

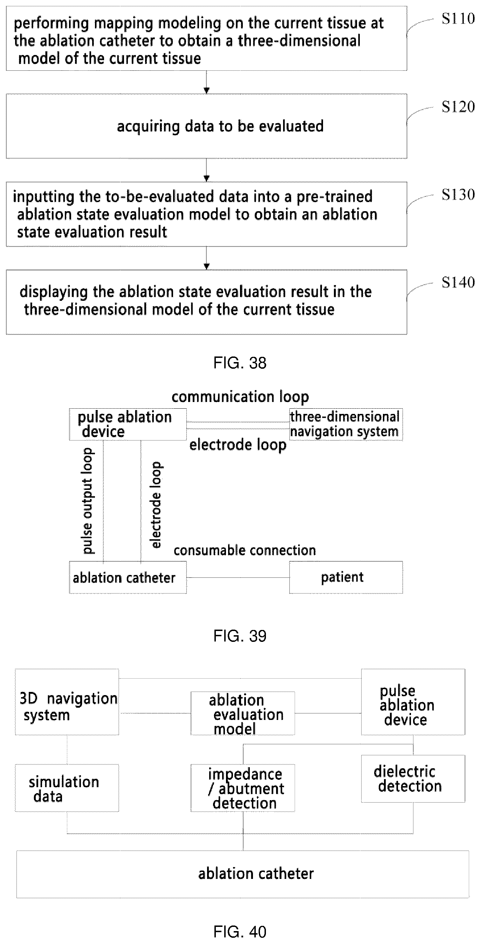

FIG. 38 is a flowchart of a pulse ablation effect evaluation method according to an embodiment of this application, as shown in FIG. 38, the pulse ablation effect evaluation method provided in this embodiment of this application may comprise the following steps:
S 110: performing mapping modeling on the current tissue at the ablation catheter to obtain a three-dimensional model of the current tissue;
In this embodiment of this application, the three-dimensional navigation system and the endoscope are used in cooperation. The lung bronchus is modeled in the surgery process through the three-dimensional navigation system, a lung model during current surgery is constructed, and an operator can observe the overall bronchial structure and trend of the lung from the navigation interface. Meanwhile, the position and internal condition of the lung bronchus can be visually observed through the endoscope. In this way, the whole bronchial structure and trend of the lung can be observed, the position and internal condition of the lung bronchus can be observed, and compared with the prior art, the condition of the lung can be more comprehensively understood.

In addition, in this embodiment of this application, a function of prediction and ablation navigation is further matched, a doctor can input an initial parameter, the doctor enters the lung through cooperation of the catheter and the endoscope, and the catheter predicts the depth and range of ablation according to the input parameter and the current thickness and position of the trachea, and presents the depth and range to the doctor for the doctor to refer to and adjust the parameter.

FIG. 39 is a connection block diagram of a pulse ablation effect evaluation system according to an embodiment of this application. As shown in FIG. 39, the ablation catheter is connected to the pulsed electric field ablation device through a pulse output loop and an electrode loop, respectively, the pulsed electric field ablation device is connected to the three-dimensional navigation system through a communication loop and an electrode loop, and the ablation catheter is connected to the patient through a consumable.

FIG. 40 is a schematic block diagram of a pulse ablation effect evaluation method according to an embodiment of this application, and specifically, as shown in FIG. 40, the three-dimensional navigation system collects data through magnetic positioning. The ablation catheter determines the impedance of the current tissue and the abutting state of the catheter through impedance/abutment detection, determines the diameter of the inner wall of the tissue through the opening and abutting range of the basket catheter, and determines the current tissue characteristic by collecting the dielectric constant. The information obtained by the dielectric constant can obtain some characteristics of the corresponding tissue, because different cells have different electroporation thresholds, and then can be configured to evaluate whether the energy of ablation can meet the requirements.

The ablation catheter transmits the collected impedance, dielectric constant and basket catheter data to the pulsed electric field ablation system, and inputs the impedance, the dielectric constant and the basket catheter data into the ablation state evaluation model together with the ablation parameters set by the pulsed electric field ablation system for calculation; the three-dimensional navigation system can perform data interaction with the pulse ablation device; and the three-dimensional navigation system can generate a three-dimensional model of the current tissue required by the ablation effect evaluation, where the current tissue herein may be lung bronchial tissue.

S 120: acquiring data to be evaluated;
specifically, the to-be-evaluated data comprises detection data collected by the ablation catheter, ablation parameters of the ablation device, and basket catheter state data;
specifically, the ablation parameter comprises one or more of a pulse voltage, a pulse width, a number of pulses, and a number of pulse groups;
specifically, the basket catheter state data comprises the basket catheter opening state data and the contact area between the basket catheter and the current tissue, and the basket catheter opening state data and the abutment area are configured to determine the thickness of the to be ablated area. The diameter of the bronchial inner wall (that is, the inner diameter of the bronchus, rather than the outer diameter of the bronchus, and the outer diameter of the bronchus is cartilage) can be determined through the opening size and the abutment area of the basket catheter, so that the thickness of the bronchial mucosa layer is obtained, and the to be ablated area here is the partial area of the mucosal layer;
regarding lung bronchial tissue structure: the bronchus is mainly divided into: epithelial cells (a ciliated false compound layer, a cup-shaped cell sandwiched between the dummy layers, and a small amount of mucus secreted by the cup-shaped cells); an intrinsic layer (the base film and the loose connective tissue together with the epithelial cells and the intrinsic layer, constituting a mucous membrane); a mucosal layer (smooth muscle & AMP; more connective tissue). The mucus gland located at the connective tissue is a place secreting most of the mucus, and in scenarios such as ablation of the lung, the pulsed ablation object is mainly a mucus gland and a cup-shaped cell in the ablation mucosal layer;
S 130: inputting the to-be-evaluated data into a pre-trained ablation state evaluation model to obtain an ablation state evaluation result, wherein the ablation state evaluation model can calculate an ablation state evaluation result corresponding to the to-be-evaluated data according to the to-be-evaluated data and the correspondence between the to-be-evaluated data and the ablation state, and the ablation state evaluation result comprises an ablation range, an ablation depth, and an ablation effective degree;
in some possible embodiments, the ablation effective degree can be judged by comparing the dielectric constants before and after ablation, because the thresholds of different cell tissues under the electroporation are different, and the effective degree of evaluating the ablation parameters can be helped by knowing the contents of different types of cells in the bronchial tissues;
lung ablation is mainly to ablate abnormal tissues (such as cup-shaped cells and mucus glands) in a bronchial mucosal layer, so as to input an ablation state evaluation model by means of simulation data, impedance/close data, and dielectric parameters in cooperation with a currently set ablation parameter, so that an ablation effect can be effectively evaluated;
the simulation data is relationship data between a bronchial inner diameter, an ablation range, a contact parameter, a pulse parameter, and a bronchial thickness and an ablation effect obtained by simulation according to impedance data;
the evaluation process may be before ablation, so that the effect before ablation may be indicated in advance, thereby guiding the parameters and times of the ablation of the operator. Of course, after ablation, such an operator may help determine whether ablation is valid. Moreover, after ablation, the range and effect of ablation may be determined by means of re-mapping evaluation to optimize, thereby improving the effectiveness of treatment;
S 140: displaying the ablation state evaluation result in the three-dimensional model of the current tissue;
based on the bronchial pathological structure diagram shown in FIG. 33 in the three-dimensional system, the ablation evaluation after the ablation parameters are indicated by the color of the ablation model can be distinguished based on the bronchial pathological structure diagram shown in FIG. 33, so as to obtain the ablation range prediction map shown in FIG. 34, and the operator can be clear according to the model, so that the ablation data can be optimized, and possible risks can also be avoided;
in an embodiment, after inputting the to-be-evaluated data into a pre-trained ablation state evaluation model to obtain an ablation state evaluation result, the method comprises:
   storing the to-be-evaluated data and the ablation state evaluation result in the sample data to obtain updated sample data; and
   training and updating the ablation state evaluation model based on the updated sample data.

The ablation state evaluation model may perform non-stop optimization and learning on the ablation after each ablation, because the feedback action range, the impedance abutment information, the dielectric parameter, and the ablation parameter have a certain update after pulse ablation, so that the ablation state evaluation model may be optimized, thereby improving the quality of evaluation.

In an embodiment, before inputting the to-be-evaluated data into the pre-trained ablation state evaluation model, the method comprises:
acquiring sample data, wherein the sample data may be data including the to-be-evaluated data, the ablation state, and the correspondence between the to-be-evaluated data and the ablation state obtained through pre-simulation, or may be data collected clinically and including the to-be-evaluated data, the ablation state, and the correspondence between the to-be-evaluated data and the ablation state; or may be a combination of the simulation data and the clinically collected data, so that the number of samples may be increased, and the workload of collecting the sample data may be reduced;
training a pre-constructed machine learning model by using the sample data, and adjusting parameters of the machine learning model in the training process until an ablation state evaluation result output by the machine learning model meets a requirement; and storing the machine learning model as an ablation state evaluation model.

Certainly, this model training process may be completed in a process of performing navigation mapping modeling through the catheter in the three-dimensional navigation system.

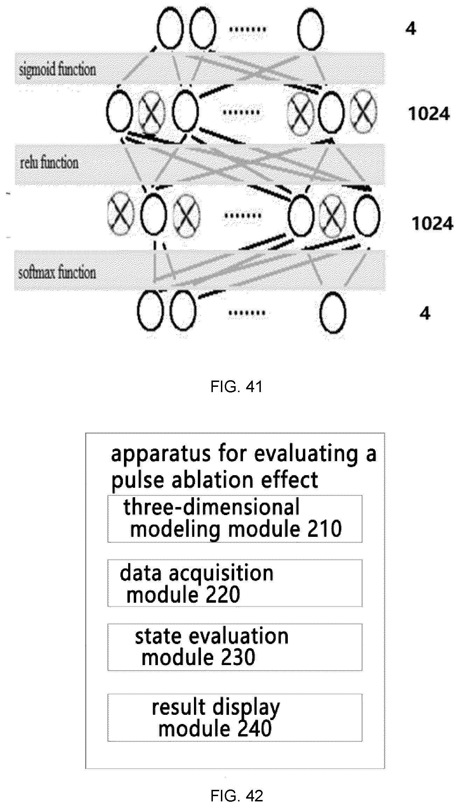

The ablation state evaluation model may be a machine learning model, specifically a neural network model. FIG. 41 is an example of an ablation state evaluation model according to an embodiment of this application. As shown in FIG. 41, in an embodiment, the ablation state evaluation model uses a full neural network (for example, TensorFlow) as a main algorithm, selects an Encode and a Decode as a neural network machine learning framework, selects three functions, i.e. Sigmold, ReLU, and Softmax as an activation function of each layer of network, and selects a fully connected neural network and a total of five layers. The network structure is divided into four types: an input layer, a hidden layer, a dropout layer, and an output layer; The entire neural network respectively selects three functions of Sigmold, ReLU and Softmax as an activation function of each layer of network. The loss function is a cross entropy. There are two parameter optimization methods: AdamOptimizer and GradientDescentOptimizer, with a learning rate of 1e-4. The input and output of the model correspond to each other, with a shape of (1*4) for both.

In an embodiment, displaying the ablation state evaluation result in the three-dimensional model of the current tissue comprises the follows:
determining a display color corresponding to the ablation state evaluation result according to the ablation state evaluation result and the correspondence between the ablation state evaluation result and the display color; preferably, different ablation states correspond to different display colors, so that the ablation state of each area can be intuitively reflected; wherein the correspondence between the ablation state and the display color is preferably displayed in the form of a legend;
displaying the ablation state evaluation result and/or the display color corresponding to the ablation state in the three-dimensional model of the current tissue, wherein the ablation state evaluation result comprises an ablation range, an ablation depth, and an ablation effective degree.

As shown in FIG. 32, the ablation effect can be represented by different color depth changes, so that a doctor can intuitively observe the range and effect of lung tissue ablation, and can intuitively help a doctor to determine whether the ablation parameter needs to be adjusted.

Specifically, that the detection data comprises an impedance signal to obtain the to-be-evaluated data comprises:
loading a first signal to an electrode of the ablation catheter, wherein a frequency range of the first signal is a first preset frequency range, and the first preset frequency range is preferably 2 KHz to 200 KHz;
collecting a feedback signal after the first signal acts on the electrode; and
filtering the feedback signal to convert the feedback signal into an impedance signal.

The impedance detection method may adopt one or more of time-division switching detection, fusion extraction detection, and frequency division switching detection.

Here, the impedance signal may indicate the current load condition, and the impedance signal and the ablation parameter, such as the pulse voltage, the pulse width, the number of pulses, and the number of pulse groups, may calculate the energy currently loaded to the tissue. The contact quality and area may also be determined by the change rate of the impedance. The size of the abutment area can determine the quality of ablation.

Specifically, that the detection data comprises a dielectric constant signal to obtain the to-be-evaluated data comprises:
applying the sinusoidal excitation signal to the current tissue through the electrode of the ablation catheter; the frequency range of the sinusoidal excitation signal is a second preset frequency range; and the second preset frequency range is preferably 5 KHz to 300 MHz.

The complex impedance electrical signal returned after the sinusoidal excitation signal with different frequencies acts on the current tissue is obtained, and the complex impedance electrical signal is used as the dielectric constant signal.

The detection of the dielectric constant is to generate a sinusoidal excitation signal with a plurality of frequencies in the range of 5KHz to 300 MHz, to excite the change of the complex impedance electrical signal corresponding to the tissue of the reaction cell under different frequency signals. These signals are converted into frequencydomain signals by means of wavelet transform, Fourier variation, and the like. The dielectric constants of different tissue parameters are different, and the proportions of different tissues can be guided by different dielectric constant ratios to determine the content of the cells. Because the thresholds of different cell tissues under electroporation are different, by knowing the levels of different types of cells in the bronchial tissue, the degree of validity of the parameters of the ablation can be evaluated.

The relationship between the inside diameter of the bronchus and the product contact length can be obtained experimentally. According to the data, the correspondence between the to-be-evaluated data and the ablation state may be further obtained through simulation, and the correspondence between the to-be-evaluated data and the ablation state is preferably an ablation state, a contact data, a pulse parameter, and a bronchial thickness, and specifically, the ablation state comprises an ablation range and an ablation depth.

FIG. 42 is a structural block diagram of an apparatus for evaluating a pulse ablation effect according to an embodiment of this application, and specifically, as shown in FIG. 42, the apparatus for evaluating a pulse ablation effect according to an embodiment of this application may comprise the following modules:
a three-dimensional modeling module 210, configured to perform mapping modeling on the current tissue at the ablation catheter to obtain a three-dimensional model of the current tissue; and
a data acquisition module 220, configured to obtain to-be-evaluated data, where the to-be-evaluated data comprises detection data collected by an ablation catheter, ablation parameters of an ablation device, and basket catheter state data; and
a state evaluation module 230, configured to input the to-be-evaluated data into a pre-trained ablation state evaluation model to obtain an ablation state evaluation result; the ablation state evaluation model can calculate an ablation state evaluation result corresponding to the to-be-evaluated data according to the to-be-evaluated data and the correspondence between the to-be-evaluated data and the ablation state; and
the result display module 240, configured to display the ablation status evaluation result in the three-dimensional model of the current tissue;
in an embodiment, the apparatus further comprises a feedback module, and the feedback module comprises a sample data updating module and a model updating module,
wherein the sample data updating module is configured to save the to-be-evaluated data and the ablation state evaluation result into the sample data to obtain updated sample data; and
the model updating module, configured to train and update the ablation state evaluation model based on the updated sample data;
in an embodiment, the apparatus further comprises a model training module, and the model training module comprises a sample data acquisition module and a parameter adjustment module;
the sample ata acquisition module, configured to obtain sample data before inputting the to-be-evaluated data into a pre-trained ablation state evaluation model; and
the parameter adjustment module, configured to train a pre-constructed machine learning model by using the sample data, and adjust parameters of the machine learning model in the training process until an ablation state evaluation result output by the machine learning model meets a requirement; and save the machine learning model as an ablation state evaluation model.

In one embodiment, the result presentation module 240 comprises:
a display color determining module, configured to determine a display color corresponding to the ablation state evaluation result according to the ablation state evaluation result and the correspondence between the ablation state evaluation result and the display color; and
a display module, configured to display an ablation state evaluation result and/or a display color corresponding to the ablation state in the three-dimensional model of the current tissue, wherein the ablation state evaluation result comprises an ablation range and an ablation depth.

In an embodiment, the detection data comprises an impedance signal, and the data acquisition module 220 comprises:
a first signal loading module, configured to load a first signal to an electrode of an ablation catheter, where a frequency range of the first signal is a first preset frequency range; and
a rotation signal acquisition module, configured to acquire a return signal of a first signal acting on an electrode; and
the signal conversion module, configured to perform filtering processing on the feedback signal to convert the feedback signal into an impedance signal.

In an embodiment, the detection data comprises a dielectric constant signal, and the data acquisition module 220 comprises:
a second signal loading module, configured to apply a sinusoidal excitation signal to a current tissue through an electrode of the ablation catheter; a frequency range of the sinusoidal excitation signal is a second preset frequency range; and
a dielectric constant obtaining module, configured to obtain a complex impedance electrical signal returned after the sinusoidal excitation signal with different frequencies act on the current tissue, and use the complex impedance electrical signal as a dielectric constant signal.

It should be noted that the above apparatus embodiments and method embodiments are based on the same embodiment.

The navigation control device and the ablation device of the object ablation system of the present application may run on a terminal or a server, and comprise a processor and a memory, where the memory stores at least one instruction or at least one program, and the at least one instruction or the at least one program is loaded and executed by the processor to implement the object ablation control method and the pulse ablation effect evaluation method provided in the foregoing method embodiments.

The memory may be configured to store a software program and a module, and the processor executes various functional applications and object ablation operations by running software programs and modules stored in the memory. The memory may mainly comprise a storage program area and a storage data area, where the storage program area may store an operating system, an application program required by a function, and the like; and the storage data area may store data created according to use of the device, and the like. In addition, the memory may comprise a high-speed random access memory, and may further comprise a non-volatile memory, for example, at least one magnetic disk storage device, a flash memory device, or another volatile solid-state storage device. Correspondingly, the memory may further comprise a memory controller to provide access to the memory by the processor.

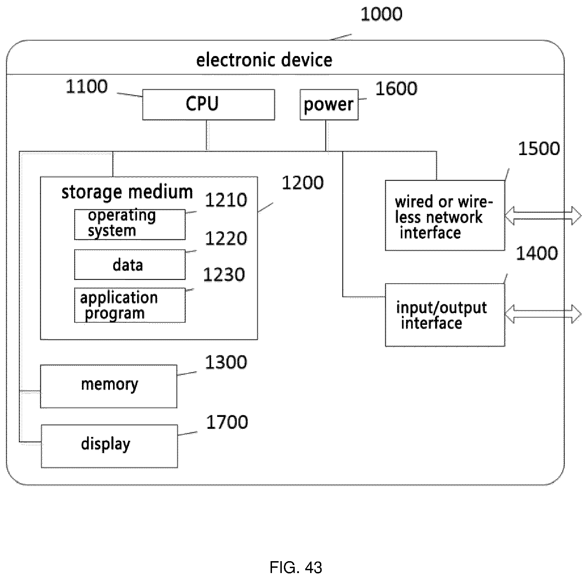

The method embodiments provided in the embodiments of this application may be performed in an electronic device such as a mobile terminal, a computer terminal, a server, or a similar computing apparatus. FIG. 43 is a block diagram of a hardware structure of an electronic device for an object ablation control method according to an embodiment of this application. As shown in FIG. 43, the electronic device 1000 may generate a relatively large difference due to different configuration or performance, and may comprise one or more central processing units (CPU) 1100 (the processor 1100 may comprise, but is not limited to, a processing apparatus such as a microprocessor MCU or a programmable logic device FPGA), a memory 1300 for storing data, and one or more storage media 1200 (for example, one or more mass storage devices) storing application programs 1230 or data 1220. The memory 130 and the storage medium 1200 may be transient storage or persistent storage. The program stored in the storage medium 1200 may comprise one or more modules, and each module may comprise a series of instruction operations for the electronic device. Further, the central processor 1100 may be configured to communicate with the storage medium 1200, and execute a series of instruction operations in the storage medium 1200 on the electronic device 1000. The electronic device 1000 may further comprise one or more power supplies 1600, one or more wired or wireless network interfaces 1500, one or more input/output interfaces 1400, one or more displays 1700, and/or one or more operating systems 1210, such as Windows Server TM, MAC OS XTM,UnixTM, LinuxTM, FreeBSDTM, and the like.

The input/output interface 140 may be configured to receive or send data via a network. Specific examples of the above-mentioned network may comprise a wireless network provided by a communication provider of the electronic device 1000. In one example, the input/output interface 1400 comprises a Network Interface Controller (NIC), which may be connected to other network devices through a base station to communicate with the Internet. In an example, the input/output interface 1400 may be a Radio Frequency (RF) module, which is configured to communicate with the Internet in a wireless manner.

The display 1700 may be configured to display the electronic file on the screen through a specific transmission device and then reflect the electronic file to the human eye. The display 1700 of the present disclosure may be configured for interface display, data management display, endoscopic image display, two-dimensional image display, three-dimensional image display, combination of several two-dimensional images and three-dimensional images, multi-dimensional display, etc.

A person of ordinary skill in the art may understand that the structure shown in FIG. 43 is merely an example, and does not limit the structure of the electronic device. For example, the electronic device 1000 may further comprise more or fewer components than those shown in FIG. 43, or have a configuration different from that shown in FIG. 43.

An embodiment of this application further provides a computer-readable storage medium, where the storage medium may be disposed in an electronic device to store at least one instruction or at least one program related to an object ablation control method in the method embodiments, and the at least one instruction or the at least one program is loaded and executed by the processor to implement the object ablation control method and the pulse ablation effect evaluation method provided in the foregoing method embodiments.

Optionally, in this application, the storage medium may be located in at least one of a plurality of network servers of a computer network. Optionally, in this application, the storage medium may comprise, but is not limited to, any medium that can store program code, such as a USB flash disk, a read-only memory (ROM), a random access memory (RAM), a mobile hard disk, a magnetic disk, or an optical disk.

According to an aspect of this application, a computer program product or a computer program is provided, where the computer program product or the computer program comprises computer instructions, and the computer instructions are stored in a computer-readable storage medium. A processor of the computer device reads the computer instruction from the computer-readable storage medium, and the processor executes the computer instruction, so that the computer device performs the method provided in the foregoing optional implementations.

The sequence of the foregoing embodiments of this application is merely for description, and does not represent the advantages and disadvantages of the embodiments. In addition, specific embodiments of this application are described above. Other embodiments are within the scope of the following claims. In some cases, the actions or steps recited in the claims may be performed in an order different from that in the embodiments and still achieve the desired results. In addition, the processes depicted in the figures do not necessarily require the particular order shown or the sequential order to achieve the desired results. In some embodiments, multitasking and parallel processing are also possible or may be advantageous.

The embodiments in this application are all described in a progressive manner, and the same or similar parts between the embodiments may refer to each other, and each embodiment focuses on a difference from other embodiments. In particular, for the embodiments of the apparatus, the device, and the storage medium, since they are substantially similar to the method embodiments, the description is relatively simple, and for the related parts, reference may be made to the description of the method embodiments.

A person of ordinary skill in the art may understand that all or some of the steps of implementing the foregoing embodiments may be completed by using hardware, or may indicate, by using a program, that related hardware is completed.

The preferred embodiments of the present application are shown and described above, and as stated above, it should be understood that the present application is not limited to the forms disclosed herein, should not be construed as excluding other embodiments, but may be used in various other combinations, modifications and environments, and may be modified by the above teachings or techniques or knowledge in the relevant art, within the scope of the disclosure described herein. Modifications and variations made by those skilled in the art do not depart from the spirit and scope of the present disclosure, and shall fall within the protection scope of the appended claims of this application.

## Claims

1. An object ablation system, comprising an interventional device (1), a navigation control device (2), a first position acquisition device (3) and an ablation device (4), wherein the interventional device (1) comprises a second position acquisition device (11) and an intervention assembly (12); the first position acquisition device (3), the second position acquisition device (11), the intervention assembly (12) and the ablation device (4) are communicatively connected to the navigation control device (2), respectively;
the first position acquisition device (3) is configured to acquire movement position information of a target object and transmit the movement position information to the navigation control device (2);
the second position acquisition device (11) is configured to acquire intervention position information of the intervention assembly (12) in a target intervention region, and transmit the intervention position information to the navigation control device (2), where the target intervention region belongs to the target object;
the navigation control device (2) is configured to construct a virtual model based on region image information of the target intervention region, and morphological attribute information, the movement position information, and the intervention position information of the intervention assembly (12) to obtain a three-dimensional navigation model and an operation object three-dimensional model, wherein the navigation three-dimensional model is configured to represent three-dimensional space features of the target intervention region, three-dimensional space features of the intervention assembly (12), and spatial position information between a to be ablated object, the intervention assembly (12), and the target intervention region, and the operation object three-dimensional model is configured to represent a three-dimensional space feature of the to be ablated object;
the navigation control device (2) is further configured to determine a target ablation parameter corresponding to the to be ablated object according to the navigation three-dimensional model, the operation object three-dimensional model, and object attribute information of the to be ablated object; and control the ablation device (4) to operate based on the target ablation parameter, so that the intervention assembly (12) performs ablation processing on the to be ablated object.

2. The object ablation system according to claim 1, wherein the navigation control device (2) comprises:
an intervention region sub-model construction module (21) configured to perform image recognition processing on the region image information of the target intervention region to obtain an image recognition result; and perform image reconstruction based on the image recognition result to obtain an intervention region sub-model and the operation object three-dimensional model, wherein the intervention region sub-model is configured to represent a three-dimensional space feature of the target intervention region;
an intervention assembly sub-model construction module (22) configured to perform virtual model construction according to the morphological attribute information of the intervention assembly (12) to obtain an intervention assembly sub-model, wherein the intervention assembly sub-model is configured to represent three-dimensional space features of the intervention assembly (12);
a fusion module (23) configured to perform spatial fusion processing on the intervention region sub-model, the operation object three-dimensional model and the intervention assembly sub-model based on the intervention position information and the movement position information to obtain the navigation three-dimensional model.

3. The object ablation system according to claim 2, wherein the navigation control device (2) further comprises a navigation planning module (24), and the navigation planning module (24) is communicatively connected to the navigation three-dimensional model:
the navigation planning module (24) is configured to perform navigation planning according to the intervention region sub-model and the operation object three-dimensional model to obtain a navigation path of the intervention assembly (12) in the intervention region sub-model, wherein the navigation path is configured to instruct the intervention assembly (12) to arrive at a travel path of a required path in the process of the to be ablated object in the target intervention region.

4. The object ablation system according to claim 3, wherein the navigation planning module (24) is further configured to determine, in the movement process of the intervention assembly (12) in the target intervention region, the intervention trajectory information of the intervention assembly (12) based on the intervention
position information and the movement position information of the intervention assembly (12); if the path trajectory information corresponding to the intervention trajectory information and the navigation path meets a preset deviation condition, the intervention position of the intervention assembly (12) in the target intervention region is corrected, until the updated intervention trajectory information of the intervention assembly (12) matches the path trajectory information.

5. The object ablation system according to any one of claims 1-4, wherein the navigation control device (2) further comprises an initial parameter acquisition module (25) and a target parameter determination module (26),
the initial parameter acquisition module (25) is configured to obtain an initial ablation parameter corresponding to object attribute information of the to be ablated object, impedance data of the to be ablated object, an abutment parameter of the intervention assembly (12), and a dielectric constant of the to be ablated object, wherein the initial ablation parameter at least comprises at least one of a pulse voltage, a pulse width, a number of pulses, and a number of pulse groups;
the target parameter determination module (26) is configured to obtain ablation effect data corresponding to the initial ablation parameter, based on the navigation three-dimensional model, the operation object three-dimensional model, the object attribute information of the object to be ablated, the initial ablation parameter corresponding to the object attribute information of the object to be ablated, the impedance data of the object to be ablated, the abutment parameter of the intervention assembly (12), and the dielectric constant of the object to be ablated; the initial ablation parameter is determined as the target ablation parameter if the ablation effect data meets a target ablation condition.

6. The object ablation system according to claim 5, wherein the interventional device (1) comprises a contact detection device and a dielectric constant detection device, and the contact detection device and the dielectric constant detection device are communicatively connected to the navigation control device (2), respectively;
the abutting detection device is configured to detect the impedance data of the to be ablated object and the abutment parameter of the intervention assembly (12), wherein the impedance data is configured to indicate a load of the intervention assembly (12), and the abutment parameter is configured to indicate a degree of contact between the intervention assembly (12) and the to be ablated object; and send
the abutment parameter to the initial parameter acquisition module (25); and
the dielectric constant detection device is configured to detect a dielectric constant of the to be ablated object.

7. The object ablation system according to claim 5, wherein the target parameter determination module (26) comprises a simulation ablation model construction unit (261);
the simulation ablation model construction unit (261) is configured to perform ablation effect simulation based on the object attribute information, the initial ablation parameter, the impedance data, the abutment parameter, the dielectric constant, the operation object three-dimensional model, and the navigation three-dimensional model as inputs of an ablation evaluation model, to obtain a simulation ablation model corresponding to the initial ablation parameter, wherein the simulation ablation model is configured to represent ablation effect data corresponding to the initial ablation parameter.

8. The object ablation system according to any one of claims 1-4, wherein the navigation control device (2) further comprises an ablation three-dimensional model generation module (27);
the ablation three-dimensional model generation module (27) is configured to obtain ablation trajectory information and ablation data of the intervention assembly (12) after the intervention assembly (12) performs ablation processing on the to be ablated object; and generate an ablation three-dimensional model according to the ablation trajectory information, the ablation data, and the target ablation parameter, wherein the ablation data comprises an ablation region that has been ablated and ablation position information of the ablation region, and the ablation three-dimensional model represents a three-dimensional spatial feature of the ablation object to be ablated.

9. The object ablation system according to claim 7, wherein the navigation control device (2) comprises a pulse energy generation module (28);
the pulse energy generation module (28) is configured to generate an energy generation control signal based on the target ablation parameter to control the ablation device (4) to operate.

10. The object ablation system according to any one of claim 9, wherein the control circuit of the ablation device (4) comprises a switch circuit (42), the switch circuit (42)
comprises at least two branch circuits (421) connected in parallel, a full bridge, a half bridge or a series connection, and each branch circuit (421) comprises an input terminal switch module (4211) and an output terminal switch module (4212); the switch circuit (42) controls the corresponding input terminal switch module (4211) and the output terminal switch module (4212) to conduct in response to the energy generation control signal of the pulse energy generation module (28), to generate pulse energy.

11. The object ablation system according to claim 10, wherein the branch circuit (421) comprises a filter module (4215), the filter module (4215) comprises a plurality of filtering units, the filtering units comprises a plurality of filters and a plurality of filtering selectors, and the filtering selectors are configured to select different filters for filtering processing.

12. The object ablation system according to any one of claims 1-4, wherein the interventional device (1) comprises a mandrel (13) and a manipulation assembly (14), and the mandrel (13) is configured to be electrically connected to the intervention assembly (12) and the ablation device (4), respectively;
the second position acquisition device (11) is disposed on the mandrel (13) and/or the intervention assembly (12);
the intervention assembly (12) comprises at least one ablation electrode (121), the at least one ablation electrode (121) is mesh, the at least one ablation electrode (121) is sequentially arranged along the mandrel (13), the manipulation assembly (14) is sleeved on an outer side of the mandrel (13), and the manipulation assembly (14) moves relative to the mandrel (13) to drive the ablation electrode (121) to expand or contract.

13. The object ablation system according to any one of claims 1-4, wherein the contact detection device and the dielectric constant detection device are both arranged on the intervention assembly (12), and both of the contact detection device and the dielectric constant detection device are communicatively connected to the navigation control device (2).

14. The object ablation system according to claim 12, wherein the interventional device (1) comprises a fixing sleeve (15), the two ends of the sleeve body of the fixing sleeve (15) are provided with inner tooth structures (151) in the circumferential direction, and both of the inner tooth structures (151) are obliquely arranged from the end face of the sleeve body to the inner side of the end face of the sleeve body.

15. The object ablation system according to claim 14, wherein the second position acquisition device (11) is disposed between the two inner tooth structures (151) of the fixing sleeve (15).

16. The object ablation system according to claim 12, wherein the manipulation assembly (14) comprises at least one control tube (141) connected to the ablation electrode (121).

17. The object ablation system according to claim 12, wherein at least one of the ablation electrodes (121) is configured as a unipolar electrode or a bipolar electrode.

18. The object ablation system according to claim 12, wherein The maximum expandable distance of the plurality of ablation electrodes (121) sequentially arranged along the mandrel (13) are sequentially increased in a direction from a distal end to a proximal end of the mandrel (13).

19. The object ablation system according to claim 18, wherein the ablation electrode (121) comprises a plurality of electrode lines, and the plurality of electrode lines are crossed and woven into a mesh-shaped ablation electrode (121).

20. The object ablation system according to claim 19, wherein an axial section of the ablation electrode (121) after expansion is elliptical, spindle-shaped, polygonal or umbrella-shaped.

21. The object ablation system according to claim 12, further comprising an operating handle (16), wherein a channel is provided in the operating handle (16), through which the mandrel (13) and the manipulation assembly (14) pass.

22. The object ablation system according to claim 21, wherein the operating handle (16) is provided with a manipulation assembly (17), the manipulation assembly (17) is slidably or rotatably connected to the operating handle (16), and the manipulation assembly (17) is configured to control the at least one control tube (141) to move.

23. A medical intervention device, comprising an inner core tube (100), a control outer tube (101), an electrode wire (102) and an ablation electrode (121), wherein the electrode wire (102) is detachably provided in the inner core tube (100) in a penetrating manner, and the control outer tube (101) is sleeved on an outer side of the inner core tube (100);
a distal end of the ablation electrode (121) is fixedly connected to a distal end of
the inner core tube (100), a proximal end of the ablation electrode (121) is fixedly connected to an outer wall of the control outer tube (101), and the control outer tube (101) moves along the inner core tube (100) to drive the ablation electrode (121) to expand or contract.

24. The medical intervention device according to claim 23, wherein the inner core tube (100) is a through tube, and a distal end of the electrode wire (102) passes through a distal end of the inner core tube (100) to be in contact with a lesion tissue.

25. The medical intervention device according to claim 23, wherein the electrode wire (102) and the ablation electrode (121) are both electrically connected to the ablation device (4).

26. The medical intervention device according to claim 23, wherein the ablation electrode (121) comprises a plurality of electrode lines, and the plurality of electrode lines are crossed and woven into a mesh.

27. The medical intervention apparatus according to claim 26, wherein distal ends of the plurality of electrode wires is fixedly connected to an outer wall of a distal end of the inner core tube (100).

28. An energy delivery device, comprising a guide pipe (20), an expandable structure (21), an intervention assembly (12) and a handle (22), wherein one end of the guide tube (20) is connected to the handle (22), and the other end of the guide tube (20) is connected to the expandable structure (21);
a cooling medium channel (201) is formed in the guide tube (20), one end of the cooling medium channel (201) is configured for communicating with a cooling medium source, and the other end of the cooling medium channel (201) is in communication with the expandable structure (21);
the intervention assembly (12) comprises a conveying line (120) and an ablation electrode (121), one end of the conveying line (120) is connected to the ablation electrode (121), the other end of the conveying line (120) is configured for connecting to an ablation device (4), the ablation electrode (121) is in a mesh shape, and the ablation electrode (121) covers the expandable structure (21).

29. The energy delivery device according to claim 28, wherein one end of the guide pipe (20) away from the handle (22) is provided with a second position acquisition device (11).

30. The energy delivery device according to claim 28, wherein the guide pipe (20)
comprises an outer tube (202) and an inner tube (203), and a cooling medium channel (201) is formed between the outer tube (202) and the inner tube (203).

31. The energy delivery device according to claim 30, wherein one end of the expandable structure (21) close to the handle (22) is connected to the outer tube (202), and the other end of the expandable structure (21) is connected to the inner tube (203).

32. The energy delivery device according to claim 28, wherein the ablation electrode (121) is a quadrilateral mesh structure.

33. The energy delivery device according to claim 32, wherein the ablation electrode (121) is made by cutting, weaving or electroforming.

34. The energy delivery device according to claim 33, wherein two ends of the ablation electrode (121) are respectively connected to two ends of the expandable structure (21) through a connector (23).

35. The energy delivery device according to claim 34, wherein the ablation electrode (121) expands with the expansion of the expandable structure (21) and contract with the contraction of the expandable structure (21).

36. The energy delivery device according to claim 28, wherein the handle (22) is provided with a communication part (221), and the communication part (221) is respectively in communication with the cooling medium source and the cooling medium channel (201).

37. The energy delivery device according to claim 29, wherein the second position acquisition device (11) is communicatively connected to the navigation control device (2).

38. An adjustable bending guide tube, comprising a manipulation portion (30), and a first layer tube (31) and a second layer tube (32) connected with the manipulation portion (30), wherein the second layer tube (32) is arranged in the first layer tube (31), and the second layer tube (32) is communicated with the interior of the manipulation portion (30);
a second position acquisition device (11) is arranged at the end, away from the manipulation portion, of the second layer tube (32), and the second position acquisition device (11) is arranged on the outer wall of the second layer tube (32);
the manipulation portion (30) is provided with a bending control member (34) and an adjusting wire (35), one end of the adjusting wire (35) is connected to the bending control member (34), and the other end of the adjusting wire (35) is configured to fixedly
connect to the bending section (321) of the second layer tube (32).

39. The adjustable bending guide tube according to claim 38, wherein a diameter of the first layer tube (31) and a diameter of the second layer tube (32) gradually decrease from a proximal end to a distal end.

40. The adjustable bending guide tube according to claim 38, further comprising a conductive line (33), and the second position acquisition device (11) is connected to the navigation control device (2) by means of the conductive line (33).

41. The adjustable bending guide tube according to claim 38, wherein the bending section (321) is disposed on the second layer tube (32), and a distance from the bending section (321) to a distal end of the second layer tube (32) is 2 -5 cm.

42. The adjustable bending guide tube according to claim 41, wherein a connecting structure (37) is provided on an outer side of the bending section (321), and the bending section (321) is connected to the adjusting wire (35) by means of the connecting structure (37).

43. The adjustable bending guide tube according to claim 38, wherein the bending control member (34) is slidably or rotatably connected to the manipulation portion (30).

44. The adjustable bending guide tube according to claim 43, wherein the curvature of the bending of the second layer tube (32), pulled by the adjusting wire (35) via the bending control member (34), ranges from 0-180°.

45. The adjustable bending guide tube according to claim 38, wherein the adjusting wire (35) is a round wire rope or a flat wire rope composed of a single strand or a plurality of strands of metal wires.

46. The adjustable bending guide tube according to claim 45, wherein an outer side of the adjusting wire (35) is sleeved with a wire bending channel (38), and the wire bending channel (38) is axially arranged along the second layer tube (32) and is connected to the manipulation portion (30).

47. A medical biopsy sampling device, comprising an adjusting portion (40), a guide tube (41), a sampling member (42), and a bending adjustment member (43), wherein the guide tube (41) is sleeved on the sampling member (42), the guide tube (41) is connected to the adjusting portion (40), and the adjusting portion (40) is provided with a first adjusting member (401) and a second adjusting member (402);
one end of the sampling member (42) is connected to the first adjusting member (401), and the other end of the sampling member (42) is provided with a sampling head
(421); one end of the bending adjustment member (43) is connected to the second adjusting member (402), the other end of the bending adjustment member (43) is connected to the bending portion (411) of the guide tube (41), and the bending adjustment member (43) pulls the guide tube (41) to bend through the bending portion (411).

48. The medical biopsy sampling device according to claim 47, wherein the sampling member (42) is provided with a second position acquisition device (11), the second position acquisition device (11) is disposed close to the sampling head (421), and the second position acquisition device (11) is configured to acquire position information of the sampling head (421) and transmit the position information to the navigation control device (2).

49. The medical biopsy sampling device according to claim 47, wherein the bending portion (411) is disposed on the guide tube (41) and is close to the distal end of the guide tube (41).

50. The medical biopsy sampling device according to claim 49, wherein the bending adjustment member (43) comprises a regulating wire (431), one end of the regulating wire (431) is connected to the second adjusting element (402), and the other end of the regulating wire (431) is connected to the bending portion (411).

51. The medical biopsy sampling device according to claim 50, wherein the bending adjustment member (43) comprises an adjustment tube (432), the adjustment tube (432) is sleeved on the adjusting wire (431), and the adjustment tube (432) is connected to the guide tube (41).

52. The medical biopsy sampling device according to claim 51, wherein a connecting member (44) is provided at the bending portion (411) of the guide tube (41), and the adjusting wire (431) is connected to the bending portion (411) by means of the connecting member (44).

53. The medical biopsy sampling device according to claim 47, wherein the first adjusting member (401) and the second adjusting member (402) are respectively slidably or rotatably connected to the adjusting portion (40).

54. The medical biopsy sampling device according to claim 47, wherein the curvature of the guide tube (41), pulled by the bending adjustment member (43), is 0 to 120°.

55. The medical biopsy sampling device according to claim 48, wherein the
second position acquisition device (11) is connected to the navigation control device (2) by means of a signal line (46).

56. An object ablation control method, comprising:
receiving the movement position information of the target object acquired by the first position acquisition device (3) and the intervention position information of the intervention assembly (12) acquired by the second position acquisition device (11) in the target intervention region, the target intervention region belonging to the target object;
performing a virtual model construction based on the regional image information of the target intervention region, the morphological attribute information of the intervention assembly (12), the movement position information and the intervention position information to obtain a navigation three-dimensional model and an operation object three-dimensional model, wherein the navigation three-dimensional model is configured for representing three-dimensional space features of the target intervention region, three-dimensional space features of the intervention assembly (12), and spatial position information between the to be ablated object, the intervention assembly (12) and the target intervention region, and the operation object three-dimensional model is configured for representing a three-dimensional space feature of the to be ablated object;
determining a target ablation parameter corresponding to the to be ablated object according to the navigation three-dimensional model, the operation object three-dimensional model, and object attribute information of the to be ablated object; and
controlling the ablation device (4) to operate based on the target ablation parameter, so that the intervention assembly (12) performs ablation processing on the to be ablated object.

57. An object ablation control apparatus, comprising:
an information receiving module, configured to receive movement position information of a target object collected by a first position acquisition device (3) and intervention position information of an intervention assembly (12) collected by a second position acquisition device (11) in a target intervention region, wherein the target intervention region belongs to the target object; and
model construction module, configured to perform virtual model construction based on regional image information of a target intervention region, morphological attribute information of an intervention assembly (12), the movement position information, and the intervention position information to obtain a three-dimensional navigation model and an operation object three-dimensional model, wherein the navigation three-dimensional model is configured to represent three-dimensional space features of the target intervention region, three-dimensional space features of the intervention assembly (12), and spatial position information between the to be ablated object, the intervention assembly (12), and the target intervention region, and the operation object three-dimensional model is configured to represent a three-dimensional space feature of the to be ablated object;
a parameter determining module, configured to determine a target ablation parameter corresponding to the to be ablated object based on the navigation three-dimensional model, the operation object three-dimensional model, and object attribute information of the to be ablated object; and
an object control module, configured to control the ablation device (4) to operate based on the target ablation parameter, to enable the intervention assembly (12) to perform ablation processing on the to be ablated object.

58. A method for evaluating a pulse ablation effect, comprising:
performing mapping modeling on the current tissue at the ablation catheter to obtain a three-dimensional model of the current tissue; and
obtaining to-be-evaluated data, wherein the to-be-evaluated data comprises detection data collected by an ablation catheter, ablation parameters of an ablation device, and basket catheter state data; and
inputting the to-be-evaluated data into a pre-trained ablation state evaluation model to obtain an ablation state evaluation result, wherein the ablation state evaluation mode calculates the ablation state evaluation result corresponding to the to-be-evaluated data based on the to-be-evaluated data and the correspondence between the to-be-evaluated data and the ablation state; and
displaying the ablation state evaluation result in the three-dimensional model of the current tissue.

59. The pulse ablation effect evaluation method according to claim 58, wherein after the inputting the to-be-evaluated data into a pre-trained ablation state evaluation model to obtain an ablation state evaluation result, comprising:
storing the to-be-evaluated data and the ablation state evaluation result into sample data to obtain updated sample data; and
training and updating the ablation state evaluation model based on the updated sample data.

60. The pulse ablation effect evaluation method according to claim 58, wherein before inputting the to-be-evaluated data into a pre-trained ablation state evaluation model, comprising:
acquiring sample data;
training a pre-constructed machine learning model by using the sample data, and adjusting parameters of the machine learning model in a training process until the ablation state evaluation result output by the machine learning model meets a requirement; and storing the machine learning model as the ablation state evaluation model.

61. The pulse ablation effect evaluation method according to claim 58, wherein the displaying the ablation state evaluation result in the three-dimensional model of the current tissue comprising:
determining a display color corresponding to the ablation state evaluation result based on the ablation state evaluation result and the correspondence between the ablation state evaluation result and the display color; and
displaying the ablation state evaluation result and/or the display color corresponding to the ablation state in the three-dimensional model of the current tissue, wherein the ablation state evaluation result comprises an ablation range and an ablation depth.

62. The pulse ablation effect evaluation method according to claim 58, wherein the detection data comprises an impedance signal, and the obtaining the to-be-evaluated data comprising:
loading a first signal to an electrode of the ablation catheter, wherein a frequency range of the first signal is a first preset frequency range; and
collecting a feedback signal after the first signal acts on the electrode; and
filtering the feedback signal to convert the feedback signal into the impedance signal.

63. The pulse ablation effect evaluation method according to claim 58, wherein the detection data comprises a dielectric constant signal, and obtaining the to-be-evaluated data comprises:
applying a sinusoidal excitation signal to a current tissue through an electrode of the ablation catheter, wherein a frequency range of the sinusoidal excitation signal is a second preset frequency range; and
obtaining a complex impedance electrical signal returned after the sinusoidal excitation signal with different frequencies acts on the current tissue, and using the complex impedance electrical signal as the dielectric constant signal.

64. The pulse ablation effect evaluation method according to claim 58, wherein the basket catheter state data comprises basket catheter opening state data and an abutment area between the basket catheter and the current tissue, and the basket catheter opening state data and the abutment area are configured to determine a thickness of a region to be ablated.

65. An apparatus for evaluating a pulse ablation effect, comprising:
a model establishing module, configured to perform mapping modeling on the current tissue at the ablation catheter to obtain a three-dimensional model of the current tissue; and
a data acquisition module, configured to obtain to-be-evaluated data, wherein the to-be-evaluated data comprises detection data collected by an ablation catheter, ablation parameters of an ablation device, and basket catheter state data; and
a state evaluation module, configured to input the to-be-evaluated data into a pre-trained ablation state evaluation model to obtain an ablation state evaluation result, wherein the ablation state evaluation model calculates the ablation state evaluation result corresponding to the to-be-evaluated data based on to the to-be-evaluated data and the correspondence between the to-be-evaluated data and the ablation state; and
a result display module, configured to display the ablation state evaluation result in the three-dimensional model of the current tissue.

66. A computer-readable storage medium, wherein the storage medium stores at least one instruction or at least one program, and the at least one instruction or the at least one program is loaded and executed by a processor to implement the object ablation control method according to claim 56 and the pulse ablation effect evaluation method according to claims 58-64.

67. An electronic device, comprising a processor and a memory, wherein the memory stores at least one instruction or at least one program, and the at least one
instruction or the at least one program is loaded and executed by the processor to implement the object ablation control method according to claim 56 and the pulse ablation effect evaluation method according to claims 58-64.
